# EUROPEAN PATENT SPECIFICATION

(11) **EP 2 817 340 B1**
(45) Date of publication and mention of the grant of the patent: **09.12.2020**
(21) Application number: 13705987.9
(22) Date of filing: 22.02.2013
(51) Int. Cl.: C07K 16/32, A61K 39/00, A61P 35/00

(54) **HER3 INHIBITOR FOR MODULATING RADIOSENSITIVITY**
HER3 INHIBITOR ZUR MODULIERUNG DER RADIOSENSITIVITÄT
HER3 INHIBITEUR POUR LA MODULATION DE LA RADIOSENSIBILITÉ

(30) Priority: 23.02.2012 US 201261602239 P
(43) Date of publication of application: 31.12.2014
(73) Proprietor: Daiichi Sankyo Europe GmbH, 81379 München (DE)
(72) Inventor: WHEELER, Deric L., Madison, Wisconsin 53705 (US); HETTMANN, Thore, 82152 Martinsried (DE)
(74) Representative: Weickmann & Weickmann PartmbB
(86) International application number: PCT/EP2013/053562
(87) International publication number: WO 2013/124419

(56) References cited:
- EP-A1- 2 138 511
- WO-A2-2007/077028
- WO-A2-2011/060206
- JOSEPH N CONTESSA ET AL: "Compensatory ErbB3/c-Src signaling enhances carcinoma cell survival to ionizing radiation", BREAST CANCER RESEARCH AND TREATMENT, KLUWER ACADEMIC PUBLISHERS, BO, vol. 95, no. 1, 1 January 2006 (2006-01-01), pages 17-27, XP019274943, ISSN: 1573-7217
- H. DOTE: "ErbB3 Expression Predicts Tumor Cell Radiosensitization Induced by Hsp90 Inhibition", CANCER RESEARCH, vol. 65, no. 15, 1 August 2005 (2005-08-01), pages 6967-6975, XP055063197, ISSN: 0008-5472, DOI: 10.1158/0008-5472.CAN-05-1304
- RAO G S ET AL: "Radiosensitization of human breast cancer cells by a novel ErbB family receptor tyrosine kinase inhibitor", INTERNATIONAL JOURNAL OF RADIATION: ONCOLOGY BIOLOGY PHYSICS, PERGAMON PRESS, USA, vol. 48, no. 5, 1 December 2000 (2000-12-01), pages 1519-1528, XP002257052, ISSN: 0360-3016, DOI: 10.1016/S0360-3016(00)01358-4
- JUNTTILA TEEMU T ET AL: "Ligand-independent HER2/HER3/PI3K complex is disrupted by trastuzumab and is effectively inhibited by the PI3K inhibitor GDC-0941", CANCER CELL, CELL PRESS, US, vol. 15, no. 5, 5 May 2009 (2009-05-05), pages 429-440, XP002592637, ISSN: 1535-6108, DOI: 10.1016/J.CCR.2009.03.020 [retrieved on 2009-05-04]
- Ke Liang ET AL: "Sensitization of breast cancer cells to radiation by trastuzumab", Molecular Cancer Therapeutics, 1 November 2003 (2003-11-01), pages 1113-1120, XP055340789, United States Retrieved from the Internet: URL:http://mct.aacrjournals.org/content/mo lcanther/2/11/1113.full.pdf [retrieved on 2017-01-31]
- P M Harari ET AL: "Beyond Cetuximab for Head and Neck Cancer: Dual Target Antibody Blockade of EGFR and HER3 Enhances Radiosensitivity and Overcomes Acquired Resistance to EGFR Inhibitors", International Journal of Radiation Oncology Biology Physics, 1 October 2011 (2011-10-01), pages 1-2, XP055588061, Retrieved from the Internet: URL:https://www.redjournal.org/article/S03 60-3016(11)00977-1/abstract [retrieved on 2019-05-13]

## Description

The present invention relates to an inhibitor of HER-3 for use in the treatment of cancer in combination with radiation treatment.

The human epidermal growth factor receptor 3 (HER-3, also known as ErbB3) is a receptor protein tyrosine kinase and belongs to the epidermal growth factor receptor (EGFR) subfamily of receptor protein tyrosine kinases, which also includes HER-1 (also known as EGFR), HER-2, and HER-4 (Plowman et al., Proc. Natl. Acad. Sci. U.S.A. 87 (1990), 4905-4909; Kraus et al., Proc. Natl. Acad. Sci. U.S.A. 86 (1989), 9193-9197; and Kraus et al., Proc. Natl. Acad. Sci. U.S.A. 90 (1993), 2900-2904). Like the prototypical epidermal growth factor receptor, the transmembrane receptor HER-3 consists of an extracellular ligand-binding domain (ECD), a dimerization domain within the ECD, a transmembrane domain, an intracellular protein tyrosine kinase domain (TKD) and a C-terminal phosphorylation domain.

The ligand Heregulin (HRG) binds to the extracellular domain of HER-3 and activates the receptor-mediated signaling pathway by promoting dimerization with other human epidermal growth factor receptor (HER) family members and transphosphorylation of its intracellular domain. Dimer formation between HER family members expands the signalling potential of HER-3 and is a means not only for signal diversification but also signal amplification. For example the HER-2/HER-3 heterodimer induces one of the most important mitogenic signals among HER family members.

HER-3 has been found to be overexpressed in several types of cancer such as breast, gastrointestinal and pancreatic cancers. Interestingly a correlation between the expression of HER-2/HER-3 and the progression from a non-invasive to an invasive stage has been shown (Alimandi et al., Oncogene 10,1813-1821; deFazio et al., Cancer 87, 487-498; Naidu et al., Br. J. Cancer 78, 1385-1390). Accordingly, agents that interfere with HER-3 mediated signaling are desirable. Murine or chimeric HER-3 antibodies have been reported, such as in US5968511, US5480968 and WO03013602.

Further, WO 2011/060206 discloses the monoclonal anti-HER-3 antibody U1-59 and the use thereof in combination with a second active agent that binds to and inhibits the activity of another member of the HER family.

EP 2 138 511 discloses inhibition of HER-3 by siRNA for the treatment of melanoma.

Harari et al. (Int. J. Radiation, 2011, 81(2) Suppl.S77) discloses a dual specific antibody targeting EGFR and Her3, MEHD7945A (MEHD), which enhances radiosensitivity and overcomes acquired resistance to EGFR inhibitors in HNSCC cell lines in vitro.

Moreover, Contessa et al. (Breast Cancer Res. Treat. 2006, 95, 17-27) discloses that ErbB3/c-Src signaling is radioprotective and that its elimination enhances radiation-induced apoptosis, dependent on the phosphorylation status of ErbB3 (= HER-3).

The object of the present invention was to provide treatment of cancer, based on the inhibition of HER-3 being more effective than known from the prior art. This object is solved by the provision of an inhibitor of HER-3 for the use in treatment of cancer in combination with radiation treatment.

The invention is defined by the claims and any other aspects or embodiments set forth herein not falling within the scope of the claims are for information only.

According to a first aspect the present invention relates to an inhibitor of HER-3 as defined in the claims for the use in treatment of cancer in combination with radiation treatment.

The treatment of a combination of a HER-3 inhibitor and radiotherapy leads to synergistic effects which exceed the advantages of a treatment with either a HER-3 inhibitor or radiotherapy alone.

An inhibitor of HER-3 for use according to the present disclosure may act on the protein level or on the nucleic acid level. Examples for inhibitors acting on the nucleic acid level are known to the person skilled in the art and comprise antisense molecules, RNAi molecules and/or ribozymes.

Examples for an inhibitor acting on the protein level is an anti-HER-3-antibody or an antigen-binding fragment thereof as well as scaffold proteins. The inhibitor being an anti-HER-3 antibody or a fragment thereof, in particular an antigen-binding fragment thereof, represents a preferred embodiment of the present invention. According to the invention term "antibody fragment" comprises any portion of the afore-mentioned antibodies, preferably their antigen binding or variable regions.

As used herein, "scaffold protein" represents an protein having an antibody like binding activity or an antibody, i.e. an anti-HER-3 antibody. Within the context of the present invention, the term "scaffold protein", as used herein, means a polypeptide or protein with exposed surface areas in which amino acid insertions, substitutions or deletions are highly tolerable. Examples of scaffold proteins that can be used in accordance with the present disclosure are protein A from *Staphylococcus aureus,* the bilin binding protein from *Pieris brassicae* or other lipocalins, ankyrin repeat proteins, and human fibronectin (reviewed in Binz and Pluckthun, Curr Opin Biotechnol, 16, 459-69). In addition, a scaffold protein having an antibody like binding activity can be derived from an acceptor polypeptide containing the scaffold domain, which can be grafted with binding domains of a donor polypeptide to confer the binding specificity of the donor polypeptide onto the scaffold domain containing the acceptor polypeptide.

The anti-HER-3 antibody for use according to the invention may be a monoclonal antibody, a recombinant antibody, a human antibody, a humanized antibody, a chimeric antibody, or an antigen-binding fragment thereof. The use of a monoclonal antibody is especially preferred. A person skilled in the art knows how to produce such antibodies. Monoclonal antibodies may, e.g., be produced by any suitable method such as that of Köhler and Millstein (Nature, 1975; 256:495-497).

An antibody fragment for use according to the invention is preferably a Fab fragment, a Fab' fragment, a F(ab')₂ fragment, a Fv fragment, a diabody, or a single chain antibody molecule. The "Fab fragment" differs from the "Fab' fragment" by the addition of a few residues at the carboxy terminus of the heavy chain CH1 domain including one or more cysteines from the antibody hinge region. The "F(ab')₂ fragment" originally is produced as a pair of "Fab' fragments" which have hinge cysteines between them. In accordance with the present invention, the "Fv fragment" is the minimum antibody fragment that contains a complete antigen-recognition and -binding site.

The antibody used according to the invention may be preferably of the IgG1, IgG2, IgG3 or IgG4 antibody type.

Humanized forms of antibodies represent a further preferred embodiment and may be generated according to the methods known in the art such as humanization or CDR grafting. Alternative methods for producing humanized antibodies are well-known in the art and described, for instance, in EP 0 239 400 and WO 90/07861. Human antibodies avoid certain of the problems associated with xenogeneic antibodies, for example antibodies that possess murine or rat variable and/or constant regions. Monoclonal humanized anti-HER-3 antibodies represent an especially preferred embodiment of the invention.

According to a further preferred embodiment the antibody is directed against the extracellular domain of HER-3. The anti-HER-3 antibody preferably interacts with at least one epitope in the extracellular part of HER-3. The epitopes are preferably located in domain L1 (AA19-184), which is the amino terminal domain, in domain S1 (AA185-327) and S2 (AA500-632), which are the two cystein-rich domains or in domain L2 (328-499), which is flanked by the two cystein-rich domains. The epitopes may also be located in any combination of domains such as, but not limited to, an epitope comprised by parts of L1 and S2.

The anti-HER-3 antibody used may be coupled to an effector and/or a labelling group. Corresponding coupling and/or labelling techniques are known to the person skilled in the art. Such effector and/or labelling groups may be attached for drug targeting, imaging applications and/or diagnostic applications.

As used herein, the term "labelling group" refers to a detectable marker, e.g. a radiolabelled amino acid or biotinyl moiety that can be detected by marked avidin (e.g. streptavidin bound to a fluorescent marker or enzymatic activity that can be detected by optical or colorimetric methods). Examples of suitable labelling groups include, but are not limited to, the following: radioisotopes or radionuclides (e.g. ³H, ¹⁴C, ¹⁵N, ³⁵S, ⁹⁰Y, ⁹⁹Tc, ¹¹¹In, ¹²⁵I, ¹³¹I), fluorescent groups (e.g. FITC, rhodamine, lanthanide phosphors), enzymatic groups (e.g. horseradish peroxidase, β-galactosidase, luciferase, alkaline phosphatase), chemiluminescent groups, biotinyl groups, or predetermined polypeptide epitopes recognized by a secondary reporter (e.g. leucine zipper pair sequences, binding sites for secondary antibodies, metal binding domains, epitope tags). In certain aspects, it may be desirable that the labelling groups are attached by spacer arms of various lengths to reduce potential steric hindrance.

Alternatively, an anti-HER-3 antibody used according to the invention may be coupled to an effector group. As used herein, the term "effector group" refers to a cytotoxic group such as a radioisotope or radionuclide, a toxin, a therapeutic group or other effector group known in the art. Examples for suitable effector groups are radioisotopes or radionuclides (e.g. ³H, ¹⁴C, ¹⁵N, ³⁵S, ⁹⁰Y, ⁹⁹Tc, ¹¹¹In, ¹²⁵I, ¹³¹I), calicheamicin, dolastatin analogs such as auristatins, and chemotherapeutic agents such as geldanamycin and maytansine derivates, including DM1. In certain aspects, it may be desirable that the effector groups are attached by spacer arms of various lengths to reduce potential steric hindrance.

In addition to the inhibition of HER-3, it is possible according to a further preferred embodiment to carry out or support the radiation treatment by labelling the anti-HER-3 antibody with a corresponding effector group such as radioisotopes or radionuclides outlined above.

Moreover, the HER-3 inhibitor used, and in particular the anti-HER-3 antibody used, may be further characterized in that its binding to HER-3 reduces HER-3-mediated signal transduction. In accordance with the present invention, a reduction of HER-3-mediated signal transduction may, e.g. be caused by a downregulation of HER-3 resulting in an at least partial disappearance of HER-3 molecules from the cell surface or by a stabilization of HER-3 on the cell surface in a substantially inactive form, i.e. a form which exhibits a lower signal transduction compared to the non-stabilized form. Alternatively, a reduction of HER-3-mediated signal transduction may also be caused by influencing, e.g. decreasing or inhibiting, the binding of a ligand or another member of the HER family to HER-3, of GRB2 to HER-2 or of GRB2 to SHC, by inhibiting receptor tyrosine phosphorylation, AKT phosphorylation, PYK2 tyrosine phosphorylation or ERK2 phosphorylation, or by decreasing tumour invasiveness. Alternatively, a reduction of HER-3 mediated signal transduction may also be caused by influencing, e.g., decreasing or inhibiting, the formation of HER-3 containing dimers with other HER family members. One example among others may be the decreasing or inhibiting of the HER3-EGFR protein complex formation.

Preferred anti-HER-3 antibodies are described in WO 2007/077028.

According to an especially preferred embodiment the antibody comprises a heavy chain amino acid sequence that comprises at least one of the CDR's selected from the group consisting of (a) CDRH1's as shown in SEQ ID NOs: 2, 6, 10, 14, 18, 22, 26, 30, 34, 36, 40, 42, 46, 50, 54, 60, 62, 66, 70, 74, 78, 80, 84, 88, 92, 96, 100, 104, 108, 112, 116, 120, 122, 126, 130, 134, 138, 142, 146, 150, 154, 158, 162, 166, 170, 174, 178, 182, 186, 190, 194, 198, 202, 206, 210, 214, 218, 222, 226 and 230; (b) CDRH2's as shown in SEQ ID NOs: 2, 6, 10, 14, 18, 22, 26, 30, 34, 36, 40, 42, 46, 50, 54, 60, 62, 66, 70, 74, 78, 80, 84, 88, 92, 96, 100, 104, 108, 112, 116, 120, 122, 126, 130, 134, 138, 142, 146, 150, 154, 158, 162, 166, 170, 174, 178, 182, 186, 190, 194, 198, 202, 206, 210, 214, 218, 222, 226 and 230; and (c) CDRH3's as shown in SEQ ID NOs: 2, 6, 10, 14, 18, 22, 26, 30, 34, 36, 40, 42, 46, 50, 54, 60, 62, 66, 70, 74, 78, 80, 84, 88, 92, 96, 100, 104, 108, 112, 116, 120, 122, 126, 130, 134, 138, 142, 146, 150, 154, 158, 162, 166, 170, 174, 178, 182, 186, 190, 194, 198, 202, 206, 210, 214, 218, 222, 226 and 230
or a heavy chain amino acid sequence that shows at least 90%, 92%, 94%, 96%, 97%, 98%, 99% homology to one of the CDRs according to (a) to (c).

According to a further preferred embodiment the antibody comprises wherein the antibody comprises a light chain amino acid sequence that comprises at least one of the CDR's selected from the group consisting of: (d) CDRL1's as shown in SEQ ID NOs: 4, 8, 12, 16, 20, 24, 28, 32, 38, 44, 48, 52, 56, 58, 64, 68, 72, 76, 82, 86, 90, 94, 98, 102, 106, 110, 114, 118, 124, 128, 132, 136, 140, 144, 148, 152, 156, 160, 164, 168, 172, 176, 180, 184, 188, 192, 196, 200, 204, 208, 212, 216, 220, 224, 228 and 232; (e) CDRL2's as shown in SEQ ID NOs: 4, 8, 12, 16, 20, 24, 28, 32, 38, 44, 48, 52, 56, 58, 64, 68, 72, 76, 82, 86, 90, 94, 98, 102, 106, 110, 114, 118, 124, 128, 132, 136, 140, 144, 148, 152, 156, 160, 164, 168, 172, 176, 180, 184, 188, 192, 196, 200, 204, 208, 212, 216, 220, 224, 228 and 232; and (f) CDRL3's as shown in SEQ ID NOs: 4, 8, 12, 16, 20, 24, 28, 32, 38, 44, 48, 52, 56, 58, 64, 68, 72, 76, 82, 86, 90, 94, 98, 102, 106, 110, 114, 118, 124, 128, 132, 136, 140, 144, 148, 152, 156, 160, 164, 168, 172, 176, 180, 184, 188, 192, 196, 200, 204, 208, 212, 216, 220, 224, 228 and 232 or
or a light chain amino acid sequence that shows at least 90%, 92%, 94%, 96%, 97%, 98%, 99% homology to one of the CDRs according to (d) to (f).

In yet another embodiment the antibody comprises a heavy chain amino acid sequence that comprises at least one of the CDR's selected from the group consisting of
(a) CDRH1's as shown in SEQ ID NOs: 2, 6, 10, 14, 18, 22, 26, 30, 34, 36, 40, 42, 46, 50, 54, 60, 62, 66, 70, 74, 78, 80, 84, 88, 92, 96, 100, 104, 108, 112, 116, 120, 122, 126, 130, 134, 138, 142, 146, 150, 154, 158, 162, 166, 170, 174, 178, 182, 186, 190, 194, 198, 202, 206, 210, 214, 218, 222, 226 and 230;
(b) CDRH2's as shown in SEQ ID NOs: 2, 6, 10, 14, 18, 22, 26, 30, 34, 36, 40, 42, 46, 50, 54, 60, 62, 66, 70, 74, 78, 80, 84, 88, 92, 96, 100, 104, 108, 112, 116, 120, 122, 126, 130, 134, 138, 142, 146, 150, 154, 158, 162, 166, 170, 174, 178, 182, 186, 190, 194, 198, 202, 206, 210, 214, 218, 222, 226 and 230; and
(c) CDRH3's as shown in SEQ ID NOs: 2, 6, 10, 14, 18, 22, 26, 30, 34, 36, 40, 42, 46, 50, 54, 60, 62, 66, 70, 74, 78, 80, 84, 88, 92, 96, 100, 104, 108, 112, 116, 120, 122, 126, 130, 134, 138, 142, 146, 150, 154, 158, 162, 166, 170, 174, 178, 182, 186, 190, 194, 198, 202, 206, 210, 214, 218, 222, 226 and 230,
and a light chain amino acid sequence that comprises at least one of the CDR's selected from the group consisting of:
(d) CDRL1's as shown in SEQ ID NOs: 4, 8, 12, 16, 20, 24, 28, 32, 38, 44, 48, 52, 56, 58, 64, 68, 72, 76, 82, 86, 90, 94, 98, 102, 106, 110, 114, 118, 124, 128, 132, 136, 140, 144, 148, 152, 156, 160, 164, 168, 172, 176, 180, 184, 188, 192, 196, 200, 204, 208, 212, 216, 220, 224, 228 and 232;
(e) CDRL2's as shown in SEQ ID NOs: 4, 8, 12, 16, 20, 24, 28, 32, 38, 44, 48, 52, 56, 58, 64, 68, 72, 76, 82, 86, 90, 94, 98, 102, 106, 110, 114, 118, 124, 128, 132, 136, 140, 144, 148, 152, 156, 160, 164, 168, 172, 176, 180, 184, 188, 192, 196, 200, 204, 208, 212, 216, 220, 224, 228 and 232; and
(f) CDRL3's as shown in SEQ ID NOs: 4, 8, 12, 16, 20, 24, 28, 32, 38, 44, 48, 52, 56, 58, 64, 68, 72, 76, 82, 86, 90, 94, 98, 102, 106, 110, 114, 118, 124, 128, 132, 136, 140, 144, 148, 152, 156, 160, 164, 168, 172, 176, 180, 184, 188, 192, 196, 200, 204, 208, 212, 216, 220, 224, 228 and 232.

According to a further preferred embodiment, the antibody comprises a CDRH1, a CDRH2, a CDRH3, a CDRL1, a CDRL2 and a CDRL3.

According to a further preferred embodiment, an anti-HER-3 antibody comprises a heavy chain amino acid sequence selected from the group consisting of SEQ ID NOs: 2, 6, 10, 14, 18, 22, 26, 30, 34, 36, 40, 42, 46, 50, 54, 60, 62, 66, 70, 74, 78, 80, 84, 88, 92, 96, 100, 104, 108, 112, 116, 120, 122, 126, 130, 134, 138, 142, 146, 150, 154, 158, 162, 166, 170, 174, 178, 182, 186, 190, 194, 198, 202, 206, 210, 214, 218, 222, 226 and 230.

A further preferred embodiment relates to the use of an anti-HER-3 antibody comprising a light chain amino acid sequence selected from the group consisting of SEQ ID NOs: 4, 8, 12, 16, 20, 24, 28, 32, 38, 44, 48, 52, 56, 58, 64, 68, 72, 76, 82, 86, 90, 94, 98, 102, 106, 110, 114, 118, 124, 128, 132, 136, 140, 144, 148, 152, 156, 160, 164, 168, 172, 176, 180, 184, 188, 192, 196, 200, 204, 208, 212, 216, 220, 224, 228 and 232.

According to yet another preferred embodiment, an anti-HER-3 antibody comprises a heavy chain amino acid sequence selected from the group consisting of SEQ ID NOs: 2, 6, 10, 14, 18, 22, 26, 30, 34, 36, 40, 42, 46, 50, 54, 60, 62, 66, 70, 74, 78, 80, 84, 88, 92, 96, 100, 104, 108, 112, 116, 120, 122, 126, 130, 134, 138, 142, 146, 150, 154, 158, 162, 166, 170, 174, 178, 182, 186, 190, 194, 198, 202, 206, 210, 214, 218, 222, 226 and 230; and a light chain amino acid sequence selected from the group consisting of SEQ ID NOs: 4, 8, 12, 16, 20, 24, 28, 32, 38, 44, 48, 52, 56, 58, 64, 68, 72, 76, 82, 86, 90, 94, 98, 102, 106, 110, 114, 118, 124, 128, 132, 136, 140, 144, 148, 152, 156, 160, 164, 168, 172, 176, 180, 184, 188, 192, 196, 200, 204, 208, 212, 216, 220, 224, 228 and 232.

Especially preferred embodiments relate to anti-HER-3 antibodies comprising the heavy chain amino acid sequence of SEQ ID NO:42 and the light chain amino acid sequence of SEQ ID NO:44,
or comprising the heavy chain amino acid sequence of SEQ ID NO:54 and the light chain amino acid sequence of SEQ ID NO:56,
or comprising the heavy chain amino acid sequence of SEQ ID NO:70 and the light chain amino acid sequence of SEQ ID NO:72.

In particular, the anti-HER-3 antibody is selected from the group consisting of U1-1 antibody, U1-2 antibody, U1-3 antibody, U1-4 antibody, U1-5 antibody, U1-6 antibody, U1-7 antibody, U1-8 antibody, U1-9 antibody, U1-10 antibody, U1-11 antibody, U1-12 antibody, U1-13 antibody, U1-14 antibody, U1-15 antibody, U1-16 antibody, U1-17 antibody, U1-18 antibody, U1-19 antibody, U1-20 antibody, U1-21 antibody, U1-22 antibody, U1-23 antibody, U1-24 antibody, U1-25 antibody, U1-26 antibody, U1-27 antibody, U1-28 antibody, U1-29 antibody, U1-30 antibody, U1-31 antibody, U1-32 antibody, U1-33 antibody, U1-34 antibody, U1-35 antibody, U1-36 antibody, U1-37 antibody, U1-38 antibody, U1-39 antibody, U1-40 antibody, U1-41 antibody, U1-42 antibody, U1-43 antibody, U1-44 antibody, U1-45 antibody, U1-46 antibody, U1-47 antibody, U1-48 antibody, U1-49 antibody, U1-50 antibody, U1-51 antibody, U1-52 antibody, U1-53 antibody, U1-55.1 antibody, U1-55 antibody, U1-57.1 antibody, U1-57 antibody, U1-58 antibody, U1-59 antibody, U1-61.1 antibody, U1-61 antibody, U1-62 antibody or an antibody having at least one heavy or light chain of one of said antibodies. Especially preferred are the antibodies U1-49 (SEQ ID NO: 42/44), U1-53 (SEQ ID NO: 54/56) and U1-59 (SEQ ID NO: 70/72) or an antibody having at least one heavy or light chain of one of said antibodies. The antibody U1-59 is particular preferred.

The disease to be treated according to the present invention is cancer, in particular squamous cell carcinoma.

Examples of cancer which are preferably treated according to the present invention are selected from the group consisting of breast cancer, gastrointestinal cancer, pancreas cancer, prostate cancer, ovarian cancer, stomach cancer, endometrial cancer, salivary gland cancer, lung cancer, kidney cancer, colon cancer, colorectal cancer, thyroid cancer, bladder cancer, glioma, melanoma, testis cancer, soft tissue sarcoma, head and neck cancer, other HER-3 expressing or overexpressing cancers, and formation of tumour metastases.

According to an especially preferred embodiment the cancer is squamous cell carcinoma of the lung, of the head and/or the neck.

Radiation treatment according to the present invention is selected from external beam radiation therapy and brachytherapy. The person skilled in the art can determine which kind of radiation treatment and in particular which kind of radiation source to be used for a particular patient to be treated.

External beam radiotherapy is the most common form of radiotherapy. In contrast to internal radiotherapy (brachytherapy), external beam radiotherapy directs the radiation at the tumour from outside the body. The voltage to be used may be determined by the person skilled in the art and depends inter alia on the kind of the tumour to be treated. For example, kilovoltage X-rays may be used for treating skin cancer and superficial structures whereas megavoltage X-rays may be used for treating deep-seated tumours (e.g., prostate, lung or brain). While X-ray and electron beams are by far the most widely used sources for external beam radiotherapy, also heavier particle beams such as proton beams may be used.

As already outlined above, brachytherapy is also known as internal radiotherapy. It is a form of radiotherapy, where a radiation source is placed inside or next to the area requiring treatment. In brachytherapy radiation sources are precisely placed directly at the side of the cancerous tumour. This means that the radiation only affects a limited local area which provides advantages over X-ray beam radiation therapy. For example, the tumour may be treated with very high doses of localized radiation whilst reducing the probability of unnecessary damage to surrounding healthy tissues. Examples of radiation sources used for brachytherapy comprise ¹²⁵I/¹⁰³Pd, ⁹⁰Y as well as Selective internal radiation therapy (SIRT). SIRT comprises the use of micospheres, which might be, for example, injected. Such microspheres can be made of resin or glass. Inside the microsphere the radiation source such as yttrium-90 can be placed. Examples of microspheres which are used already are ThereSphere and SIR-Spheres, which differ in their radioactivity per sphere and embolic effect. Of course, the use of any further suitable carrier coupled to a radiation source is also contemplated. Further examples for medicaments used in brachytherapy comprise SAVI, MammoSite, Contura, Proxcelan and I-Seed.

Radiation treatment used according to the invention may be based on an single dose or fractionated dosing of radiation. Of course, it is also possible to vary the duration of radiation therapy. The duration may depend on the location of the tumours to be treated, the size of the tumour to be treated and the physical condition of the patient.

The combination of an inhibitor of HER-3 and radiation therapy and/or treatment for use according to the present disclosure contemplates the simultaneous treatment with an HER-3 inhibitor and radiation therapy as well as a timely shifted combination. According to a preferred embodiment tumour cells to be treated are radiosensitized by the HER-3 inhibitor before radiation treatment, i.e. the HER-3 inhibitor is administered before radiation treatment/therapy.

The present invention can be used in combination with a further active compound like a further chemotherapeutic compound such as cytotoxic agents, such as doxorubicin, cis-platin or carboplatin, cytokines or antineoplatic agents.

Another aspect of the present disclosure relates to the use of an inhibitor of HER-3 for the modulation of radiosensitivity of cells.

### BRIEF DESCRIPTION OF THE FIGURES

**Figure 1****.**
   **(A) HER3 profile in multiple carcinoma celllines.**
      Five human lung carcinoma celllines (NCI-H226, H292, H358, H520 and A549), five colorectal carcinoma celllines (Caco2, SW48, LS180, Lovo and HCT116) and five head and neck carcinoma celllines (SCC1, SCC6, SCC11A, SCC38 and SCC1483) were cultured in relevant mediums. Whole cell lysate was obtained with lysis buffer. Protein was quantitated using the Bradford method, sepreated by SDS gel and followed by immunoblot with the indicated antibodies.
   **(B) Basal activity of HER3 is blocked by U1-59.**
      Four head and neck carcinoma celllines (SCC6, SCC11A, SCC38, SCC1483) with high p-HER3 and two lung cancer cellines (H226 and A549) were treated with doses of U1-59 for 24h. Whole cell lysates were isolated and followed by immumoblot with indicated antibodies. U1-59 inhibited HER3 activity and its downstream signal in a dose-dependent manner in those cells.
**Figure 2****:**
   **Radiation-induced activation of HER3 is blocked by U1-59. (A, D)** HER3 was transient activated by radiation during the indicated time and blocked by U1-59. SCC6 and H226 cells were incubated with/without U1-59 (20ug/ml) for 24h before radiation. After exposure to 4Gy radiation, whole cell lysates were isolated at the indicated times, followed by immunoblot with p-HER, p-AKT and p-MAPK antibodies. **(B, E)** U1-59 kept blocking activations of AKT and MAPK 24h and 48h after radiation. **(C, F)** Immunoflurescence showing HER3 were activated by radiation and blocked by U1-59 in SCC6 and H226 cells (30 minutes after radiation).
**Figure 3****.**
   **(A, F) U1-59 inhibited cell proliferation in a dose-dependent manner.** SCC6 and H226 cells were seeded in 96-well plates and incubated with doses of U1-59 for 72h. Cell proliferation were detected using CCK8 72h. **(B, G) Sensitizing effect of U1-59 on SCC6 cells in response to radiation.**
   SCC6 and H226 were incubated with U1-59 for 4 h, and radiated with indicated doses. Clonegenic assay were performed as described. Control curves were exposed to radiation without U1-59 treatment. **(C, H) Impact of U1-59 on cell cycle.**
   SCC6 and H226 cells were incubated with U1-59 (20ug/ml) for 48h, followed by exposure to 6Gy radiation. 24h after radiation, cell cycle (stained with PI) was detected using flow cytometer and analyzed by FlowJo. U1-59 caused G1 arrest and radiation caused G2 arrest. Combination of U1-59 and radiation reassorted cell within cell cycle, induced accumulation of cells in G1 and G2, and reduced the population of cells in S phase.
   **(D, I) Impact of U1-59 on cell apoptosis.**
   Apoptosis (stained with annexin V/PI) was detected using flow cytometer and analyzed by FlowJo. Combination of U1-59 with radiation increased the percentage of apoptotic cells.
   **(E, J) U1-59 contribute to γ-H2AX focus formation.**
   SCC6 and H226 were incubated with U1-59 for 24h before 4 Gy radiation. 4h after radiation, cells were fixed and performed immunoflurescence staining. Compared with drug or radiation alone, increased number of γ-H2AX foci was detected in the cell treated with XRT combined with U1-59, demonstrated enhanced DNA damage by U1-59
**Figure 4****.**
   Antitumour effect of U1-59 combined with radiation on xenograft tumours in athymic mice. SCC6, SCC1483 and H226 xenograft were performed as described in "Materials and methods". (A) Mice were treated with single dose of IgG, radiation (10Gy or 16Gy), U1-59 (8mg/kg) or combination of both. Data points were expressed as mean tumour size±SD. Results were graphed with tumour growth curve and Kaplan Meir survival curve. (B) Mice were treated with fractionated doses of IgG, radiation, U1-59 (100ug/mouse) or combination of both twice a week as shown. Results were graphed with tumour growth curve and Kaplan Meir survival curve.
**Figure 5****.**
   U1-59 inhibits basal and radiation-induced activation of HER3 in xenograft tumours. SCC6, SCC1483 and H226 xenograft tumours were collected at 24h post radiation or the last radiation in fractionated treatment groups. Protein was isolated from the tumours and p-HER3 was analyzed by western blot. Increased p-HER3 was found in radiated tumours and reduced p-HER3 was found in tumours with U1-59 or combined treatment.
**Figure 6****.**
   U1-59 inhibits cell survival signals and enhances DNA damage in combination with radiation on xenograft tumours in athymic mice. SCC6 xenograft tumours with single dose treatment of radiation (16 Gy), U1-59 (8mg/kg) or combination of both. tumours were harvested 24h post treatment. IHC stain were performed as describe in "Materials and methods". Reduced p-MAPK, p-S6 and PCNA staining, decreased PCNA positive cells and increased γ-H2AX positive cells were detected in tumours with U1-59 or combined treatment.

### Examples

### MATERIALS AND METHODS

### Cell culture and drug

Five human lung carcinoma celllines (NCI-H226, H292, H358, H520 and A549) and five colorectal carcinoma celllines (Caco2, SW48, LS180, Lovo and HCT116) were purchased from ATCC (Manassas, VA, USA) and maintained in 10% fetal bovine serum (FBS) in RPMI1640 or DMEM (Mediatech Inc., Manassas, VA, USA) with 1% penicillin and streptomycin. Five head and neck carcinoma celllines (UM-SCC1, UM-SCC4, UM-SCC6, UM-SCC11A, and UM-SCC1483 cells) were obtained from University of Michigan and maintained in 10% FBS (Invitrogen, Carlsbad, CA, USA) in DMED supplemented with 1% hydrocortisone.

The anti-HER-3 antibody U1-59 was used.

### Cell proliferation assay

Cells were seeded in 96-well plate and exposed to doses of U1-59 for 72h. Cell proliferation was tested by Cell Counting Kit-8 (Dojindo Molecular Technologies, Gaithersbury, MD, USA).

### Clonogenic assay

A specified number of cells were seeded into each well of six-well tissue culture plates. After allowing cells time to attach (6 hours), U1-59 or the vehicle control (PBS) was added at specified concentrations. The plates were irradiated 4 hours later at the doses of 2, 4, 6, 8Gy. 10 to 14 days after seeding, colonies were stained with crystal violet, the number of colonies containing at least 50 cells was determined and the surviving fractions were calculated. Survival curves were generated after normalizing for cytotoxicity generated by U1-59 alone. Data presented are the mean±SD from at least three independent experiments.

### Cell cycle analysis

Cells were incubated with U1-59 (20ug/ml) for 48h, followed 6Gy radiation. After 24h, cells were trypsinized and washed with PBS, fixed in 90% ethanol and stored at 4 °C overnight. After remove of ethanol by centrifugation, cells were stained with PI stain buffer (50ug/ml PI, 100ug/ml Rnase A, 0.1% Triton X-100). Cells were sorted by FACSCalibur flow cytometer (BD Biosciences, San Jose, CA, USA). Histogram analysis was performed with FlowJo software (Tree Star Inc., Ashland, OR, USA).

### Apoptosis

Apoptosis were detected using Annexin V /PI kit. Following treatment, a cell suspension containing 1 × 10⁵ cells in 100µl staining buffer was incubated with 5µl Annexin V and PI. Cells were sorted by FACSCalibur flow cytometer (BD Biosciences, San Jose, CA, USA). Population analysis was performed with FlowJo software.

### Immunoblotting analysis

Following treatment, cells were lysed with buffer (50 mM HEPES, pH 7.4, 150 mM NaCl, 0.1% Tween-20, 10% glycerol, 2.5 mM EGTA, 1 mM EDTA, 1 mM DTT, 1 mM PMSF and 10 µg/ml of leupeptin and aprotinin). Protein was quantized using a standard Bradford absorbance assay. Equal amounts of protein were fractionated by SDS-PAGE. Thereafter, proteins were transferred to PVDF membrane and analyzed by incubation with the appropriate primary antibody. Proteins were detected via incubation with HRP-conjugated secondary antibodies and ECL chemiluminescence detection system. The NIH ImageJ program was used to measure densitometry of the western bands. The antibodies used in this study were as follows: HER3, AKT, MAPK, horseradish peroxidase-conjugated goat-anti-rabbit IgG and goat-anti-mouse IgG were purchased from Santa Cruz Biotechnology Inc. (Santa Cruz, CA, USA). p-HER3 (Tyr1289), p-AKT and p-MAPK were obtained from Cell Signaling Technology (Beverly, MA, USA). α-tubulin was from Calbiochem (San Diego, CA, USA).

### Immunofluorescence assay

Approximately 2 × 10³ cells were seeded on a four-well glass chamber slide (Nalgene Nunc, Naperville, IL, USA). Forty-eight hours later, cells were washed 3 times with PBS and fixed with 2% formaldehyde for 15 min at room temperature. Cells were incubated in ice-cold 100% methanol for 10 min at -20 °C and blocked with 5% normal serum in PBS with 0.3% Triton X100 solution for 1 h at room temperature and incubated with p-HER3 or λ-H2AX antibody overnight at 4 °C. Next, cells were incubated with FITC-conjugated appropriate secondary antibody in PBS containing 0.3% Triton X100 and 1% BSA for 2h. Slides were mounted using ProLong gold with DAPI (Invitrogen). Photographs were captured by confocal microscopy or fluorescence microscopy.

### Mouse xenograft model

Athymic nude mice (4- to 6-week old; male) were obtained from the Harlan Laboratories (Indianapolis, IN, USA). All animal procedures and maintenance were conducted in accordance with the institutional guidelines of the University of Wisconsin. Cells were injected bilaterally in the dorsal flanks of the mice at day 0 (2 × 106 cells). Once tumours reached expected volumes , Mice were single or fractionated dosed-treated with 1) IgG, 2) U1-59, 3) radiation or 4) the combination. Measurements were evaluated by digital calipers and calculated by the formula (π)/6 × (large diameter) × (small diameter).

### Immunohistochemistry

### Xenograft tumours were fixed in neutral formalin and embedded in Paraffin.

Immunohistochemical staining was performed for PCNA, p-MAPK, p-AKT, p-S6 and γ-H2AX. In brief, specimen was deparaffinized and rehydratded routinely, following antigene retrieval by citrate buffer for 15mins at 98°C in water bath, incubation in 3% hydrogen peroxide for 10 minutes, 3% BSA blocking for 30min. Staining were performed as below: incubation in primary antibody diluted in recommended antibody diluents at 4°C overnight, incubation in biotinylated secondary antibody for 30 minutes at room temperature, peroxidase visualization using Dakocytomation Liquid DAB+Substracte Chromogen System, Counterstain in Hematoxylin, routine Dehydrate and Clear, mounting with coverslip.

### Statistical analysis

Xenograft tumour growths were graphed and analyzed using GraphPad Prism 5 (GraphPad, San Diego, CA).

### RESULTS

### HER3 is expressed in multiple solid tumour cell lines

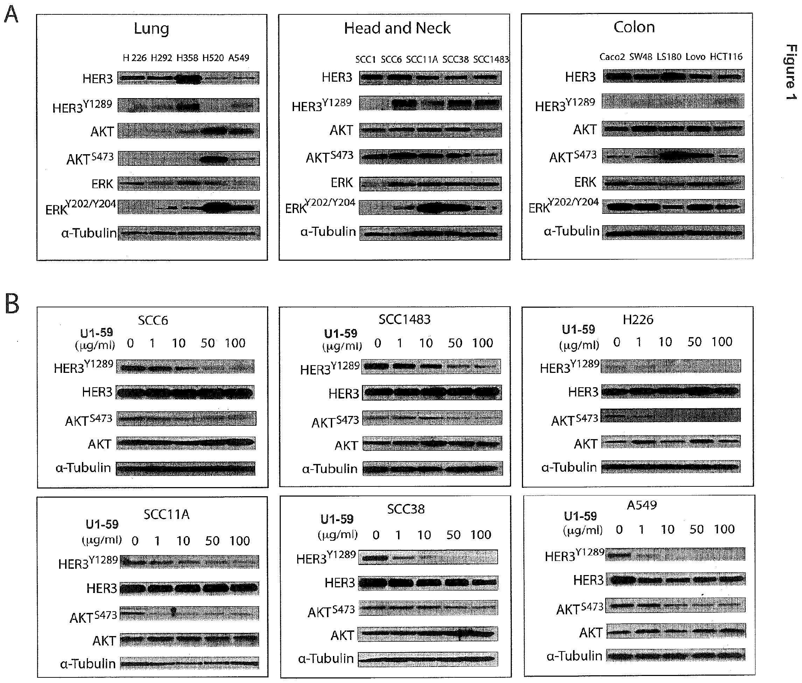

Human epidermal growth factor receptor 3 (HER3) is a key dimerization partner for the HER family and activates oncogenic signaling pathways. Its overexpression in many solid tumours has been linked to poor prognosis. In **Figure 1A** we charactered 15 cell lines derived from non-small cell lung carcinoma (H226, H292, H358, H520 and A549), colorectal carcinoma (Caco2, SW48, LS180, Lovo and HCT116) and head and neck squamous cell carcinoma (SCC1, SCC6, SCC11A, SCC38 and SCC1483) for the expression of both HER3 and p-HER3. Both HNSCC and NSCLC cell lines contain activated HER3 suggesting that HER3 may be enhancing their tumourigenic phenotype.

### U1-59 can inhibit basal activity of HER3 and radiation-induced activation of HER3

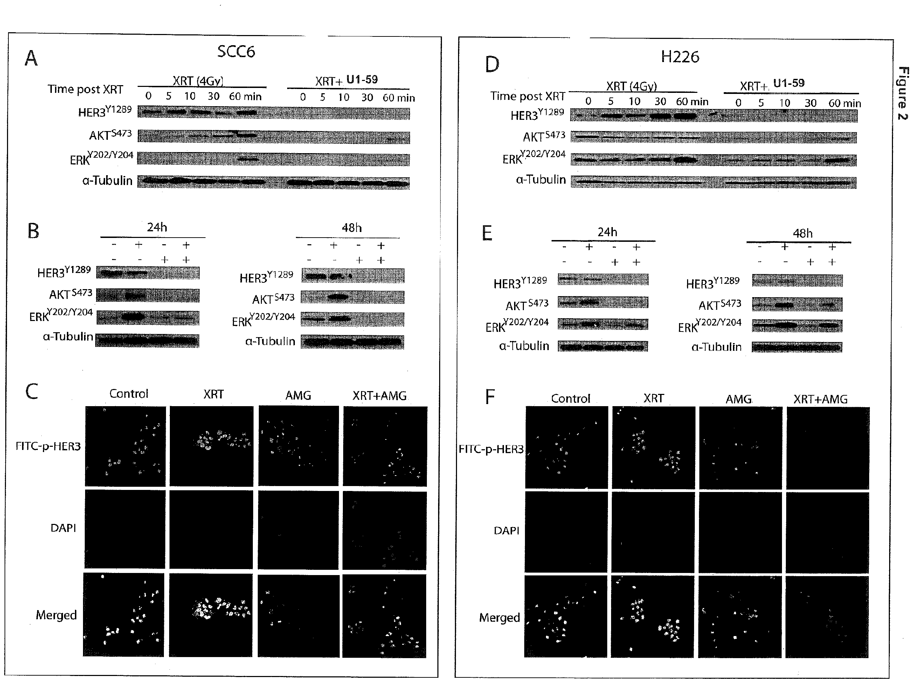

HER3 lacks intrinsic tyrosine kinase activity. However, upon binding to multiple ligands, such as heregulin (neuregulin-1), HER3 can form heterodimers with other HER family member receptors to initiate the activation of multiple signaling pathways that strongly influence cell proliferation and survival. U1-59 is a fully humanized monoclonal antibody that binds to and inactivates HER3 oncogenic signaling pathways. In figure 1B, we demonstrate that treatment of multiple HNSCC and NSCLC cell lines with escalating doses of U1-59 can successfully inhibit the activation of both HER3 and AKT **(****Figure 1B****).** In addition, using immunoblotting and immunofluorescence assays, we found that radiation treatment of the HNSCC cell line SCC6 and the NSCLC cell line H226 increased activated forms of HER3 **(****Figure 2A, 2C, 2D, and 2F****).** The transient activation of HER3 after radiation treatment was also accompanied by an increase in activation of both ERK and AKT in both SCC6 and H226 cell lines **(****Figure 2A and 2D****).** In **Figures 2B and 2E** we demonstrate that radiation can lead to the continual activation of HER3, AKT, and ERK in both SCC6 and H226 cell lines both 24 and 48 hours post radiation treatment. We subsequently demonstrate that the pre-incubation of either SCC6 or H226 cell lines with U1-59 for 24hrs prior to radiation treatment could prevent the radiation-induced activation of HER3, ERK, and AKT **(****Figure 2A, 2C, 2D, and 2F****).** Additionally, U1-59 could lead to the continued inhibition HER3, ERK, and AKT activation at both 24 and 48 hours post radiation treatment **(****Figure 2B and 2E****).** Collectively, these data suggest that pre-incubation of cells with U1-59 prior to radiation treatment can prevent radiation induced activation of cell survival and proliferation pathways.

### U1-59 can radiosensitize HNSCC and NSCLC cell lines in-vitro

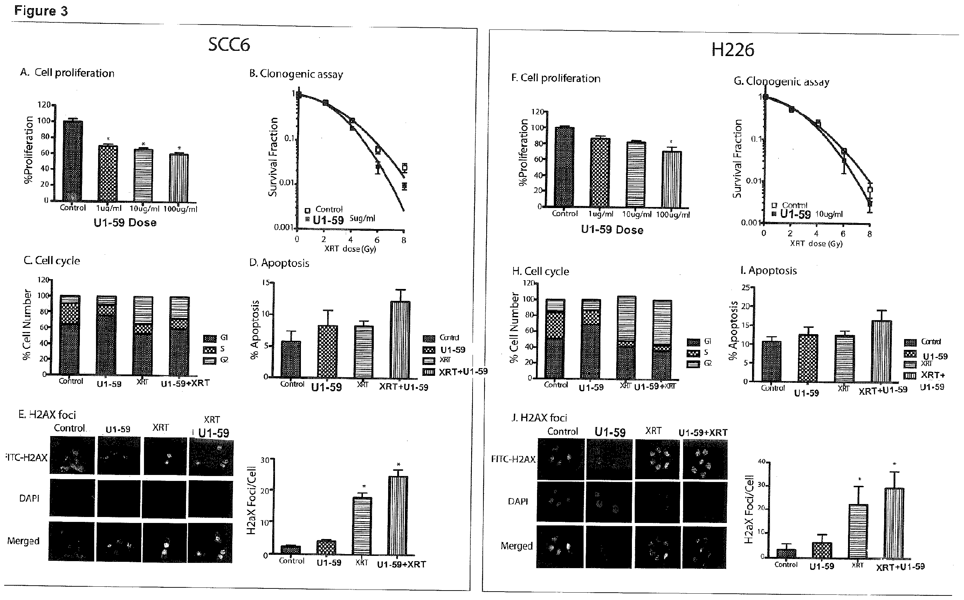

In **Figure 2** we demonstrated that U1-59 could prevent radiation-induced activation of HER3 and subsequent downstream effectors ERK and AKT, therefore we hypothesized that U1-59 could enhance the radiosensitivity of both HNSCC and NSCLC cell lines. First, we demonstrate in **Figure 3A and 3F** that U1-59 can inhibit the growth of both SCC6 and H226 cell lines in a dose-dependent manner.To evaluate the *in vitro* effect of U1-59 combination with radiation, we conducted clonogenic assays as described in "materials and methods". We pre-incubated both SCC6 and H226 cells with U1-59 for 4 hr, and subsequently subjected cells to radiation with the indicated doses. As shown in **Figures 3B and 3G****,** U1-59 increased the slope of the radiation curves, demonstrating a sensitizing effect of U1-59 on both SCC6 and H226 cells in response to radiation. Control curves were exposed to radiation without U1-59 treatment.

### U1-59 can promote cell cycle arrest and apoptosis in combination with radiation treatment in-vitro

We further hypothesized that U1-59 may also enhance HNSCC and NSCLC cell lines to radiation induced cell cycle arrest and apoptosis. To study this we pre-incubated both SCC6 and H226 cells with U1-59 for 48h followed by exposure to 6Gy radiation. 24h post radiation we analyzed the cell cycle phase distribution and apoptotic levels by PI and annexin V staining. As shown in **Figure 3C and 3H** U1-59 pre-treated cells demonstrated a G1 phase arrest, while radiation treated cells demonstrated a G2 phase arrest as compared to control cells. We observe a decrease in S-phase with either treatment. It is well known that cells in G1 phase are more sensitive to radiation treatment, while S-phase cells are more resistant. Therefore we demonstrate through Annexin V staining that apoptosis is indeed enhanced in radiation treated cells pre-incubated with U1-59 **(****Figure 3D and 3I****),** suggesting that the G1 phase arrest induced by U1-59 may have promoted the radiosentitivy of both SCC6 and H226. In addition, we further show an enhanced level of DNA double strand breaks via γ-H2AX staining in SCC6 and H226 cells pre-treated with U1-59 prior to radiation **(****Figure 3E** **abd 3J).** Overall, these data demonstrate the enhanced antitumour effect of U1-59 in combination with radiation.

### U1-59 in combination with radiation can have antitumour effects in HNSCC and NSCLC mouse tumour xenografts

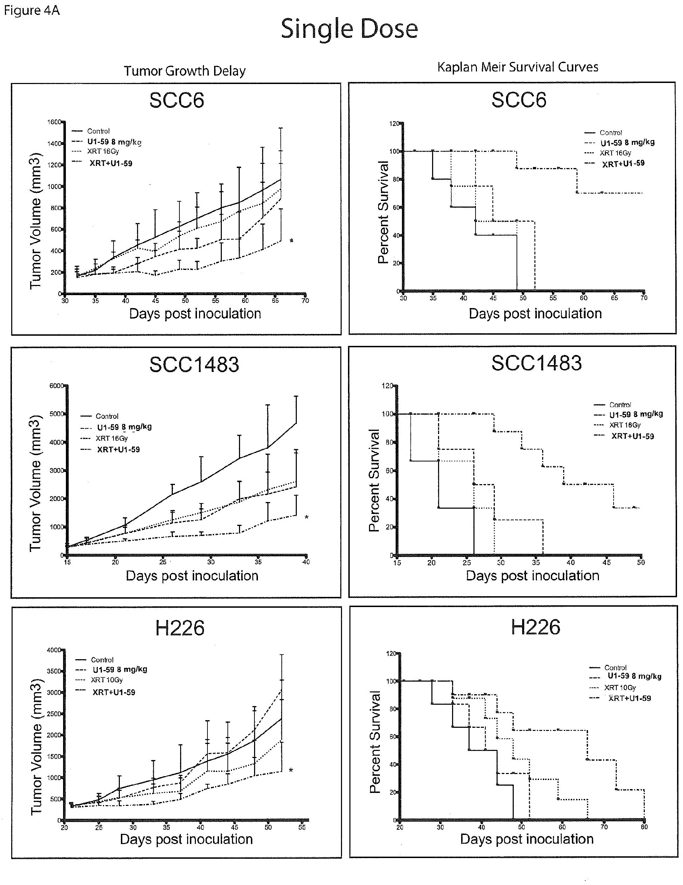

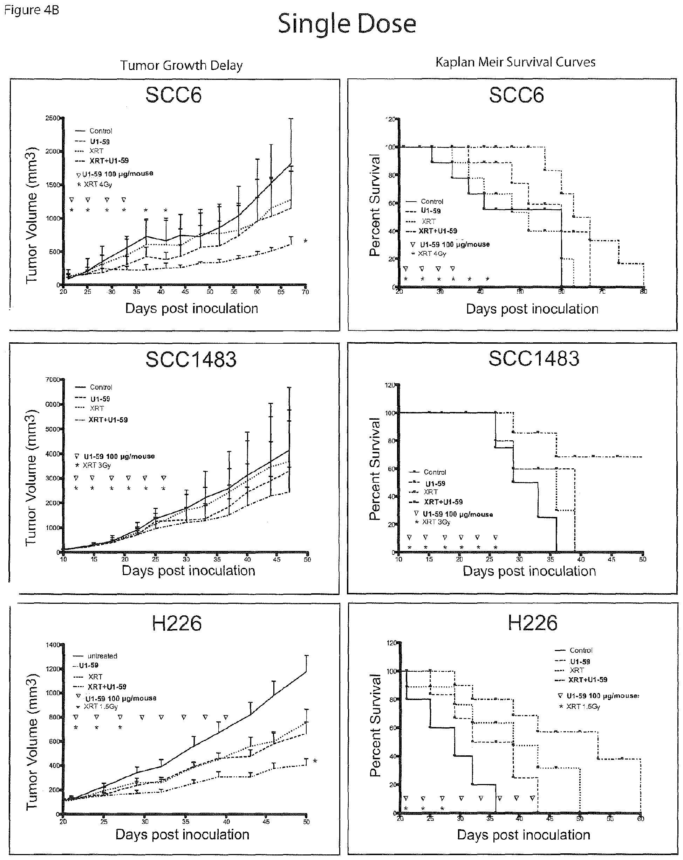

To further evaluate the efficacy of radiotherapy combined with U1-59, we inoculated SCC6, SCC1483 and H226 cell lines into athymic mice. Once tumours reached 100-200mm³ tumours were divided up for both single and fractionated doses of radiation. In the single dose treated group, xenograft mice were administered with IgG, U1-59 (8mg/kg), radiation (XRT16Gy or XRT10Gy) or a combination of both (Figure 4A). In fractionated treated group, xenograft mice were administered with IgG, U1-59 (100ug/mouse), radiation (twice a week at indicated doses), or combination **(****Figure 4B****).** All three xenografts models demonstrated a marked reduction in tumour volume when treated with both U1-59 in combination with either a single dose or fractionated dose of radiation as compared to single therapy treated mice or IgG control treated mice. Additionally, xenografts treated with both U1-59 and either a single or fractionated dose of radiation demonstrate a clear survival advantage as depicted via Kaplan Meir survival curve analysis.

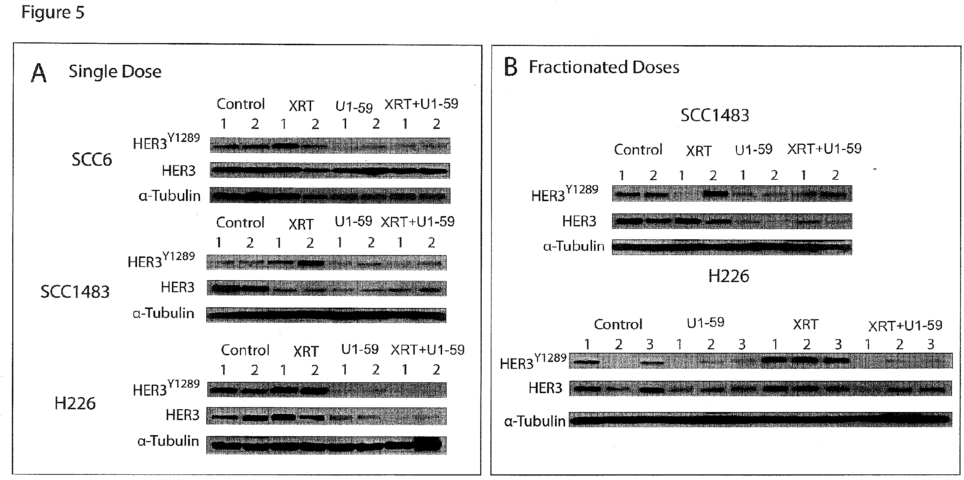

To validate that U1-59 could effectively inhibit the activation of HER3 in-vivo, we isolated protein from various tumours from each treatment group and performed western blot analysis for both total and activated forms of HER3. In **Figure 5A** we demonstrate that tumours isolated from mice treated with U1-59 alone or U1-59 in combination with a single dose of radiation have lower levels of HER3 activation. In SCC1483 and H226 xenograft models U1-59 treated tumours also contained lower total amounts of HER3 protein **(****Figure 5A****),** something that was not observed in these cell lines treated with U1-59 in-vitro. Additionally, at least one of the two tumours isolated from each xenograft model in **Figure 5A** demonstrated a clear activation of HER3 upon a single dose of radiation, which was blocked in the U1-59 treated tumours. In **Figure 5B** we further demonstrate that U1-59 treated tumours in combination with fractionated doses of radiation also contained reduced levels of activated and total levels of HER3; activation of HER3 by fractionated doses of radiation was also blocked by U1-59 as well. Collectively, xenograft tumours treated with both U1-59 in combination with either a single dose or fractionated doses of radiation demonstrate a clear growth delay with marked reduction in tumour volumes as compared to controls or either treatment alone, suggesting that the loss of both activated and total levels of HER3 expression may radiosensitize HNSCC and NSCLC tumours.

### U1-59 in combination with radiation can prevent the activation of cell survival signals and enhance DNA damage in-vivo

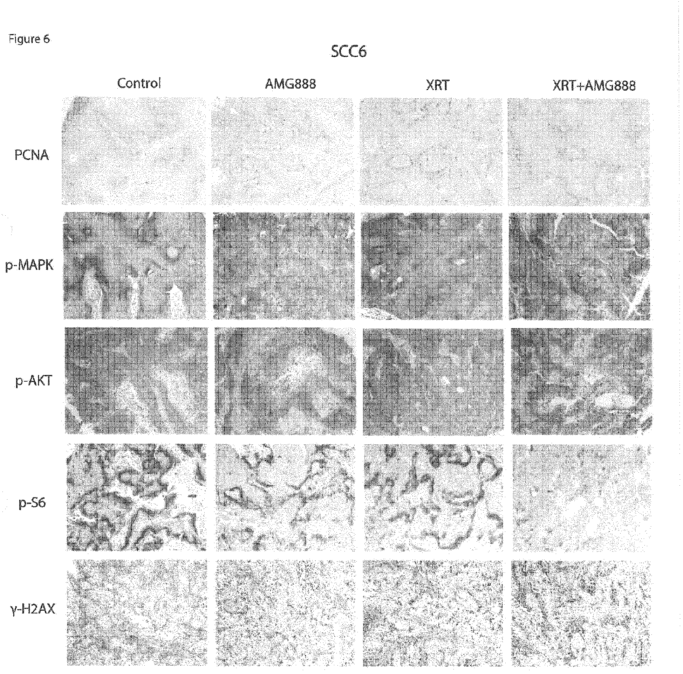

To further analyze the growth inhibitory effects of U1-59 in combination with radiation treatment, we analyzed the activation of ERK, AKT, S6 kinase, and **γ-H2AX** in SCC6 treated tumours via immunohistochemistry **(****Figure 6****).** We demonstrate that SCC6 cells treated with both U1-59 and a single dose of radiation can have lower activation levels of the downstream effector molecules ERK, AKT, and S6 kinase. Additionally, U1-59 treated tumours demonstrated a decreased level of PCNA positive cells. Finally, increased levels of γ-H2AX were found in SCC6 tumours treated with both U1-59 and radiation. Overall, these data indicate that U1-59 in combination with radiation can efficiently inhibit cell survival and proliferation pathways, in addition to enhancing DNA damage. These data provide mechanistic evidence for the enhanced antitumour effect of U1-59 combined with radiation *in vivo.*

The results of these experiments indicate that the combination of U1-59 and radiation had a strong impact on tumour growth in studies using single dose or fractionated dosing of radiation. Tumour analysis indicated that radiation treatment activated HER3 in vivo and U1-59 could abrogate this activation. Collectively our findings in vitro and in vivo indicate that U1-59 in combination with radiation has an impact on cell and tumour growth by delaying cell cycle progression, increasing apoptosis and increasing DNA damage. These findings indicate that HER3 may play an important role in response to radiation therapy and blocking its activity may be of strong therapeutic benefit in human tumours.

**Table of CDR Sequences**

| Ab chain | Pat. ID: | CDR1 | CDR2 | CDR3 |
|---|---|---|---|---|
| heavy | U1-1 | GGSINSGDYYWS | YIYYSGSTYYNPSLKS | ADYDFWSGYFDY |
| light | | RASQGIRNDLG | AASSLQS | LQHNSYPWT |
| heavy | U1-2 | GGSISSGDYYWS | YIYYSGSTYYNPSLRS | ADYDFWSGYFDY |
| light | | RASQGIRNDLG | AASSLQS | LQHNGYPWT |
| heavy | U1-3 | GGSISSGGYYWS | YIYYSGSTYYNPSLKS | DGYDSSGYYHGYFDY |
| light | | KSSQSVLYSSNNKNYLA | WASTRES | QQYYSTPLT |
| heavy | U1-4 | GGSISSGDYYWS | YIYYSGSTYYNPSLKS | ADYDFWSGYFDY |
| light | | RASQGIRNDLG | AASSLQS | LQHNNYPWT |
| heavy | U1-5 | GGSISSGDYYWS | YIYYSGSTYYNPSLKS | ADYDFWSGYFDY |
| light | | RASQGIRNDLG | AASSLQS | LQHNTYPWT |
| heavy | U1-6 | GGSISSGDYYWS | YIYYSGSTYYNPSLKS | ADYDFWNGYFDY |
| light | | RASQGIRNDLG | AASSLQS | LQHNTYPWT |
| heavy | U1-7 | GGSISSGDYYWS | YIYYSGSTYYNPSLKS | ADYDFWSGYFDY |
| light | | RASQDIRNDLG | AASSLQS | LQHNSYPWT |
| heavy | U1-8 | GYTLTELSMY | GFDPEDGETIYAQKFQG | GWNYVFDY |
| light | | RSSQSLLHSNGYNYLD | LDSHRAS | MQALQTPLT |
| heavy | U1-9 | GGSISSGDYYWS | YIYYSGSTYYNPSLKS | ADYDFWNGYFDY |
| light | | RASQDIRNDLG | AASSLQS | LQHNSYPWT |
| heavy | U1-10 | GGSISSGDYYWS | YIYYSGSTYYNPSLKS | ADYDFWSGYFDY |
| light | | RASQGIRNDLG | AASSLQS | LQHNNYPWT |
| heavy | U1-11 | GGSISSGDYYWS | YIYYSGSTYYNPSLKS | ADYDFWSGYFDY |
| light | | RASQGIRNDLG | AASSLQS | LQHNTYPWT |
| heavy | U1-12 | GGSISSGDYYWS | YIYYSGSTYYNPSLKS | ADYDFWSGYFDY |
| light | | RASQGIRNDLG | AASSLQS | LQHNNYPWT |
| heavy | U1-13 | GGSISSGGYYWS | YIYYSGSTYYNPSLKS | EDDGMDV |
| light | | RSSQSLLHSNGYNYLE | LGSNRAS | MQALQTPIT |
| heavy | U1-14 | GGSISSGDYYWS | YIYYSGSTYYNPSLKS | ADYDFWSGYFDY |
| light | | RASQGIRNDLG | AASSLQS | LQHNTYPWT |
| heavy | U1-15 | GGSVSSGGYYWS | YIYYSGSTNYNPSLKS | DGDVDTAMVDAFDI |
| light | | RASQSLSGNYLA | GASSRAT | QQYDRSPLT |
| heavy | U1-16 | GGSISSGDYYWS | YIYYSGSTYYNPSLKS | ADYDFWSGYFDY |
| light | | RASQGIRNDLG | AASSLQS | LQHNSYPWT |
| heavy | U1-17 | GGSISSGDYYWS | YIYYSGSTYYNSSLKS | ADYDFWSGYFDY |
| light | | RASQGIRNDLG | AASSLQS | LQHNSYPWT |
| heavy | U1-18 | GGSISSGDYYWS | YIYYSGSTYYNPSLKS | ADYDFWSGYFDY |
| light | | RASQGIRNDLG | AASSLQS | LQHNSYPWT |
| heavy | U1-19 | GGSISSGDYYWS | YIYYSGSTYYNPSLKS | GDYDFWSGEFDY |
| light | | sequence not available | | |
| heavy | U1-20 | GGSISSGGYYWS | YIYDSGSTYYNPSLKS | |
| light | | QASQDISNYLN | VASNLET | QQCDNLPLT |
| heavy | U1-21 | GGSISSGDYYWS | YIYYSGSTYYNPSLKS | ADYDFWSGYFDY |
| light | | RASQDIRNDLG | AASRLQS | LQHNSYPWT |
| heavy | U1-22 | GGSISSGDYYWS | YIYYSGSTYYNPSLKS | ADYDFWSGYFDY |
| light | | RASQGIRNDLG | AASSLQN | LQHNSYPWT |
| heavy | U1-23 | GGSISSGDYYWS | YIYYSGSTYYNPSLKS | ADYDFWSGYFDY |
| light | | RASQGIRNDLG | AASSLQS | LQHNSYPWT |
| heavy | U1-24 | GGSISSGDYYWS | YIYYSGSTYYNPSLKS | ADYDFWNGYFDY |
| light | | RASQGIRNDLG | AASSLQS | LQHNNYPWT |
| heavy | U1-25 | GGSISSGDYYWS | YIYYSGSTYYNPSLKS | ADYDFWSGYFDY |
| light | | RASQGIRNDLG | AASSLQN | LQHNSYPWT |
| heavy | U1-26 | GGSISSGDYYWS | YIYYSGSTYYNPSLKS | ADYDFWSGYFDF |
| light | | RASQGIRNDLG | AASSLQS | LQHNGYPWT |
| heavy | U1-27 | GGSISSGDYYWS | YIYYSGSTYYNPSLKS | ADYDFWSGYFDF |
| light | | RASQGIRNDLG | AASSLQS | LQHNGYPWT |
| heavy | U1-28 | GGSISSGDYYWS | YIYYSGSTYYNPSLKS | ADYDFWSGYFDS |
| light | | RASQGIRNDLG | AASSLQS | LQHNGYPWT |
| heavy | U1-29 | GFTFNSYDMH | VIWYDGSNKYYADSVKG | DRLCTNGVCYEDYGMDV |
| light | | QASQDISNYLN | DASNLET | QHYDTLPLT |
| heavy | U1-30 | GGSISSGDYYWS | YIYYSGTTYYNPSLKS | ADYDFWSGYFDY |
| light | | RAGQGIRNDLG | AASSLQS | LQHNSYPWT |
| heavy | U1-31 | GYTFTNYGIS | WISAYDGYRNYAQKLQG | DVQDYGDYDYFDY |
| light | | RASQSISSYLN | AASSLQS | QQSYSTPIT |
| heavy | U1-32 | GGSISSGDYYWS | YIYYSGTTYYNPSLKS | ADYDFWSGYFDY |
| light | | RAGQGIRNDLG | AASSLQS | LQHNSYPWT |
| heavy | U1-33 | GGSISSGDYYWS | YIYYSGSTYYNPSLKS | ADYDFWSGHFDC |
| light | | RASQGIRDDLG | AESSLQS | LQHHSYPWT |
| heavy | U1-34 | GYTFTNYGIS | WISAYDGYRNYAQKLQG | DVQDYGDYDYFDY |
| light | | RASQSISSYLN | AASSLQS | QQSYSTPIT |
| heavy | U1-35 | GFTFSDYYMS | YISSSGNNIYHADSVKG | ERYSGYDDPDGFDI |
| light | | QASQDISNYLS | DASNLET | QQYDNPPCS |
| heavy | U1-36 | GGSISSGYYYWS | YIYYSGTTYYNPSFKS | ADYDFWSGHFDY |
| light | | RASQGIRNDLG | AASSLQS | LQHNSYPWT |
| heavy | U1-37 | GYTFTSYGIS | WISAYDGHTNYAQKLQG | DPHDYSNYEAFDF |
| light | | RASQSISSYLN | AASSLQS | QQSYSTPIT |
| heavy | U1-38 | GFSLSTSGVGVG | LIYWNDDKRYSPSLKS | RDEVRGFDY |
| light | | RSSQSLVYSDGYTYLH | KVSNWDS | MQGAHWPIT |
| heavy | U1-39 | GFTVSSNYMS | VIYSGGSTYYADSVKG | GQWLDV |
| light | | RSSQSLLHSNGYNYLD | LGFHRAS | RQALQTPLT |
| heavy | U1-40 | GGSISSGGYYWS | YIYSSGSTYYNPSLKS | DRELELYYYYYGMDV |
| light | | RSSQSLLYSNGYNYLD | LGSNRAS | MQALQTPLT |
| heavy | U1-41 | GGSISSGGYYWS | YIYYSGSTYYNPSLKS | DRELEGYSNYYGVDV |
| light | | RASQAISNYLN | AASSLQS | QQNNSLPIT |
| heavy | U1-42 | GYSFTSYWIG | IIYPGDSDTRYSPSFQG | HENYGDYNY |
| light | | RASQSIRSYLN | AASSLQS | QQSNGSPLT |
| heavy | U1-43 | GGSISSGGYYWS | YIYYSGSTYYNPSLRS | DREREWDDYGDPQGMDV |
| light | | RASQSISSYLH | AASSLQS | QQSYSNPLT |
| heavy | U1-44 | GYSFTSYWIG | IIWPGDSDTIYSPSFQG | HENYGDYNY |
| light | | RASQSIRSYLN | AASSLQS | QQSISSPLT |
| heavy | U1-45 | GYTFTSYDIN | WMNPNSGDTGYAQVFQG | FGDLPYDYSYYEWFDP |
| light | | RASQSISSYLN | AASSLQS | QQSYSTPLT |
| heavy | U1-46 | GDSVSSNSAAWN | | DLYDFWSGYPYYYGMDV |
| light | | sequence not available | | |
| heavy | U1-47 | GDSVSSNSAAWN | | DYYGSGSFYYYYGMDV |
| light | | RASQSISSYLN | AASNLQS | QQSYSTPRT |
| heavy | U1-48 | GGSISSYYWS | HIYTSGSTNYNPSLKS | EAIFGVGPYYYYGMDV |
| light | | sequence not available | | |
| heavy | U1-49 | GYTFTGYYMH | WINPNIGGTNCAQKFQG | GGRYSSSWSYYYYGMDV |
| light | | KSSQSLLLSDGGTYLY | EVSNRFS | MQSMQLPIT |
| heavy | U1-50 | GGSVSSGGYYWS | YIYYSGSTNYNPSLKS | GGDSNYEDYYYYYGMDV |
| light | | RASQSISIYLH | AASSLQS | QQSYTSPIT |
| heavy | U1-51 | GGSISSYYWS | YIYYSGSTNYNPSLKS | |
| light | | KSSQSVLYSSNNKNYLA | WASTRES | QQYYTTPLT |
| heavy | U1-52 | GGSISSGGYYWS | NIYYSGSTYYNPSLKS | GGTGTNYYYYYGMDV |
| light | | RASQSVSSSYLA | GASSWAT | QQYGSSPLT |
| heavy | U1-53 | GFTFSIYSMN | YISSSSSTIYYADSVKG | DRGDFDAFDI |
| light | | QASQDITNYLN | DASNLET | QQCENFPIT |
| heavy | U1-55.1 | GGSVSSGGYYWN | YINYSGSTNYNPSLKS | DRELELYYYYYGMDV |
| light | | will be same as U1-55 | | |
| heavy | U1-55 | will be same as U1-55.1 | | |
| light | | RSSQSLLYSNGYKYLD | LGSNRAS | MQALQTPIT |
| heavy | U1-57.1 | will be same as U1-57 | | |
| light | | RSSQSLLYSNGYKYLD | LGSNRAS | MQALQTPIT |
| heavy | U1-57 | GGSVSSGGYYWN | YINYSGSTNYNPSLKS | DRELELYYYYYGMDV |
| light | | will be same as U1-57.1 | | |
| heavy | U1-58 | GFTFSSYGMH | VIWYDGSNKYYADSVKG | AARLDYYYGMDV |
| light | | RASQSINSYLN | GASGLQS | QQSYSSPLT |
| heavy | U1-59 | GGSFSGYYWS | EINHSGSTNYNPSLKS | DKWTWYFDL |
| light | | RSSQSVLYSSSNRNYLA | WASTRES | QQYYSTPRT |
| heavy | U1-61.1 | GVSISSGGYYWS | YIYYSGSTYYNPSLKS | DSESEYSSSSNYGMDV |
| light | | RASQTISSYLN | AASSLQG | QQSYSNPLT |
| heavy | U1-61 | GVSISSGGYYNS | YIYYSGSTYYNPSLKS | DSESEYSSSSNYGMDV |
| light | | will be the same as U1-61.1 | | |
| heavy | U1-62 | GYSFTSYWIG | IIYPGDSDTRYSPSFQG | QMAGNYYYGMDV |
| light | | RASQSVISIYLA | GASSRAT | QQYGSSPCS |

## Claims

1. An inhibitor of HER-3 for use in the treatment of cancer in combination with radiation treatment selected from external beam radiation therapy and brachytherapy, wherein the inhibitor is an anti-HER3 antibody or an antigen-binding fragment thereof wherein the antibody is directed against the extracellular domain of HER-3 and comprises a CDRH1 as shown in SEQ ID No. 562, a CDRH2 as shown in SEQ ID No. 563, a CDRH3 as shown in SEQ ID No. 564, a CDRL1 as shown in SEQ ID No. 565, a CDRL2 as shown in SEQ ID No. 566 and a CDRL3 as shown in SEQ ID No. 567,
and wherein the inhibitor is administered before radiation treatment.

2. The inhibitor of HER3 for the use according to claim 1, wherein the antibody is a monoclonal antibody, a recombinant antibody, a human antibody, a chimeric antibodyor an antigen-binding fragment thereof.

3. The inhibitor of HER3 for the use according to any of the previous claims, wherein said antibody fragment is a Fab fragment, a Fab' fragment, a F(ab')₂ fragment, a Fv fragment, a diabody, or a single chain antibody molecule.

4. The inhibitor of HER3 for the use according to any of the previous claims, wherein said antibody is coupled to an effector group and/or labelling group, wherein said effector group is preferably a radioisotope, a radionuclide, a toxin, a therapeutic group, and/or a chemotherapeutic group or
wherein said labelling group is preferably a radioisotope or radionuclide, a fluorescent group, an enzymatic group, a chemiluminescent group, a biotinyl group, or a predetermined polypeptide epitope.

5. The inhibitor of HER3 for the use according to any of the previous claims, wherein the cancer is selected from the group consisting of breast cancer, gastrointestinal cancer, pancreas cancer, prostate cancer, ovarian cancer, stomach cancer, endometrial cancer, salivary gland cancer, lung cancer, kidney cancer, colon cancer, colorectal cancer, thyroid cancer, bladder cancer, glioma, melanoma, testis cancer, soft tissue sarcoma, head and neck cancer and formation of tumour metastases, in particular squamous cell carcinoma, wherein the squamous cell carcinoma is preferably squamous cell carcinoma of the lung, of the head and/or of the neck.

6. The inhibitor of HER3 for the use according to any of the previous claims, wherein the radiation treatment is based on a single dose or fractioned dosing of radiation.

7. The inhibitor of HER3 for the use according to any of the previous claims, in combination with a further active compound such as a chemotherapeutic compound, such as cytotoxic agents, such as doxorubicin, cis-platin or carboplatin, cytokines or antineoplastic agents.

8. The inhibitor of HER3 for the use according to any of the previous claims, wherein the antibody comprises the heavy chain amino acid sequence of SEQ ID NO:70 and the light chain amino acid sequence of SEQ ID NO:72

## Patentansprüche

1. Inhibitor von HER-3 zur Verwendung bei der Behandlung von Krebs in Kombination mit einer Strahlenbehandlung, ausgewählt aus externer Strahlentherapie und Brachytherapie, wobei der Inhibitor ein Anti-HER3-Antikörper oder ein Antigen-bindendes Fragment davon ist, wobei der Antikörper gegen die extrazelluläre Domäne von HER-3 gerichtet ist und eine CDRH1, wie in SEQ ID Nr. 562 gezeigt, eine CDRH2, wie in SEQ ID Nr. 563 gezeigt, eine CDRH3, wie in SEQ ID Nr. 564 gezeigt, eine CDRL1, wie in SEQ ID Nr. 565 gezeigt, eine CDRL2, wie in SEQ ID Nr. 566 gezeigt, und eine CDRL3, wie in SEQ ID Nr. 567 gezeigt, umfasst,
und wobei der Inhibitor vor einer Strahlenbehandlung verabreicht wird.

2. Inhibitor von HER3 zur Verwendung nach Anspruch 1, wobei der Antikörper ein monoklonaler Antikörper, ein rekombinanter Antikörper, ein humaner Antikörper, ein chimärer Antikörper oder ein Antigen-bindendes Fragment davon ist.

3. Inhibitor von HER3 zur Verwendung nach einem der vorhergehenden Ansprüche, wobei das Antikörperfragment ein Fab-Fragment, ein Fab'-Fragment, ein F(ab')₂-Fragment, ein Fv-Fragment, ein Diabody oder ein einkettiges Antikörpermolekül ist.

4. Inhibitor von HER3 zur Verwendung nach einem der vorhergehenden Ansprüche, wobei der Antikörper an eine Effektorgruppe und/oder Markierungsgruppe gekoppelt ist, wobei die Effektorgruppe vorzugsweise ein Radioisotop, ein Radionuklid, ein Toxin, eine therapeutische Gruppe und/oder eine chemotherapeutische Gruppe ist oder wobei die Markierungsgruppe vorzugsweise ein Radioisotop oder Radionuklid, eine fluoreszierende Gruppe, eine enzymatische Gruppe, eine chemilumineszierende Gruppe, eine Biotinylgruppe oder ein vorbestimmtes Polypeptidepitop ist.

5. Inhibitor von HER3 zur Verwendung nach einem der vorhergehenden Ansprüche, wobei der Krebs ausgewählt ist aus der Gruppe bestehend aus Brustkrebs, Gastrointestinal-Krebs, Bauchspeicheldrüsenkrebs, Prostatakrebs, Eierstockkrebs, Magenkrebs, Endometriumkrebs, Speicheldrüsenkrebs, Lungenkrebs, Nierenkrebs, Dickdarmkrebs, Kolorektalkrebs, Schilddrüsenkrebs, Blasenkrebs, Gliom, Melanom, Hodenkrebs, Weichgewebesarkom, Kopf- und Halskrebs und Bildung von Tumormetastasen, insbesondere Plattenepithelkarzinom, wobei das Plattenepithelkarzinom vorzugsweise ein Plattenepithelkarzinom der Lunge, des Kopfes und/oder des Halses ist.

6. Inhibitor von HER3 zur Verwendung nach einem der vorhergehenden Ansprüche, wobei die Strahlenbehandlung auf einer Einzeldosis oder einer fraktionierten Dosierung von Strahlung basiert.

7. Inhibitor von HER3 zur Verwendung nach einem der vorhergehenden Ansprüche in Kombination mit einem weiteren Wirkstoff, wie zum Beispiel einer chemotherapeutischen Verbindung, wie zytotoxischen Mitteln, wie Doxorubicin, cis-Platin oder Carboplatin, Cytokinen oder antineoplastischen Mitteln.

8. Inhibitor von HER3 zur Verwendung nach einem der vorhergehenden Ansprüche, wobei der Antikörper die Aminosäuresequenz der schweren Kette von SEQ ID NO:70 und die Aminosäuresequenz der leichten Kette von SEQ ID NO:72 umfasst.

## Revendications

1. Inhibiteur de HER-3 destiné à être utilisé dans le traitement du cancer en combinaison avec un traitement par rayonnement choisi parmi une radiothérapie à faisceau externe et une curiethérapie, où l'inhibiteur est un anticorps anti-HER3 ou un fragment de liaison à l'antigène de celui-ci où l'anticorps est dirigé contre le domaine extracellulaire de HER-3 et comprend une CDRH1 comme montré dans SEQ ID N° 562, une CDRH2 comme montré dans SEQ ID N° 563, une CDRH3 comme montré dans SEQ ID N° 564, une CDRL1 comme montré dans SEQ ID N° 565, une CDRL2 comme montré dans SEQ ID N° 566 et une CDRL3 comme montré dans SEQ ID N° 567,
et où l'inhibiteur est administré avant le traitement par rayonnement.

2. Inhibiteur de HER3 destiné à être utilisé selon la revendication 1, où l'anticorps est un anticorps monoclonal, un anticorps recombinant, un anticorps humain, un anticorps chimérique ou un fragment de liaison à l'antigène de celui-ci.

3. Inhibiteur de HER3 destiné à être utilisé selon l'une quelconque des revendications précédentes, où
ledit fragment d'anticorps est un fragment Fab, un fragment Fab', un fragment F(ab')₂, un fragment Fv, un diabody ou une molécule d'anticorps à une seule chaîne.

4. Inhibiteur de HER3 destiné à être utilisé selon l'une quelconque des revendications précédentes, où ledit anticorps est couplé à un groupe effecteur et/ou un groupe de marquage, où
ledit groupe effecteur est de préférence un radio-isotope, un radionucléide, une toxine, un groupe thérapeutique et/ou un groupe chimiothérapeutique ou bien
où ledit groupe de marquage est de préférence un radio-isotope ou radionucléide, un groupe fluorescent, un groupe enzymatique, un groupe chimiluminescent, un groupe biotinyle ou un épitope polypeptidique prédéterminé.

5. Inhibiteur de HER3 destiné à être utilisé selon l'une quelconque des revendications précédentes, où
le cancer est choisi dans le groupe consistant en le cancer du sein, le cancer gastro-intestinal, le cancer du pancréas, le cancer de la prostate, le cancer de l'ovaire, le cancer de l'estomac, le cancer de l'endomètre, le cancer des glandes salivaires, le cancer du poumon, le cancer du rein, le cancer du côlon, le cancer colorectal, le cancer de la thyroïde, le cancer de la vessie, le gliome, le mélanome, le cancer des testicules, le sarcome des tissus mous, le cancer de la tête et du cou et la formation de métastases tumorales, en particulier le carcinome épidermoïde, où le carcinome épidermoïde est de préférence un carcinome épidermoïde du poumon, de la tête et/ou du cou.

6. Inhibiteur de HER3 destiné à être utilisé selon l'une quelconque des revendications précédentes, où
le traitement par rayonnement est basé sur une dose unique ou une administration de rayonnement fractionnée.

7. Inhibiteur de HER3 destiné à être utilisé selon l'une quelconque des revendications précédentes, en combinaison avec un autre composé actif comme un composé chimiothérapeutique, comme les agents cytotoxiques, comme la doxorubicine, le cis-platine ou le carboplatine, les cytokines ou les agents antinéoplasiques.

8. Inhibiteur de HER3 destiné à être utilisé selon l'une quelconque des revendications précédentes, où
l'anticorps comprend la séquence d'acides aminés de chaîne lourde de SEQ ID NO:70 et la séquence d'acides aminés de chaîne légère de SEQ ID NO:72.


### SEQUENCE LISTING

<110> U3 Pharma AG
<120> HER3 inhibitor for modulating radiosensitivity
<130> 53017P US
<140>
   <141>
<150>
   <151>
<160> 582
<170> PatentIn version 3.3
<210> 1
   <211> 342
   <212> DNA
   <213> Artificial
<220>
   <223> Antibody
<400> 1
   gaggtgcagc tggtggagtc tggaggaggc ttgatccagc ctggggggtc cctgagactc      60
   tcctgtgcag cctctgggtt caccgtcagt agcaactaca tgagctgggt ccgccaggct     120
   ccagggaagg ggctggattg ggtctcagtt atttatagcg gtggtagcac atactacgca     180
   gactccgtga agggccgatt caccatctcc agagacaatt ccaagaacac gctgtatctt     240
   caaatgaaca gcctgagagc cgaggacacg gccgtgtatt actgtgcgag agggcagtgg     300
   ctggacgtct ggggccaagg gaccacggtc accgtctcct ca                        342
<210> 2
   <211> 114
   <212> PRT
   <213> Artificial
<220>
   <223> Antibody
<400> 2
   Glu Val Gln Leu Val Glu Ser Gly Gly Gly Leu Ile Gln Pro Gly Gly
   1               5                   10                  15
   Ser Leu Arg Leu Ser Cys Ala Ala Ser Gly Phe Thr Val Ser Ser Asn
               20                  25                  30
   Tyr Met Ser Trp Val Arg Gln Ala Pro Gly Lys Gly Leu Asp Trp Val
           35                  40                  45
   Ser Val Ile Tyr Ser Gly Gly Ser Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Gly Gln Trp Leu Asp Val Trp Gly Gln Gly Thr Thr Val Thr Val
               100                 105                 110
   Ser Ser
<210> 3
   <211> 336
   <212> DNA
   <213> Artificial
<220>
   <223> Antibody
<400> 3
   gatattgtga tgactcagtc tccactctcc ctgcccgtca cccctggaga gccggcctcc      60
   atctcctgca ggtcaagtca gagcctcctg catagtaatg gatacaacta tttggattgg     120
   tacctgcaga ggccagggca gtctccacaa ctcctgttct atttgggttt tcatcgggcc     180
   tccggggtcc ctgacaggtt cagtggcagt ggatcaggca cagattttac actgaaaatc     240
   agcagagtgg aggctgagga tgttggggtt tattactgca ggcaagctct acaaactccg     300
   ctcactttcg gcggagggac caaggtggag atcaaa                               336
<210> 4
   <211> 112
   <212> PRT
   <213> Artificial
<220>
   <223> Antibody
<400> 4
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Arg Pro Gly Gln Ser
           35                  40                  45
   Pro Gln Leu Leu Phe Tyr Leu Gly Phe His Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Arg Gln Ala
                   85                  90                  95
   Leu Gln Thr Pro Leu Thr Phe Gly Gly Gly Thr Lys Val Glu Ile Lys
               100                 105                 110
<210> 5
   <211> 374
   <212> DNA
   <213> Artificial
<220>
   <223> Antibody
<400> 5
   caggtgcagc tgcaggagtc gggcccagga ctggtgaagc cttcacagac cctgtccctc      60
   acctgtactg tctctggtgg ctccatcagc agtggtggtt actactggag ctggatccgc     120
   cagcacccag ggaagggcct ggagtggatt gggtacatct attccagtgg gagcacctac     180
   tacaacccgt ccctcaagag tcgagttacc atatcagtag acacgtctaa gaaccagttc     240
   tccctgaagc tgagctctgt gactgccgcg gacacggccg tgtattactg tgcgagagat     300
   agggaactgg aactttacta ctactactac ggtatggacg tctggggcca agggaccacg     360
   gtcaccgtct cctc                                                       374
<210> 6
   <211> 124
   <212> PRT
   <213> Artificial
<220>
   <223> Antibody
<400> 6
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile Tyr Ser Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Asp Arg Glu Leu Glu Leu Tyr Tyr Tyr Tyr Tyr Gly Met
               100                 105                 110
   Asp Val Trp Gly Gln Gly Thr Thr Val Thr Val Ser
           115                 120
<210> 7
   <211> 336
   <212> DNA
   <213> Artificial
<220>
   <223> Antibody
<400> 7
   gatattgtga tgactcagtc tccactctcc ctgcccgtca cccctggaga gccggcctcc      60
   atctcctgca ggtctagtca gagcctcctg tatagtaatg gatacaacta tttggattgg     120
   tacctgcaga agccagggca gtctccacag ctcctgatct atttgggttc taatcgggcc     180
   tccggggtcc ctgacaggtt cagtggcagt ggatcaggca cagattttac actgaaaatc     240
   agcagagtgg aggctgagga tgttgggatt tattactgca tgcaagctct acaaactccg     300
   ctcactttcg gcggagggac caaggtggag atcaaa                               336
<210> 8
   <211> 112
   <212> PRT
   <213> Artificial
<220>
   <223> Antibody
<400> 8
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu Tyr Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Ile Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 9
   <211> 357
   <212> DNA
   <213> Artificial
<220>
   <223> Antibody
<400> 9
   cagatcacct tgaaggagtc tggtcctacg ctggtgaaac ccacacagac cctcacgctg 60
   acctgcacct tctctgggtt ctcactcagc actagtggag tgggtgtggg ctggatccgt 120
   cagcccccag gaaaggccct ggactggctt gcactcattt attggaatga tgataagcgc 180
   tacagcccat ctctgaagag caggctcacc atcaccaagg acacctccaa aaaccaggtg 240
   gtccttacaa tgaccaacat ggatcttgtg gacacagcca catattactg tgtacacaga 300
   gacgaagttc gagggtttga ctactggggc cagggaaccc tggtcaccgt ctcctca 357
<210> 10
   <211> 119
   <212> PRT
   <213> Artificial
<220>
   <223> Antibody
<400> 10
   Gln Ile Thr Leu Lys Glu Ser Gly Pro Thr Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Asp
           35                  40                  45
   Trp Leu Ala Leu Ile Tyr Trp Asn Asp Asp Lys Arg Tyr Ser Pro Ser
       50                  55                  60
   Leu Lys Ser Arg Leu Thr Ile Thr Lys Asp Thr Ser Lys Asn Gln Val
   65                  70                  75                  80
   Val Leu Thr Met Thr Asn Met Asp Leu Val Asp Thr Ala Thr Tyr Tyr
                   85                  90                  95
   Cys Val His Arg Asp Glu Val Arg Gly Phe Asp Tyr Trp Gly Gln Gly
               100                 105                 110
   Thr Leu Val Thr Val Ser Ser
           115
<210> 11
   <211> 336
   <212> DNA
   <213> Artificial
<220>
   <223> Antibody
<400> 11
   gatgttgtga tgactcagtc tccactctcc ctgcccgtca cccttggaca gccggcctcc      60
   atctcctgca ggtctagtca aagcctcgta tacagtgatg gatacaccta cttgcattgg     120
   tttcagcaga ggccaggcca atctccaagg cgccttattt ataaggtttc taactgggac     180
   tctggggtcc cagacagatt cagcggcagt gggtcaggca ctgatttcac actgaaaatc     240
   agcagggtgg aggctgagga tgttggggtt tattactgca tgcaaggtgc acactggccg     300
   atcaccttcg gccaagggac acgactggag attaaa                               336
<210> 12
   <211> 112
   <212> PRT
   <213> Artificial
<220>
   <223> Antibody
<400> 12
   Asp Val Val Met Thr Gln Ser Pro Leu Ser Leu Pro Val Thr Leu Gly
   1               5                   10                  15
   Gln Pro Ala Ser Ile Ser Cys Arg Ser Ser Gln Ser Leu Val Tyr Ser
               20                  25                  30
   Asp Gly Tyr Thr Tyr Leu His Trp Phe Gln Gln Arg Pro Gly Gln Ser
           35                  40                  45
   Pro Arg Arg Leu Ile Tyr Lys Val Ser Asn Trp Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Ala His Trp Pro Ile Thr Phe Gly Gln Gly Thr Arg Leu Glu Ile Lys
               100                 105                 110
<210> 13
   <211> 374
   <212> DNA
   <213> Artificial
<220>
   <223> Antibody
<400> 13
   caggtgcagc tgcaggagtc gggcccagga ctggtgaagc cttcacagac cctgtccctc      60
   acctgcactg tctctggtgg ctccatcagc agtggtgggt actactggag ctggatccgc     120
   cagcacccag ggaagggcct ggagtggatt gggtacatct attacagtgg gagcacctac     180
   tacaacccgt ccctcaagag tcgagttacc atatcagtag acacgtctaa gaaccagttc     240
   tccctgaagc tgagctctgt gactgccgcg gacacggccg tgtatttctg tgcgagagat     300
   cgggaacttg agggttactc caactactac ggtgtggacg tctggggcca agggaccacg     360
   gtcaccgtct cctc                                                       374
<210> 14
   <211> 124
   <212> PRT
   <213> Artificial
<220>
   <223> Antibody
<400> 14
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Phe
                   85                  90                  95
   Cys Ala Arg Asp Arg Glu Leu Glu Gly Tyr Ser Asn Tyr Tyr Gly Val
               100                 105                 110
   Asp Val Trp Gly Gln Gly Thr Thr Val Thr Val Ser
           115                 120
<210> 15
   <211> 321
   <212> DNA
   <213> Artificial
<220>
   <223> Antibody
<400> 15
   gacatccaga tgacccagtc tccatcctcc ctgtctgcat ctgtaggaga cagagtcacc     60
   atcacttgcc gggcaagtca ggccattagc aactatttaa attggtatca gcagaaacca    120
   gggaaagccc ctaagctcct gatctatgct gcatccagtt tgcaaagtgg ggtcccatca    180
   aggttcagtg gcagtggatc tgggacagat ttcactctca ccatcagcag cctgcagcct    240
   gaagattttg caacttatta ctgtcaacag aataatagtc tcccgatcac cttcggccaa    300
   gggacacgac tggagattaa a                                              321
<210> 16
   <211> 107
   <212> PRT
   <213> Artificial
<220>
   <223> Antibody
<400> 16
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ala Ile Ser Asn Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Asn Asn Ser Leu Pro Ile
                   85                  90                  95
   Thr Phe Gly Gln Gly Thr Arg Leu Glu Ile Lys
               100                 105
<210> 17
   <211> 354
   <212> DNA
   <213> Artificial
<220>
   <223> Antibody
<400> 17
   gaggtgcagc tggtgcagtc tggagcagag gtgaaaaagc ccggggagtc tctgaagatc      60
   tcctgtaagg gttctggata cagctttacc agctactgga tcggctgggt gcgccagatg     120
   cccgggaaag gcctggagtg gatggggatc atctatcctg gtgactctga taccagatac     180
   agcccgtcct tccaaggcca ggtcaccatc tcagccgaca agtccatcag caccgcctac     240
   ctgcagtgga gcagcctgaa ggcctcggac accgccatgt attactgtgc gagacatgaa     300
   aactacggtg actacaacta ctggggccag ggaaccctgg tcaccgtctc ctca           354
<210> 18
   <211> 118
   <212> PRT
   <213> Artificial
<220>
   <223> Antibody
<400> 18
   Glu Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Ser Tyr
               20                  25                  30
   Trp Ile Gly Trp Val Arg Gln Met Pro Gly Lys Gly Leu Glu Trp Met
           35                  40                  45
   Gly Ile Ile Tyr Pro Gly Asp Ser Asp Thr Arg Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg His Glu Asn Tyr Gly Asp Tyr Asn Tyr Trp Gly Gln Gly Thr
               100                 105                 110
   Leu Val Thr Val Ser Ser
           115
<210> 19
   <211> 321
   <212> DNA
   <213> Artificial
<220>
   <223> Antibody
<400> 19
   gacatccaga tgacccagtc tccatcctcc ctgtctgcat ctgtcggaga cagagtcacc     60
   atcacttgcc gggcaagtca gagcattcgc agctatttaa attggtatca gcagaaacca    120
   gggaaagccc ctaagctcct gatctatgct gcttccagtt tgcaaagtgg ggtcccatca    180
   aggttcagtg gcagtggatc tgggacagat ttcactctca ccatcagcag tctgcaacct    240
   gaagattttg cactttactg ctgtcaacag agtaacggtt ccccgctcac tttcggcgga    300
   gggaccaagg tggagatcaa a                                              321
<210> 20
   <211> 107
   <212> PRT
   <213> Artificial
<220>
   <223> Antibody
<400> 20
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Arg Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Leu Tyr Cys Cys Gln Gln Ser Asn Gly Ser Pro Leu
                   85                  90                  95
   Thr Phe Gly Gly Gly Thr Lys Val Glu Ile Lys
               100                 105
<210> 21
   <211> 380
   <212> DNA
   <213> Artificial
<220>
   <223> Antibody
<400> 21
   caggtgcagc tgcaggagtc gggcccagga ctggtgaagc cttcacagac cctgtccctc     60
   acctgcactg tctctggtgg ctccatcagc agtggtggtt actactggag ctggatccgc    120
   cagcacccag ggaagggcct ggagtggatt gggtacatct attacagtgg gagcacctac    180
   tacaacccgt ccctcaggag tcgagttacc atatcagtag acacgtctaa gaaccagttc    240
   tccctgaagc tgagctctgt gactgccgcg gacacggccg tgtattactg tgcgagagat    300
   agagagagag agtgggatga ttacggtgac ccccaaggta tggacgtctg gggccaaggg    360
   accacggtca ccgtctcctc                                                380
<210> 22
   <211> 126
   <212> PRT
   <213> Artificial
<220>
   <223> Antibody
<400> 22
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Arg Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Asp Arg Glu Arg Glu Trp Asp Asp Tyr Gly Asp Pro Gln
               100                 105                 110
   Gly Met Asp Val Trp Gly Gln Gly Thr Thr Val Thr Val Ser
           115                 120                 125
<210> 23
   <211> 321
   <212> DNA
   <213> Artificial
<220>
   <223> Antibody
<400> 23
   gacatccaga tgacccagtc tccatcctcc ctgtctgcat ctgtaggaga cagagtcacc      60
   atcacttgcc gggcaagtca gagcattagc agctatttac attggtatca gcagaaacca     120
   gggaaagccc ctaagctcct gatccatgct gcatccagtt tacaaagtgg ggtcccatca     180
   aggttcagtg gcagtggatc tgggacagat ttcactctca ccatcagtag tctgcaacct     240
   gaagattttg caacttacta ctgtcaacag agttacagta acccgctcac tttcggcgga     300
   gggaccaagg tggagatcca a                                               321
<210> 24
   <211> 107
   <212> PRT
   <213> Artificial
<220>
   <223> Antibody
<400> 24
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu His Trp Tyr Gln Gln Lys Pro Gly Lys Ala Pro Lys Leu Leu Ile
           35                  40                  45
   His Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Asn Pro Leu
                   85                  90                  95
   Thr Phe Gly Gly Gly Thr Lys Val Glu Ile Gln
               100                 105
<210> 25
   <211> 354
   <212> DNA
   <213> Artificial
<220>
   <223> Antibody
<400> 25
   gaggtgcagc tggtgcagtc tggagcagag gtgaaaaagc ccggggagtc tctgaagatc      60
   tcctgtaagg gttctggata cagctttacc agctactgga tcggctgggt gcgccagatg     120
   cccgggaaag gcctggagtg gatggggatc atctggcctg gtgactctga taccatatac     180
   agcccgtcct tccaaggcca ggtcaccatc tcagccgaca agtccatcag caccgcctac     240
   ctgcagtgga gcagcctgaa ggcctcggac accgccatgt attactgtgc gagacatgaa     300
   aactacggtg actacaacta ctggggccag ggaaccctgg tcaccgtctc ctca           354
<210> 26
   <211> 118
   <212> PRT
   <213> Artificial
<220>
   <223> Antibody
<400> 26
   Glu Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Ser Tyr
               20                  25                  30
   Trp Ile Gly Trp Val Arg Gln Met Pro Gly Lys Gly Leu Glu Trp Met
           35                  40                  45
   Gly Ile Ile Trp Pro Gly Asp Ser Asp Thr Ile Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg His Glu Asn Tyr Gly Asp Tyr Asn Tyr Trp Gly Gln Gly Thr
               100                 105                 110
   Leu Val Thr Val Ser Ser
           115
<210> 27
   <211> 321
   <212> DNA
   <213> Artificial
<220>
   <223> Antibody
<400> 27
   gacatccaga tgacccagtc tccatcctcc ctgtctgcat ctgtgggaga cagagtcacc      60
   atcacttgcc gggcaagtca gagcattcga agttatttaa attggtatca gcagaaaccg     120
   gggaatgccc ctaaactcct gatctatgct gcatccagtt tgcaaagtgg ggtcccatca     180
   aggttcagtg gcagtggatc tgggacagat ttcactctca ccatcagcag tctgcaacct     240
   gaagattttg cactttacta ctgtcaacag agtatcagtt ccccgctcac tttcggcgga     300
   gggaccaagg tggagatcaa a                                               321
<210> 28
   <211> 107
   <212> PRT
   <213> Artificial
<220>
   <223> Antibody
<400> 28
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Arg Ser Tyr
               20                  25                  30
   Leu Asn Trp Tyr Gln Gln Lys Pro Gly Asn Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Leu Tyr Tyr Cys Gln Gln Ser Ile Ser Ser Pro Leu
                   85                  90                  95
   Thr Phe Gly Gly Gly Thr Lys Val Glu Ile Lys
               100                 105
<210> 29
   <211> 374
   <212> DNA
   <213> Artificial
<220>
   <223> Antibody
<400> 29
   caggtgcagc tggtgcagtc tggggctgag gtgaagaagc ctggggcctc agtgaaggtc 60
   tcctgcaagg cttctggata caccttcacc agttatgata tcaactgggt gcgacaggcc 120
   actggacaag ggcttgagtg gatgggatgg atgaacccta acagtggtga cactggctat 180
   gcacaggtgt tccagggcag agtcaccatg acctggaaca cctccataag cacagcctac 240
   atggaactga gcagcctgag atctgaggac acggccgtgt attactgtgc gagatttggg 300
   gatctcccgt atgactacag ttactacgaa tggttcgacc cctggggcca gggaaccctg 360
   gtcaccgtct cctc 374
<210> 30
   <211> 124
   <212> PRT
   <213> Artificial
<220>
   <223> Antibody
<400> 30
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Ser Tyr
               20                  25                  30
   Asp Ile Asn Trp Val Arg Gln Ala Thr Gly Gln Gly Leu Glu Trp Met
           35                  40                  45
   Gly Trp Met Asn Pro Asn Ser Gly Asp Thr Gly Tyr Ala Gln Val Phe
       50                  55                  60
   Gln Gly Arg Val Thr Met Thr Trp Asn Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Phe Gly Asp Leu Pro Tyr Asp Tyr Ser Tyr Tyr Glu Trp Phe
               100                 105                 110
   Asp Pro Trp Gly Gln Gly Thr Leu Val Thr Val Ser
           115                 120
<210> 31
   <211> 321
   <212> DNA
   <213> Artificial
<220>
   <223> Antibody
<400> 31
   gacatccaga tgacccagtc tccatcctcc ctgtctgcat ctgtaggaga cagagtcacc      60
   atcacttgcc gggcaagcca gagcattagc agctatttaa attggtatca gcagagacca     120
   gggaaagccc ctaagctcct gatctatgca gcatccagtt tgcaaagtgg ggtcccatca     180
   aggttcagtg gcagtggatc tgggacagat ttcactctca ccatcagcag tctgcaacct     240
   gaagattttg caacttacta ctgtcaacag agttacagta ccccgctcac tttcggcgga     300
   gggaccaagg tggagatcaa a                                               321
<210> 32
   <211> 107
   <212> PRT
   <213> Artificial
<220>
   <223> Antibody
<400> 32
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Arg Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Leu
                   85                  90                  95
   Thr Phe Gly Gly Gly Thr Lys Val Glu Ile Lys
               100                 105
<210> 33
   <211> 386
   <212> DNA
   <213> Artificial
<220>
   <223> Antibody
<400> 33
   caggtacagc tgcagcagtc aggtccagga ctggtgaagc cctcgcagac cctctcactc      60
   acctgtgcca tctccgggga cagtgtctct agcaacagtg ctgcttggaa ctggatcagg     120
   cagtccccat cgagaggcct tgagtggctg ggaaggacat actacaggtc caagtggtat     180
   aatgattatg cagtatctgt gaaaagtcga ataaccatca acccagacac atccaagaac     240
   cagttctccc tgcagctgaa ctctgtgact cccgaggaca cggctgtgta ttactgtgca     300
   agagatctct acgatttttg gagtggttat ccctactact acggtatgga cgtctggggc     360
   caagggacca cggtcaccgt ctcctc                                          386
<210> 34
   <211> 128
   <212> PRT
   <213> Artificial
<220>
   <223> Antibody
<400> 34
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Asn Trp Ile Arg Gln Ser Pro Ser Arg Gly Leu Glu
           35                  40                  45
   Trp Leu Gly Arg Thr Tyr Tyr Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Asp Leu Tyr Asp Phe Trp Ser Gly Tyr Pro Tyr
               100                 105                 110
   Tyr Tyr Gly Met Asp Val Trp Gly Gln Gly Thr Thr Val Thr Val Ser
           115                 120                 125
<210> 35
   <211> 383
   <212> DNA
   <213> Artificial
<220>
   <223> Antibody
<400> 35
   caggtacagc tgcagcagtc aggtccagga ctggtgaagc cctcgcagac cctctcactc     60
   acctgtgcca tctccgggga cagtgtctct agcaacagtg ctgcttggaa ctggatcagg    120
   cagtccccat cgagaggcct tgagtggctg ggaaggacat actacaggtc caagtggtat    180
   aatgattatg cagtatctgt gaaaagtcga ataaccatca acccagacac atccaagaac    240
   cagttctccc tgcagctgaa ctctgtgact cccgaggaca cggctgtgta ttactgtgca    300
   agagattact atggttcggg gagtttctac tactactacg gtatggacgt ctggggccaa    360
   gggaccacgg tcaccgtctc ctc                                            383
<210> 36
   <211> 127
   <212> PRT
   <213> Artificial
<220>
   <223> Antibody
<400> 36
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Asn Trp Ile Arg Gln Ser Pro Ser Arg Gly Leu Glu
           35                  40                  45
   Trp Leu Gly Arg Thr Tyr Tyr Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Asp Tyr Tyr Gly Ser Gly Ser Phe Tyr Tyr Tyr
               100                 105                 110
   Tyr Gly Met Asp Val Trp Gly Gln Gly Thr Thr Val Thr Val Ser
           115                 120                 125
<210> 37
   <211> 321
   <212> DNA
   <213> Artificial
<220>
   <223> Antibody
<400> 37
   gacatccaga tgacccagtc tccatcctcc ctgtctgcat ctgtaggaga cagagtcacc     60
   atcacttgcc gggcaagtca gagcattagc agctatttaa attggtatca gcagaaacca    120
   gggaaagccc ctaaggtcct gatctatgct gcatccaatt tgcaaagtgg ggtcccatca    180
   aggttcagtg gcagtggatc tgggacagat ttcactctca ccatcagcag tctgcaacct    240
   gaagattttg caacttacta ctgtcaacag agttacagta cccctcggac gttcggccaa    300
   gggaccaagg tggaaatcaa a                                              321
<210> 38
   <211> 107
   <212> PRT
   <213> Artificial
<220>
   <223> Antibody
<400> 38
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Val Leu Ile
           35                  40                  45
   Tyr Ala Ala Ser Asn Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Arg
                   85                  90                  95
   Thr Phe Gly Gln Gly Thr Lys Val Glu Ile Lys
               100                 105
<210> 39
   <211> 371
   <212> DNA
   <213> Artificial
<220>
   <223> Antibody
<400> 39
   caggtgcagc tgcaggagtc gggcccagga ctggtgaagc cttcggagac cctgtccctc     60
   acctgcactg tctctggtgg ctccatcagt agttactact ggagctggat ccggcagccc    120
   gccgggaagg gactggagtg gattgggcat atctatacca gtgggagcac caactacaac    180
   ccctccctca agagtcgagt caccatgtca gtagacacgt ccaagaacca gttctccctg    240
   aagctgagct ctgtgaccgc cgcggacacg gccgtgtatt actgtgcgag agaagcgatt    300
   tttggagtgg gcccctacta ctactacggt atggacgtct ggggccaagg gaccacggtc    360
   accgtctcct c                                                         371
<210> 40
   <211> 123
   <212> PRT
   <213> Artificial
<220>
   <223> Antibody
<400> 40
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Tyr
               20                  25                  30
   Tyr Trp Ser Trp Ile Arg Gln Pro Ala Gly Lys Gly Leu Glu Trp Ile
           35                  40                  45
   Gly His Ile Tyr Thr Ser Gly Ser Thr Asn Tyr Asn Pro Ser Leu Lys
       50                  55                  60
   Ser Arg Val Thr Met Ser Val Asp Thr Ser Lys Asn Gln Phe Ser Leu
   65 70 75 80
   Lys Leu Ser Ser Val Thr Ala Ala Asp Thr Ala Val Tyr Tyr Cys Ala
   85 90 95
   Arg Glu Ala Ile Phe Gly Val Gly Pro Tyr Tyr Tyr Tyr Gly Met Asp
   100 105 110
   Val Trp Gly Gln Gly Thr Thr Val Thr Val Ser
   115 120
<210> 41
   <211> 377
   <212> DNA
   <213> Artificial
<220>
   <223> Antibody
<400> 41
   caggtgcagc tggtgcagtc tggggctgag gtgaagaagc ctggggcctc agtgaaggtc 60
   tcctgcaagg cttctggata caccttcacc ggctactata tgcactgggt gcgacaggcc 120
   cctggacaag ggcttgagtg gatgggatgg atcaacccta atattggtgg cacaaactgt 180
   gcacagaagt ttcagggcag ggtcaccatg accagggaca cgtccatcag cacagcctac 240
   atggagctga gcaggctgag atctgacgac acggccgtgt attactgtgc gagaggggga 300
   cggtatagca gcagctggtc ctactactac tacggtatgg acgtctgggg ccaagggacc 360
   acggtcaccg tctcctc 377
<210> 42
   <211> 125
   <212> PRT
   <213> Artificial
<220>
   <223> Antibody
<400> 42
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ile Gly Gly Thr Asn Cys Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Gly Gly Arg Tyr Ser Ser Ser Trp Ser Tyr Tyr Tyr Tyr Gly
               100                 105                 110
   Met Asp Val Trp Gly Gln Gly Thr Thr Val Thr Val Ser
           115                 120                 125
<210> 43
   <211> 336
   <212> DNA
   <213> Artificial
<220>
   <223> Antibody
<400> 43
   gatattctga tgacccagac tccactctct ctgtccgtca cccctggaca gccggcctcc     60
   atctcctgca agtctagtca gagcctcctg cttagtgatg gagggaccta tttgtattgg    120
   tacctgcaga agccaggcca gcctccacag ctcctgatct atgaagtttc caaccggttc    180
   tctggagtgc cagataggtt cagtggcagc gggtcaggga cagatttcac actgaaaatc    240
   agccgggtgg aggctgagga tgttggggtt tattactgca tgcaaagtat gcagcttccg    300
   atcaccttcg gccaagggac acgactggaa attaaa                              336
<210> 44
   <211> 112
   <212> PRT
   <213> Artificial
<220>
   <223> Antibody
<400> 44
   Asp Ile Leu Met Thr Gln Thr Pro Leu Ser Leu Ser Val Thr Pro Gly
   1               5                   10                  15
   Gln Pro Ala Ser Ile Ser Cys Lys Ser Ser Gln Ser Leu Leu Leu Ser
               20                  25                  30
   Asp Gly Gly Thr Tyr Leu Tyr Trp Tyr Leu Gln Lys Pro Gly Gln Pro
           35                  40                  45
   Pro Gln Leu Leu Ile Tyr Glu Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ser
                   85                  90                  95
   Met Gln Leu Pro Ile Thr Phe Gly Gln Gly Thr Arg Leu Glu Ile Lys
               100                 105                 110
<210> 45
   <211> 380
   <212> DNA
   <213> Artificial
<220>
   <223> Antibody
<400> 45
   caggtgcagc tgcaggagtc gggcccagga ctggtgaagc cttcggagac cctgtccctc      60
   acctgcactg tctctggtgg ctccgtcagc agtggtggtt actactggag ctggatccgg     120
   cagcccccag ggaagggact ggagtggatt gggtatatct attacagtgg gagcaccaac     180
   tacaacccct ccctcaagag tcgagtcacc atatcagtag acacgtccaa gaaccagttc     240
   tccctgaagc tgagctctgt gaccgctgcg gacacggccg tgtattactg tgcgagaggg     300
   ggggacagta actacgagga ttactactac tactacggta tggacgtctg gggccaaggg     360
   accacggtca ccgtctcctc                                                 380
<210> 46
   <211> 126
   <212> PRT
   <213> Artificial
<220>
   <223> Antibody
<400> 46
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Val Ser Ser Gly
               20                  25                  30
   Gly Tyr Tyr Trp Ser Trp Ile Arg Gln Pro Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Gly Gly Asp Ser Asn Tyr Glu Asp Tyr Tyr Tyr Tyr Tyr
               100                 105                 110
   Gly Met Asp Val Trp Gly Gln Gly Thr Thr Val Thr Val Ser
           115                 120                 125
<210> 47
   <211> 321
   <212> DNA
   <213> Artificial
<220>
   <223> Antibody
<400> 47
   gacatccaga tgacccagtc tccatcctcc ctgtctgcat ctgtaggaga cagagtcacc      60
   atcacttgcc gggcaagtca gagcattagc atctatttac attggtatca gcagaaacca     120
   gggaaagccc ctaagctctt gatctctgct gcatccagtt tgcaaagtgg ggtcccgtca     180
   aggttcagtg gcagtggatc tgggacagat ttcactctca ccatcagaag tctgcaacct     240
   gaagattttg caacttacta ctgtcaacag agttacactt ccccgatcac cttcggccaa     300
   gggacacgac tggagattaa a                                               321
<210> 48
   <211> 107
   <212> PRT
   <213> Artificial
<220>
   <223> Antibody
<400> 48
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ile Tyr
               20                  25                  30
   Leu His Trp Tyr Gln Gln Lys Pro Gly Lys Ala Pro Lys Leu Leu Ile
           35                  40                  45
   Ser Ala Ala Ser Ser Leu Gln Ser Gly Val Pro Ser Arg Phe Ser Gly
       50                  55                  60
   Ser Gly Ser Gly Thr Asp Phe Thr Leu Thr Ile Arg Ser Leu Gln Pro
   65                  70                  75                  80
   Glu Asp Phe Ala Thr Tyr Tyr Cys Gln Gln Ser Tyr Thr Ser Pro Ile
                   85                  90                  95
   Thr Phe Gly Gln Gly Thr Arg Leu Glu Ile Lys
               100                 105
<210> 49
   <211> 380
   <212> DNA
   <213> Artificial
<220>
   <223> Antibody
<400> 49
   caggtgcagc tgcaggagtc gggcccagga ctggtgaagc cttcggagac cctgtccctc     60
   acctgcactg tctctggtgg ctccatcagt agttactact ggagctggat ccggcagccc    120
   ccagggaagg gactggagtg gattgggtat atctattaca gtgggagcac caactacaac    180
   ccctccctca agagtcgagt caccatatca gtagacacgt ccaagcacca gttctccctg    240
   aagctgagct ctgtgaccgc tgcggacacg gccgtgtatt actgtgcgag agattcgagt    300
   tactatgata gtagtggtta ttacttatac tactacgcta tggacgtctg gggccaaggg    360
   accacggtca ccgtctcctc                                                380
<210> 50
   <211> 126
   <212> PRT
   <213> Artificial
<220>
   <223> Antibody
<400> 50
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Tyr Tyr Ser Gly Ser Thr Asn Tyr Asn Pro Ser Leu Lys
       50                  55                  60
   Ser Arg Val Thr Ile Ser Val Asp Thr Ser Lys His Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Asp Ser Ser Tyr Tyr Asp Ser Ser Gly Tyr Tyr Leu Tyr Tyr Tyr
               100                 105                 110
   Ala Met Asp Val Trp Gly Gln Gly Thr Thr Val Thr Val Ser
           115                 120                 125
<210> 51
   <211> 339
   <212> DNA
   <213> Artificial
<220>
   <223> Antibody
<400> 51
   gacatcgtga tgacccagtc tccagactcc ctggctgtgt ctctgggcga gagggccacc      60
   atcaactgca agtccagcca gagtgtttta tacagctcca acaataagaa ctacttagct     120
   tggtaccagc agaaaccagg acagcctcct aagctgctca tttcctgggc atctacccgg     180
   gaatccgggg tccctgaccg attcagtggc agcgggtctg ggacagattt cactctcacc     240
   atcagcagcc tgcaggctga agatgtggca gtttattact gtcagcaata ttatactact     300
   cctctcactt tcggccctgg gaccaaagtg gatatcaaa                            339
<210> 52
   <211> 113
   <212> PRT
   <213> Artificial
<220>
   <223> Antibody
<400> 52
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Ser Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Thr Thr Pro Leu Thr Phe Gly Pro Gly Thr Lys Val Asp Ile
               100                 105                 110
   Lys
<210> 53
   <211> 357
   <212> DNA
   <213> Artificial
<220>
   <223> Antibody
<400> 53
   gaggtgcaac tggtggagtc tgggggaggc ttggtacagc ctggggggtc cctgagactc     60
   tcctgtgcag cctctggatt caccttcagt atctatagca tgaactgggt ccgccaggct    120
   ccagggaagg ggctggagtg ggtttcatac attagtagta gtagtagtac catatactac    180
   gcagactctg tgaagggccg attcaccatc tccagagaca atgccaagaa ctcactgtat    240
   ctgcaaatga acagcctgag agacgaggac acggctgtgt attactgtgc gagagatagg    300
   ggtgacttcg atgcttttga tatctggggc caagggacaa tggtcaccgt ctcttca       357
<210> 54
   <211> 119
   <212> PRT
   <213> Artificial
<220>
   <223> Antibody
<400> 54
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ile Tyr
               20                  25                  30
   Ser Met Asn Trp Val Arg Gln Ala Pro Gly Lys Gly Leu Glu Trp Val
           35                  40                  45
   Ser Tyr Ile Ser Ser Ser Ser Ser Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Asp Glu Asp Thr Ala Val Tyr Tyr Cys
                   85                  90                  95
   Ala Arg Asp Arg Gly Asp Phe Asp Ala Phe Asp Ile Trp Gly Gln Gly
               100                 105                 110
   Thr Met Val Thr Val Ser Ser
           115
<210> 55
   <211> 321
   <212> DNA
   <213> Artificial
<220>
   <223> Antibody
<400> 55
   gacatccaga tgacccagtc tccatcctcc ctgtctgcat ctgtaggaga cagagtcacc     60
   atcacttgcc aggcgagtca ggacattacc aactatttga attggtatca gcagaaacca    120
   gggaaagccc ctaagctcct gatctacgat gcatccaatt tggaaacagg ggtcccatca    180
   aggttcagtg gaagtggatc tgggacagat tttactttca ccatcagcag cctgcagcct    240
   gaagatattg caacatataa ctgtcaacag tgtgaaaatt tcccgatcac cttcggccaa    300
   gggacacgac tggagattaa a                                              321
<210> 56
   <211> 107
   <212> PRT
   <213> Artificial
<220>
   <223> Antibody
<400> 56
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asp Ile Thr Asn Tyr
               20                  25                  30
   Leu Asn Trp Tyr Gln Gln Lys Pro Gly Lys Ala Pro Lys Leu Leu Ile
           35                  40                  45
   Tyr Asp Ala Ser Asn Leu Glu Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Asn Cys Gln Gln Cys Glu Asn Phe Pro Ile
                   85                  90                  95
   Thr Phe Gly Gln Gly Thr Arg Leu Glu Ile Lys
               100                 105
<210> 57
   <211> 336
   <212> DNA
   <213> Artificial
<220>
   <223> Antibody
<400> 57
   gatattgtga tgactcagtc tccactctcc ctgcccgtca cccctggaga gccggcctcc      60
   atctcctgca ggtctagtca gagcctcctg tatagtaatg gatacaagta tttggattgg     120
   tacctgcaga agccagggca gtctccacag ctcctgatct atttgggttc taatcgggcc     180
   tccggggtcc ctgacaggtt cagtggcagt ggatcaggca cagattttac actgaaaatc     240
   agcagagtgg aggctgagga tgttggggtt tattattgca tgcaggctct acaaactccg     300
   atcaccttcg gccaagggac acgactggag attaaa                               336
<210> 58
   <211> 112
   <212> PRT
   <213> Artificial
<220>
   <223> Antibody
<400> 58
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu Tyr Ser
               20                  25                  30
   Asn Gly Tyr Lys Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Ile Thr Phe Gly Gln Gly Thr Arg Leu Glu Ile Lys
               100                 105                 110
<210> 59
   <211> 374
   <212> DNA
   <213> Artificial
<220>
   <223> Antibody
<400> 59
   caggtgcagc tgcaggagtc gggcccagga ctggtgaagc cttcggagac cctgtccctc     60
   acctgcactg tctctggtgg ctccgtcagc agtggtggtt actactggaa ctggatccgg    120
   cagcccccag ggaagggact ggagtggatt gggtatatca attacagtgg gagcaccaac    180
   tacaacccct ccctcaagag tcgagtcacc atatcagtag acacgtccaa gaaccagttc    240
   tccctgaagc tgagctctgt gaccgctgcg gacacggccg tgtattactg tgcgagagat    300
   cgagaactgg aactttacta ctactactac ggtatggacg tctggggcca agggaccacg    360
   gtcaccgtct cctc                                                      374
<210> 60
   <211> 124
   <212> PRT
   <213> Artificial
<220>
   <223> Antibody
<400> 60
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Val Ser Ser Gly
               20                  25                  30
   Gly Tyr Tyr Trp Asn Trp Ile Arg Gln Pro Pro Gly Lys Gly Leu Glu
           35                  40                  45
   Trp Ile Gly Tyr Ile Asn Tyr Ser Gly Ser Thr Asn Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Asp Arg Glu Leu Glu Leu Tyr Tyr Tyr Tyr Tyr Gly Met
   100 105 110
   Asp Val Trp Gly Gln Gly Thr Thr Val Thr Val Ser
   115 120
<210> 61
   <211> 374
   <212> DNA
   <213> Artificial
<220>
   <223> Antibody
<400> 61
   caggtgcagc tgcaggagtc gggcccagga ctggtgaagc cttctgagac cctgtccctc 60
   acctgcactg tctctggtgg ctccgtcagc agtggtggtt actactggaa ctggatccgg 120
   cagcccccag ggaagggact ggagtggatt gggtatatca attacagtgg gagcaccaac 180
   tacaacccct ccctcaagag tcgagtcacc atatcagtag acacgtccaa gaaccagttc 240
   tccctgaagc tgagctctgt gaccgctgcg gacacggccg tgtattactg tgcgagagat 300
   cgagaactgg aactttacta ctactactac ggtatggacg tctggggcca agggaccacg 360
   gtcaccgtct cctc 374
<210> 62
   <211> 124
   <212> PRT
   <213> Artificial
<220>
   <223> Antibody
<400> 62
   Gln Val Gln Leu Gln Glu Ser Gly Pro Gly Leu Val Lys Pro Ser Glu
   1 5 10 15
   Thr Leu Ser Leu Thr Cys Thr Val Ser Gly Gly Ser Val Ser Ser Gly
   20 25 30
   Gly Tyr Tyr Trp Asn Trp Ile Arg Gln Pro Pro Gly Lys Gly Leu Glu
   35 40 45
   Trp Ile Gly Tyr Ile Asn Tyr Ser Gly Ser Thr Asn Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Asp Arg Glu Leu Glu Leu Tyr Tyr Tyr Tyr Tyr Gly Met
   100 105 110
   Asp Val Trp Gly Gln Gly Thr Thr Val Thr Val Ser
   115 120
<210> 63
   <211> 336
   <212> DNA
   <213> Artificial
<220>
   <223> Antibody
<400> 63
   gatattgtga tgactcagtc tccactctcc ctgcccgtca cccctggaga gccggcctcc 60
   atctcctgca ggtctagtca gagcctcctg tatagtaatg gatacaagta tttggattgg 120
   tacctgcaga agccagggca gtctccacag ctcatgatct atttgggttc taatcgggcc 180
   tccggggtcc ctgacaggtt cagtggcagt ggatcaggca cagattttac actgaaaatc 240
   agcagagtgg aggctgagga tgttggggtt tattattgca tgcaggctct acaaactccg 300
   atcaccttcg gccaagggac acgactggag attaaa 336
<210> 64
   <211> 112
   <212> PRT
   <213> Artificial
<220>
   <223> Antibody
<400> 64
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu Tyr Ser
               20                  25                  30
   Asn Gly Tyr Lys Tyr Leu Asp Trp Tyr Leu Gln Lys Pro Gly Gln Ser
           35                  40                  45
   Pro Gln Leu Met Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Ile Thr Phe Gly Gln Gly Thr Arg Leu Glu Ile Lys
               100                 105                 110
<210> 65
   <211> 363
   <212> DNA
   <213> Artificial
<220>
   <223> Antibody
<400> 65
   caggtgcagc tggtggagtc tgggggaggc gtggtccagc ctgggaggtc cctgagactc      60
   tcctgtgcag cgtctggatt caccttcagt agctatggca tgcactgggt ccgccaggct     120
   ccaggcaagg ggctggagtg ggtggcagtt atatggtatg atggaagtaa taaatactat     180
   gcagactccg tgaagggccg attcaccatc tccagagaca attccaagaa cacgctgtat     240
   ctgcaaatga acagcctgag agccgaggac acggctgtgt attactgtgc gagagcagct     300
   cgccttgact actactacgg tatggacgtc tggggccaag ggaccacggt caccgtctcc     360
   tca                                                                   363
<210> 66
   <211> 121
   <212> PRT
   <213> Artificial
<220>
   <223> Antibody
<400> 66
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Trp Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ala Ala Arg Leu Asp Tyr Tyr Tyr Gly Met Asp Val Trp Gly
               100                 105                 110
   Gln Gly Thr Thr Val Thr Val Ser Ser
           115                 120
<210> 67
   <211> 321
   <212> DNA
   <213> Artificial
<220>
   <223> Antibody
<400> 67
   gacatccaga tgacccagtc tccatcctcc ctgtctgcat ctgtaggaga cagagtctcc      60
   atcacttgcc gggcaagtca gagcattaac agctatttaa attggtttca gcagaagcca     120
   gggaaagccc ctcagctcct gatctttggt gcatccggtt tgcaaagtgg ggtcccatca     180
   aggttcagtg gcagtggatc tgggacagat ttcactctca ccatcaacag tctgcaacct     240
   gaagattttg caacttacta ctgtcaacag agttacagtt ccccgctcac cttcggccaa     300
   gggacacgac tggagattaa a                                               321
<210> 68
   <211> 107
   <212> PRT
   <213> Artificial
<220>
   <223> Antibody
<400> 68
   Asp Ile Gln Met Thr Gln Ser Pro Ser Ser Leu Ser Ala Ser Val Gly
   1               5                   10                  15
   Asp Arg Val Ser Ile Thr Cys Arg Ala Ser Gln Ser Ile Asn Ser Tyr
               20                  25                  30
   Leu Asn Trp Phe Gln Gln Lys Pro Gly Lys Ala Pro Gln Leu Leu Ile
           35                  40                  45
   Phe Gly Ala Ser Gly Leu Gln Ser Gly Val Pro Ser Arg Phe Ser Gly
       50                  55                  60
   Ser Gly Ser Gly Thr Asp Phe Thr Leu Thr Ile Asn Ser Leu Gln Pro
   65                  70                  75                  80
   Glu Asp Phe Ala Thr Tyr Tyr Cys Gln Gln Ser Tyr Ser Ser Pro Leu
                   85                  90                  95
   Thr Phe Gly Gln Gly Thr Arg Leu Glu Ile Lys
               100                 105
<210> 69
   <211> 351
   <212> DNA
   <213> Artificial
<220>
   <223> Antibody
<400> 69
   caggtgcagc tacagcagtg gggcgcagga ctgttgaagc cttcggagac cctgtccctc      60
   acctgcgctg tctatggtgg gtccttcagt ggttactact ggagctggat ccgccagccc     120
   ccagggaagg ggctggagtg gattggggaa atcaatcata gtggaagcac caactacaac     180
   ccgtccctca agagtcgagt caccatatca gtagaaacgt ccaagaacca gttctccctg     240
   aagctgagct ctgtgaccgc cgcggacacg gctgtgtatt actgtgcgag agataagtgg     300
   acctggtact tcgatctctg gggccgtggc accctggtca ctgtctcctc a              351
<210> 70
   <211> 117
   <212> PRT
   <213> Artificial
<220>
   <223> Antibody
<400> 70
<210> 71
   <211> 339
   <212> DNA
   <213> Artificial
<220>
   <223> Antibody
<400> 71
   gacatcgaga tgacccagtc tccagactcc ctggctgtgt ctctgggcga gagggccacc     60
   atcaactgca ggtccagcca gagtgtttta tacagctcca gcaataggaa ctacttagct    120
   tggtaccagc agaacccagg acagcctcct aagctgctca tttactgggc ttctacccgg    180
   gaatccgggg tccctgaccg attcagtggc agcgggtctg ggacagattt cactctcacc    240
   atcagcagcc tgcaggctga agatgtggca gtttattact gtcagcaata ttatagtact    300
   cctcggacgt tcggccaagg gaccaaggtg gaaatcaaa                           339
<210> 72
   <211> 113
   <212> PRT
   <213> Artificial
<220>
   <223> Antibody
<400> 72
<210> 73
   <211> 374
   <212> DNA
   <213> Artificial
<220>
   <223> Antibody
<400> 73
   caggtgcagc tgcaggagtc gggcccagga ctggtgaagc cttcacagac cctgtccctc     60
   acctgcactg tctctggtgg ctccatcagc agtggtggtt actactggag ctggatccgc    120
   cagcacccag ggaagggcct ggagtggatg gggaacatct attacagtgg gagcacctac    180
   tacaacccgt ccctcaagag tcgagttacc atatcagtag acacgtctga gaaccagttc    240
   tccctgaagc tgaactctgt gactgccgcg gacacggccg tatattactg tgcgagaggg    300
   ggaactggaa ccaattacta ctactactac ggtatggacg tctggggcca agggaccacg    360
   gtcaccgtct cctc                                                      374
<210> 74
   <211> 124
   <212> PRT
   <213> Artificial
<220>
   <223> Antibody
<400> 74
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg Gln His Pro Gly Lys Gly Leu Glu
           35                  40                  45
   Trp Met Gly Asn Ile Tyr Tyr Ser Gly Ser Thr Tyr Tyr Asn Pro Ser
       50                  55                  60
   Leu Lys Ser Arg Val Thr Ile Ser Val Asp Thr Ser Glu Asn Gln Phe
   65                  70                  75                  80
   Ser Leu Lys Leu Asn Ser Val Thr Ala Ala Asp Thr Ala Val Tyr Tyr
                   85                  90                  95
   Cys Ala Arg Gly Gly Thr Gly Thr Asn Tyr Tyr Tyr Tyr Tyr Gly Met
               100                 105                 110
   Asp Val Trp Gly Gln Gly Thr Thr Val Thr Val Ser
           115                 120
<210> 75
   <211> 324
   <212> DNA
   <213> Artificial
<220>
   <223> Antibody
<400> 75
   gaaattgtgt tgacgcagtc tccaggcacc ctgtctttgt ctccagggga aagagccacc     60
   ctctcctgca gggccagtca gagtgttagc agcagctact tagcctggta ccagcagaaa    120
   cctggccagg ctcccaggct cctcatctat ggtgcatcca gctgggccac tggcatccca    180
   aacaggttca gtggcagtgg gtctgggaca gacttcactc tcaccatcag cagactggag    240
   cctgaagatt ttgcagtgta ttactgtcag cagtatggta gctcaccgct cactttcggc    300
   ggagggacca aggtggagat caaa                                           324
<210> 76
   <211> 108
   <212> PRT
   <213> Artificial
<220>
   <223> Antibody
<400> 76
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Trp Ala Thr Gly Ile Pro Asn Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Leu Thr Phe Gly Gly Gly Thr Lys Val Glu Ile Lys
               100                 105
<210> 77
   <211> 377
   <212> DNA
   <213> Artificial
<220>
   <223> Antibody
<400> 77
   caggtgcagc tgcaggagtc gggcccagga ctggtgaagc cttcacagac cctgtccctc      60
   acctgcactg tctctggtgt ctccatcagc agtggtggtt actactggag ctggatccgc     120
   cagcacccag ggatgggcct ggagtggatt gggtacatct attacagtgg gagcacctac     180
   tacaacccgt ccctcaagag tcgagtcacc atatcagaag acacgtctaa gaaccagttc     240
   tccctgaagc tgagctctgt gactgccgcg gacacggccg tgtattactg tgcgagagat     300
   tccgagtccg agtatagcag ctcgtcgaac tacggtatgg acgtctgggg ccaagggacc     360
   acggtcaccg tctcctc                                                    377
<210> 78
   <211> 125
   <212> PRT
   <213> Artificial
<220>
   <223> Antibody
<400> 78
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Val Ser Ile Ser Ser Gly
               20                  25                  30
   Gly Tyr Tyr Trp Ser Trp Ile Arg Gln His Pro Gly Met Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Glu Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Asp Ser Glu Ser Glu Tyr Ser Ser Ser Ser Asn Tyr Gly
               100                 105                 110
   Met Asp Val Trp Gly Gln Gly Thr Thr Val Thr Val Ser
           115                 120                 125
<210> 79
   <211> 377
   <212> DNA
   <213> Artificial
<220>
   <223> Antibody
<400> 79
   caggtgcagc tgcaggagtc gggcccagga ctggtgaagc cttcacagac cctgtccctc     60
   acctgcactg tctctggtgt ctccatcagc agtggtggtt actactggag ctggatccgc    120
   cagcacccag ggatgggcct ggagtggatt gggtacatct attacagtgg gagcacctac    180
   tacaacccgt ccctcaagag tcgagtcacc atatcagaag acacgtctaa gaaccagttc    240
   tccctgaagc tgagctctgt gactgccgcg gacacggccg tgtattactg tgcgagagat    300
   tccgagtccg agtatagcag ctcgtcgaac tacggtatgg acgtctgggg ccaagggacc    360
   acggtcaccg tctcctc                                                   377
<210> 80
   <211> 125
   <212> PRT
   <213> Artificial
<220>
   <223> Antibody
<400> 80
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Val Ser Ile Ser Ser Gly
                   20                  25                  30
   Gly Tyr Tyr Trp Ser Trp Ile Arg Gln His Pro Gly Met Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Glu Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Asp Ser Glu Ser Glu Tyr Ser Ser Ser Ser Asn Tyr Gly
               100                 105                 110
   Met Asp Val Trp Gly Gln Gly Thr Thr Val Thr Val Ser
           115                 120                 125
<210> 81
   <211> 321
   <212> DNA
   <213> Artificial
<220>
   <223> Antibody
<400> 81
   gacatccaga tgacccagtc tccatcctcc ctgtctgcat ctgtaggaga cagaatcacc      60
   atcacttgcc gggcaagtca gaccattagc agctatttaa attggtatca gcagaaacca     120
   gggaaagccc ctaagctcct gatctatgct gcatccagtt tgcaaggtgg ggtcccatca     180
   aggttcagtg gcagtgtatc tgggacagat ttcaccctca ccgtcagcag tctgcaacct     240
   gaagattttg caacttacta ctgtcaacag agttacagta acccgctcac tttcggcgga     300
   gggaccaagg tggagatcaa a                                               321
<210> 82
   <211> 107
   <212> PRT
   <213> Artificial
<220>
   <223> Antibody
<400> 82
   Asp Ile Gln Met Thr Gln Ser Pro Ser Ser Leu Ser Ala Ser Val Gly
   1               5                   10                  15
   Asp Arg Ile Thr Ile Thr Cys Arg Ala Ser Gln Thr Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Gly Gly Val Pro Ser Arg Phe Ser Gly
       50                  55                  60
   Ser Val Ser Gly Thr Asp Phe Thr Leu Thr Val Ser Ser Leu Gln Pro
   65                  70                  75                  80
   Glu Asp Phe Ala Thr Tyr Tyr Cys Gln Gln Ser Tyr Ser Asn Pro Leu
                   85                  90                  95
   Thr Phe Gly Gly Gly Thr Lys Val Glu Ile Lys
               100                 105
<210> 83
   <211> 371
   <212> DNA
   <213> Artificial
<220>
   <223> Antibody
<400> 83
   gaggtgcagc tggtgcagtc tggagcagag gtgaaaaagc ccggggagtc tctgaagatc     60
   tcctgtaagg gttctggata cagttttacc agctactgga tcggctgggt gcgccagatg    120
   cccgggaaag gcctggagtg gatggggatc atctatcctg gtgactctga taccagatac    180
   agcccgtcct tccaaggcca ggtcaccatg tcagccgaca agtccatcag taccgcctac    240
   ctgcagctga gcagccatga aggcctcgga caccgccatg tattactgtg cgagacagat    300
   ggctggaaac tacgtacatc acgggtgatc gagacgtcct ggggccaagg gaccacggtc    360
   accgtctcct c                                                         371
<210> 84
   <211> 123
   <212> PRT
   <213> Artificial
<220>
   <223> Antibody
<400> 84
   Glu Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Ser Tyr
               20                  25                  30
   Trp Ile Gly Trp Val Arg Gln Met Pro Gly Lys Gly Leu Glu Trp Met
           35                  40                  45
   Gly Ile Ile Tyr Pro Gly Asp Ser Asp Thr Arg Tyr Ser Pro Ser Phe
       50                  55                  60
   Gln Gly Gln Val Thr Met Ser Ala Asp Lys Ser Ile Ser Thr Ala Tyr
   65                  70                  75                  80
   Leu Gln Leu Ser Ser His Glu Gly Leu Gly His Arg His Val Leu Leu
                   85                  90                  95
   Cys Glu Thr Asp Gly Trp Lys Leu Arg Thr Ser Arg Val Ile Glu Thr
               100                 105                 110
   Ser Trp Gly Gln Gly Thr Thr Val Thr Val Ser
           115                 120
<210> 85
   <211> 324
   <212> DNA
   <213> Artificial
<220>
   <223> Antibody
<400> 85
   gaaattgtgt tgacgcagtc tccaggcacc ctgtctttgt ctccagggga aagagccacc      60
   ctctcctgca gggccagtca gagtgttatc agcatctact tagcctggta ccagcagaaa     120
   cctggccagg ctcccaggct cctcatctat ggtgcatcca gcagggccac tggcatccca     180
   gacaggttca gtggcagtgg gtctgggaca gacttcactc tcaccatcag cagactggag     240
   cctgaagatt ttgcagtgta ttactgtcag cagtatggta gctcaccgtg cagttttggc     300
   caggggacca aactggagat caaa                                            324
<210> 86
   <211> 108
   <212> PRT
   <213> Artificial
<220>
   <223> Antibody
<400> 86
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ile Ser Ile
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Cys Ser Phe Gly Gln Gly Thr Lys Leu Glu Ile Lys
               100                 105
<210> 87
   <211> 366
   <212> DNA
   <213> Artificial
<220>
   <223> Antibody
<400> 87
   caggtgcagc tgcaggagtc gggcccagga ctggtgaagc cttcacagac cctgtccctc     60
   acctgcactg tctctggtgg ctccatcagc agtggtgatt actactggag ctggatccgc    120
   cagcacccag ggaagggcct ggagtggatt gggtacatct attacagtgg gagcacctac    180
   tacaacccgt ccctcaggag tcgagttacc atatcagtag acacgtctaa gaaccagttc    240
   tccctgaagc tgagctctgt gactgccgcg gacacggccg tgtattactg tgcgagagcg    300
   gattacgatt tttggagtgg ttattttgac tactggggcc agggaaccct ggtcaccgtc    360
   tcctca                                                               366
<210> 88
   <211> 122
   <212> PRT
   <213> Artificial
<220>
   <223> Antibody
<400> 88
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Arg Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 89
   <211> 322
   <212> DNA
   <213> Artificial
<220>
   <223> Antibody
<400> 89
   gacatccaga tgacccagtc tccatcctcc ctgtctgcat ctgtaggaga cagagtcacc      60
   atcacttgcc gggcaagtca gggcattaga aatgatttag gctggtatca gcagatacct     120
   gggaaagccc ctaagcgcct gatctatgct gcatccagtt tgcaaagtgg ggtcccatca     180
   aggttcagcg gcagtggatc tgggacagaa ttcactctca caatcaacag cctgcagcct     240
   gaagattttg caacttatta ctgtctacag cataatggtt acccgtggac gttcggccaa     300
   gggaccaagg tggaaatcaa ac                                              322
<210> 90
   <211> 107
   <212> PRT
   <213> Artificial
<220>
   <223> Antibody
<400> 90
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Ile Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Asn Ser Leu Gln Pro
   65                  70                  75                  80
   Glu Asp Phe Ala Thr Tyr Tyr Cys Leu Gln His Asn Gly Tyr Pro Trp
                   85                  90                  95
   Thr Phe Gly Gln Gly Thr Lys Val Glu Ile Lys
               100                 105
<210> 91
   <211> 366
   <212> DNA
   <213> Artificial
<220>
   <223> Antibody
<400> 91
   caggtgcagc tgcaggagtc gggcccagga ctggtgaagc cttcacagac cctgtccctc      60
   acctgcactg tctctggtgg ctccatcagc agtggtgatt actactggag ctggatccgc     120
   cagcacccag ggaagggcct ggagtggatt ggatacatct attacagtgg gagcacctac     180
   tacaacccgt ccctcaagag tcgagttacc atatcagtag acacgtctaa gaaccagttc     240
   tccctgaagc tgagctctgt gactgccgcg gacacggccg tgtattactg tgcgagagcg     300
   gattacgatt tttggagtgg ttattttgac tactggggcc agggaaccct ggtcaccgtc     360
   tcctca                                                                366
<210> 92
   <211> 122
   <212> PRT
   <213> Artificial
<220>
   <223> Antibody
<400> 92
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 93
   <211> 322
   <212> DNA
   <213> Artificial
<220>
   <223> Antibody
<400> 93
   gacttccaga tgacccagtc tccatcctcc ctgtctgcat ctgtaggaga cagagtcacc      60
   atcacttgcc gggcaagtca ggacattcga aatgatttag gctggtatcg gcagaaacct     120
   gggaaagccc ctaagcgcct gatctatgct gcatccagtt tgcaaagtgg ggtcccatca     180
   aggttcagcg gcagtggatc tgggacagaa ttcactctca caatcagcag cctgcagcct     240
   gaagattttg caacttatta ctgtctacag cataatagtt acccgtggac gttcggccaa     300
   gggaccaagg tggaaatcaa ac                                              322
<210> 94
   <211> 107
   <212> PRT
   <213> Artificial
<220>
   <223> Antibody
<400> 94
   Asp Phe Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Asp
               20                  25                  30
   Leu Gly Trp Tyr Arg Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 95
   <211> 366
   <212> DNA
   <213> Artificial
<220>
   <223> Antibody
<400> 95
   caggtgcagc tgcaggagtc gggcccagga ctggtgaagc cttcacagac cctgtccctc      60
   acctgcactg tctctggtgg ctccatcagc agtggtgatt actactggag ctggatccgc     120
   cagcacccag ggaagggcct ggagtggatt ggatacatct attacagtgg gagcacctac     180
   tacaacccgt ccctcaagag tcgagttacc atatcaatag acacgtctaa gaaccagttc     240
   tccctgaagc tgagctctgt gactgccgcg gacacggccg tgtattactg tgcgagagcg     300
   gattacgatt tttggaatgg ttattttgac tactggggcc agggaaccct ggtcaccgtc     360
   tcctca                                                                366
<210> 96
   <211> 122
   <212> PRT
   <213> Artificial
<220>
   <223> Antibody
<400> 96
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Ile Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Asn Gly Tyr Phe Asp Tyr Trp
               100                 105                 110
   Gly Gln Gly Thr Leu Val Thr Val Ser Ser
           115                 120
<210> 97
   <211> 321
   <212> DNA
   <213> Artificial
<220>
   <223> Antibody
<400> 97
   gacatccaga tgacccagtc tccatcctcc ctgtctgcat ctgtaggaga cagagtcacc     60
   atcacttgcc gggcaagtca ggacattaga aatgatttag gctggtatcg gcagaaacct    120
   gggaaagccc ctaagcgcct gatctatgct gcatccagtt tgcaaagtgg ggtcccatca    180
   aggttcagcg gcagtggatc tgggacagaa ttcactctca caatcagcag cctgcagcct    240
   gaagattttg caacttatta ctgtctacag cataatagtt acccgtggac gttcggccaa    300
   gggaccaagg tggaaatcaa a                                              321
<210> 98
   <211> 107
   <212> PRT
   <213> Artificial
<220>
   <223> Antibody
<400> 98
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Asp
               20                  25                  30
   Leu Gly Trp Tyr Arg Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 99
   <211> 366
   <212> DNA
   <213> Artificial
<220>
   <223> Antibody
<400> 99
   caggtgcagc tgcaggagtc gggcccagga ctggtgaagc ctacacagac cctgtccctc    60
   acctgcactg tctctggtgg ctccatcagc agtggtgatt actactggag ctggatccgc   120
   cagcacccag ggaagggcct ggagtggatt gggtacatct attacagtgg gagcacctac   180
   tacaacccgt ccctcaagag tcgacttacc atatcagtag acacgtctaa gaaccagttc   240
   tccctgaagc tgagctctgt gactgccgcg gacacggccg tgtattactg tgcgagagca   300
   gattacgatt tttggagtgg ttactttgac tactggggcc agggaaccct ggtcaccgtc   360
   tcctca                                                              366
<210> 100
   <211> 122
   <212> PRT
   <213> Artificial
<220>
   <223> Antibody
<400> 100
   Gln Val Gln Leu Gln Glu Ser Gly Pro Gly Leu Val Lys Pro Thr Gln
   1               5                   10                  15
   Thr Leu Ser Leu Thr Cys Thr Val Ser Gly Gly Ser Ile Ser Ser Gly
                   20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Leu Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 101
   <211> 321
   <212> DNA
   <213> Artificial
<220>
   <223> Antibody
<400> 101
   gacatccaga tgacccagtc tccatcctcc ctgtctgcat ctgtaggaga cagagtcacc      60
   atcacttgcc gggcaagtca gggcattaga aatgatttag gctggtatca gcagaaacca     120
   gggaaagccc ctaagcgcct gatctatgct gcatccagtt tgcaaagtgg ggtcccatca     180
   aggttcagcg gcagtggatc tgggacagaa ttcactctca caatcagcag cctgcagcct     240
   gaagattttg caacttatta ctgtctacag cataataatt acccgtggac gttcggccaa     300
   gggaccaagg tggaaatcaa a                                               321
<210> 102
   <211> 107
   <212> PRT
   <213> Artificial
<220>
   <223> Antibody
<400> 102
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Asn Tyr Pro Trp
                   85                  90                  95
   Thr Phe Gly Gln Gly Thr Lys Val Glu Ile Lys
               100                 105
<210> 103
   <211> 366
   <212> DNA
   <213> Artificial
<220>
   <223> Antibody
<400> 103
   caggtgcagc tgcaggagtc gggcccagga ctggtgaagc cttcacagac cctgtccctc     60
   acctgcactg tctctggtgg ctccatcagt agtggtgatt actactggag ctggatccgc    120
   cagcacccag ggaagggcct ggagtggatt gggtacatct attacagtgg gagcacctac    180
   tacaacccgt ccctcaagag tcgagttacc atatcagtag acacgtctaa gaaccagttc    240
   tccctgaagt tgagctctgt gactgccgcg gacacggccg tgtattactg tgcgagagcc    300
   gattacgatt tttggagtgg ttattttgac tactggggcc agggaaccct ggtcaccgtc    360
   tcctca                                                               366
<210> 104
   <211> 122
   <212> PRT
   <213> Artificial
<220>
   <223> Antibody
<400> 104
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 105
   <211> 321
   <212> DNA
   <213> Artificial
<220>
   <223> Antibody
<400> 105
   gacatccaga tgacccagtc tccatcctcc ctgtctgcat ctgtaggaga cagagtcacc      60
   atcacttgcc gggcaagtca gggcattaga aatgatttag gctggtatca gcagaaacca     120
   gggaaagccc ctaagcgcct gatctatgct gcatccagtt tgcaaagtgg ggtcccatca     180
   aggttcagcg gcagtggatc tgggacagaa ttcactctca caatcagcag cctgcagcct     240
   gaagattttg caacttatta ctgtctacag cataataatt acccgtggac gttcggccaa     300
   gggaccaagg tggaaatcaa a                                               321
<210> 106
   <211> 107
   <212> PRT
   <213> Artificial
<220>
   <223> Antibody
<400> 106
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Asn Tyr Pro Trp
                   85                  90                  95
   Thr Phe Gly Gln Gly Thr Lys Val Glu Ile Lys
               100                 105
<210> 107
   <211> 351
   <212> DNA
   <213> Artificial
<220>
   <223> Antibody
<400> 107
   caggtgcagc tgcaggagtc gggcccagga ctggtgaagc cttcacagac cctgtccctc      60
   acctgcactg tctctggtgg ctccatcagc agtggtggtt actactggag ctggatccgc     120
   cagcacccag ggaagggcct ggagtggatt gggtacatct attacagtgg gagcacctac     180
   tacaacccgt ccctcaagag tcgagttacc atatcagtag acacgtctaa gaaccagttc     240
   tccctgaagc tgagctctgt gactgccgcg gacacggccg tgtattactg tgcgagagag     300
   gacgacggta tggacgtctg gggccaaggg accacggtca ccgtctcctc a              351
<210> 108
   <211> 117
   <212> PRT
   <213> Artificial
<220>
   <223> Antibody
<400> 108
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Asp Asp Gly Met Asp Val Trp Gly Gln Gly Thr Thr
               100                 105                 110
   Val Thr Val Ser Ser
           115
<210> 109
   <211> 336
   <212> DNA
   <213> Artificial
<220>
   <223> Antibody
<400> 109
   gatattgtga tgactcagtc tccactctcc ctgcccgtca cccctggaga gccggcctcc      60
   atttcctgca ggtctagtca gagcctcctg catagtaatg gatacaacta tttggaatgg     120
   tacctgcaga agccagggca gtccccacag ttcatgattt atttggggtc taatcgggcc     180
   tccggggtcc ctgacaggtt cagtggcagt ggatcaggca cagattttac actgaaaatc     240
   agcagagtgg aggctgagga tgttggggtt tattactgca tgcaagctct acaaactccg     300
   atcaccttcg gccaagggac acgactggag attaaa                              336
<210> 110
   <211> 112
   <212> PRT
   <213> Artificial
<220>
   <223> Antibody
<400> 110
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Glu Trp Tyr Leu Gln Lys Pro Gly Gln Ser
           35                  40                  45
   Pro Gln Phe Met Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Ile Thr Phe Gly Gln Gly Thr Arg Leu Glu Ile Lys
               100                 105                 110
<210> 111
   <211> 366
   <212> DNA
   <213> Artificial
<220>
   <223> Antibody
<400> 111
   caggtgcagc tgcaggagtc gggcccagga ctggtgaagc cttcacagac cctgtccctc     60
   acctgcactg tctctggtgg ctccatcagc agtggtgatt actactggag ctggatccgc    120
   cagtacccag ggaagggcct ggagtggatt gggtacatct attacagtgg gagcacctac    180
   tacaacccgt ccctcaagag tcgagttacc atatcagtag acacgtctaa gaaccagttc    240
   tccctgaagc tgaggtctgt gactgccgcg gacacggccg tgtattactg tgcgagagcg    300
   gattacgatt tttggagtgg ttattttgac tactggggcc agggaaccct ggtcaccgtc    360
   tcctca                                                               366
<210> 112
   <211> 122
   <212> PRT
   <213> Artificial
<220>
   <223> Antibody
<400> 112
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln Tyr Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Arg Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 113
   <211> 322
   <212> DNA
   <213> Artificial
<220>
   <223> Antibody
<400> 113
   gacatccaga tgacccagtc tccatcctcc ctgtctgcat ctgtaggaga cagagtcacc      60
   atcacttgcc gggcaagtca gggcattaga aatgatttag gctggtatca gcagaaacca     120
   gggaaagccc ctaagcgcct gatctatgct gcatccagtt tgcaaagtgg ggtcccatca     180
   aggttcagcg gcagtggatc tgggacagaa ttcactctca caatcagcag cctgcagcct     240
   gaagattttg caacttatta ctgtctacag cataatactt acccgtggac gttcggccaa     300
   gggaccaagg tggaaatcaa ac                                              322
<210> 114
   <211> 107
   <212> PRT
   <213> Artificial
<220>
   <223> Antibody
<400> 114
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Thr Tyr Pro Trp
                   85                  90                  95
   Thr Phe Gly Gln Gly Thr Lys Val Glu Ile Lys
               100                 105
<210> 115
   <211> 372
   <212> DNA
   <213> Artificial
<220>
   <223> Antibody
<400> 115
   caggtgcagc tgcaggagtc gggcccagga ctggtgaagc cttcggagac cctgtccctc      60
   acctgcactg tctctggtgg ctccgtcagc agtggtggtt actactggag ctggatccgg     120
   cagcccccag ggaagggact ggagtggatt gggtatatct attacagtgg gagcaccaac     180
   tacaacccct ccctcaagag tcgagtcacc atatcagtag acacgtccaa gaaccagttc     240
   tccctgaagc tgagctctgt gaccgctgcg gacacggccg tgtattactg tgcgagagat     300
   ggggacgtgg atacagctat ggtcgatgct tttgatatct ggggccaagg gacaatggtc     360
   accgtctcct ca                                                          372
<210> 116
   <211> 124
   <212> PRT
   <213> Artificial
<220>
   <223> Antibody
<400> 116
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Val Ser Ser Gly
               20                  25                  30
   Gly Tyr Tyr Trp Ser Trp Ile Arg Gln Pro Pro Gly Lys Gly Leu Glu
           35                  40                  45
   Trp Ile Gly Tyr Ile Tyr Tyr Ser Gly Ser Thr Asn Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Asp Gly Asp Val Asp Thr Ala Met Val Asp Ala Phe Asp
               100                 105                 110
   Ile Trp Gly Gln Gly Thr Met Val Thr Val Ser Ser
           115                 120
<210> 117
   <211> 324
   <212> DNA
   <213> Artificial
<220>
   <223> Antibody
<400> 117
   gaaattgtat tgacgcagtc tccaggcacc ctgtctttgt ctccagggga aagagccacc     60
   ctctcctgca gggccagtca gagtttaagc ggcaactact tagcctggta ccagcagaag    120
   cctggccagg ctcccaggct catcatctgt ggtgcatcca gcagggccac tggcatccca    180
   gacaggttca gtggcagtgg gtctgggaca gacttcactc tcaccatcac aagactggag    240
   cctgaagatt ttgcagtgta ttactgtcag cagtatgata ggtcaccgct cactttcggc    300
   ggagggacca aggtggagat caaa                                           324
<210> 118
   <211> 108
   <212> PRT
   <213> Artificial
<220>
   <223> Antibody
<400> 118
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Leu Ser Gly Asn
               20                  25                  30
   Tyr Leu Ala Trp Tyr Gln Gln Lys Pro Gly Gln Ala Pro Arg Leu Ile
           35                  40                  45
   Ile Cys Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Thr Arg Leu Glu
   65                  70                  75                  80
   Pro Glu Asp Phe Ala Val Tyr Tyr Cys Gln Gln Tyr Asp Arg Ser Pro
                   85                  90                  95
   Leu Thr Phe Gly Gly Gly Thr Lys Val Glu Ile Lys
               100                 105
<210> 119
   <211> 366
   <212> DNA
   <213> Artificial
<220>
   <223> Antibody
<400> 119
   caggtgcagc tgcaggagtc gggcccagga ctggtgaagc cttcacagac cctgtccctc      60
   acctgcactg tctctggtgg ctccatcagc agtggtgatt actactggag ctggatccgc     120
   cagcacccag ggaagggcct ggagtggatt gggtacatct attacagtgg gagcacctac     180
   tacaacccgt ccctcaagag tcgagttacc atatcagtag acacgtctaa gaaccagttc     240
   tccctgaagc tgagctctgt gactgccgcg gacacggccg tgtattactg tgcgagagga     300
   gattacgatt tttggagtgg agagtttgac tactggggcc agggaaccct ggtcaccgtc     360
   tcctca                                                                366
<210> 120
   <211> 122
   <212> PRT
   <213> Artificial
<220>
   <223> Antibody
<400> 120
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Gly Asp Tyr Asp Phe Trp Ser Gly Glu Phe Asp Tyr Trp
               100                 105                 110
   Gly Gln Gly Thr Leu Val Thr Val Ser Ser
           115                 120
<210> 121
   <211> 386
   <212> DNA
   <213> Artificial
<220>
   <223> Antibody
<400> 121
   caggtgcagc tgcaggagtc gggcccagga ctggtgaagc cttcacagac cctgtccctc     60
   acctgcactg tctctggtgg ctccatcagc agtggtggtt actactggag ctggatccgc    120
   cagcacccag ggaagggcct ggagtggatt gggtacatct atgacagtgg gagcacctac    180
   tacaacccgt ccctcaagag tcgagttacc atatcagtag acacgtctaa gaaccagttc    240
   tccctgaagc tgaggtctgt gactgccgcg gacacggccg tgtattactg tgcgagagat    300
   caggggcagg acggatacag ctatggttac ggctactact acggtatgga cgtctggggc    360
   caagggacca cggtcaccgt ctcctc                                         386
<210> 122
   <211> 128
   <212> PRT
   <213> Artificial
<220>
   <223> Antibody
<400> 122
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile Tyr Asp Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Arg Ser Val Thr Ala Ala Asp Thr Ala Val Tyr Tyr
                   85                  90                  95
   Cys Ala Arg Asp Gln Gly Gln Asp Gly Tyr Ser Tyr Gly Tyr Gly Tyr
               100                 105                 110
   Tyr Tyr Gly Met Asp Val Trp Gly Gln Gly Thr Thr Val Thr Val Ser
           115                 120                 125
<210> 123
   <211> 321
   <212> DNA
   <213> Artificial
<220>
   <223> Antibody
<400> 123
   gacatccaga tgacccagtc tccatcctcc ctgtctgcat ctgtaggaga cagagtcacc     60
   atcacttgcc aggcgagtca ggacattagc aattatttaa attggtatca gcagaaacca    120
   gggaaagccc ctaaactcct gatctacgtt gcatccaatt tggaaacagg ggtcccatca    180
   aggttcagtg gaagtggatc tgggacagat tttactttca ccatcagcag cctgcagcct    240
   gaagatattg caacatatta ctgtcaacag tgtgataatc tccctctcac tttcggcgga    300
   gggaccaagg tggagatcaa a                                              321
<210> 124
   <211> 107
   <212> PRT
   <213> Artificial
<220>
   <223> Antibody
<400> 124
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Val Ala Ser Asn Leu Glu Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Gln Gln Cys Asp Asn Leu Pro Leu
                   85                  90                  95
   Thr Phe Gly Gly Gly Thr Lys Val Glu Ile Lys
               100                 105
<210> 125
   <211> 365
   <212> DNA
   <213> Artificial
<220>
   <223> Antibody
<400> 125
   caggtgcagc tgcaggagtc gggcccagga ctggtgaagc cttcacagac cctgtccctc      60
   acctgcactg tctctggtgg ctccatcagc agtggtgatt actactggag ctggatccgc     120
   cagcacccag ggaagggcct ggagtggatt ggatacatct attacagtgg gagcacctac     180
   tacaacccgt ccctcaagag tcgagttacc atatcagtag acacgtctaa gaaccagttc     240
   tccctgaagc tgagctctgt gactgccgcg gacacggccg tgtattactg tgcgagagcg     300
   gattacgatt tttggagtgg ttattttgac tactggggcc agggaaccct ggtcaccgtc     360
   tcctc                                                                 365
<210> 126
   <211> 121
   <212> PRT
   <213> Artificial
<220>
   <223> Antibody
<400> 126
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser
           115                 120
<210> 127
   <211> 322
   <212> DNA
   <213> Artificial
<220>
   <223> Antibody
<400> 127
   gacatccaga tgacccagtc tccatcctcc ctgtctgcat ctgtaggaga cagagtcacc      60
   atcacttgcc gggcaagtca ggacattaga aatgatttag gctggtatcg gcagaaacct     120
   gggaaagccc ctaagcgcct gatctatgct gcatcccgtt tgcaaagtgg ggtcccatca     180
   aggttcagcg gcagtggatc tgggacagaa ttcactctca caatcagcag cctgcagcct     240
   gaagattttg caacttatta ctgtctacag cataatagtt acccgtggac gttcggccaa     300
   gggaccaagg tggaaatcaa ac                                              322
<210> 128
   <211> 107
   <212> PRT
   <213> Artificial
<220>
   <223> Antibody
<400> 128
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Asp
               20                  25                  30
   Leu Gly Trp Tyr Arg Gln Lys Pro Gly Lys Ala Pro Lys Arg Leu Ile
           35                  40                  45
   Tyr Ala Ala Ser Arg Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 129
   <211> 366
   <212> DNA
   <213> Artificial
<220>
   <223> Antibody
<400> 129
   caggtgcagc tgcaggagtc gggcccagga ctggtgaagc cttcacagac cctgtccctc     60
   acctgcactg tctctggtgg ctccatcagc agtggtgatt actactggag ctggatccgc    120
   cagcacccag ggaagggcct ggagtggatt gggtacatct attacagtgg gagcacctac    180
   tacaacccgt ccctcaagag tcgagttacc atatcagtag acacgtctaa gaaccagttc    240
   tccctgaagc tgagctctgt gactgccgcg gacacggccg tgtattactg tgcgagagcc    300
   gattacgatt tttggagtgg ttattttgac tactggggcc agggaaccct ggtcaccgtc    360
   tcctca                                                               366
<210> 130
   <211> 122
   <212> PRT
   <213> Artificial
<220>
   <223> Antibody
<400> 130
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 131
   <211> 322
   <212> DNA
   <213> Artificial
<220>
   <223> Antibody
<400> 131
   gacatccaga tgacccagtc tccatcctcc ctgtctgcat ctgtaggaga cagagtcacc      60
   atcacttgcc gggcaagtca gggcattaga aatgatttag gctggtatca gcagaaacca     120
   gggaaagccc ctaagcgcct gatctatgct gcatccagtt tgcaaaatgg ggtcccatca     180
   aggttcagcg gcagtggatc tgggacagaa ttcactctca caatcagcag cctgcagcct     240
   gaagattttg caacttatta ctgtctacag cataatagtt acccgtggac gttcggccaa     300
   gggaccaagg tggaaatcaa ac                                              322
<210> 132
   <211> 107
   <212> PRT
   <213> Artificial
<220>
   <223> Antibody
<400> 132
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Asn Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 133
   <211> 365
   <212> DNA
   <213> Artificial
<220>
   <223> Antibody
<400> 133
   caggtgcagc tgcaggagtc gggcccagga ctggtgaagc cttcacagac cctgtccctc      60
   acctgcactg tctctggtgg ctccatcagc agtggtgatt actactggag ctggatccgc     120
   cagcacccag ggaagggcct ggagtggatt gggtacatct attacagtgg gagcacctac     180
   tacaacccgt ccctcaagag tcgagttacc atatcagtag acacgtctaa gaaccagttc     240
   tccctgaagc tgagctctgt gactgccgcg gacacggccg tgtattactg tgcgagagcg     300
   gattacgatt tttggagtgg ttattttgac tactggggcc agggaatcct ggtcaccgtc     360
   tcctc                                                                 365
<210> 134
   <211> 121
   <212> PRT
   <213> Artificial
<220>
   <223> Antibody
<400> 134
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Ile Leu Val Thr Val Ser
           115                 120
<210> 135
   <211> 322
   <212> DNA
   <213> Artificial
<220>
   <223> Antibody
<400> 135
   gacatccaga tgacccagtc tccatcctcc ctgtctgcat ctgtaggaga cagagtcacc      60
   atcacttgcc gggcaagtca gggcattaga aatgatttag gctggtatca gcagaaacca     120
   gggaaagccc ctaagcgcct gatttatgct gcatccagtt tgcaaagtgg ggtcccatca     180
   aggttcagcg gcagtggatc tgggacagaa ttcactctca caatcagcag cctgcagcct     240
   gaagattttg caacttatta ctgtctacag cataatagtt acccgtggac gttcggccaa     300
   gggaccaagg tggaaatcaa ac                                              322
<210> 136
   <211> 107
   <212> PRT
   <213> Artificial
<220>
   <223> Antibody
<400> 136
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 137
   <211> 366
   <212> DNA
   <213> Artificial
<220>
   <223> Antibody
<400> 137
   caggtgcagc tgcaggagtc gggcccagga ctggtgaagc cttcacagac cctgtccctc      60
   acctgcactg tctctggtgg ctccatcagt agtggtgatt actactggag ctggatccgc     120
   cagcacccag ggaagggcct ggagtggatt gggtacatct attacagtgg gagcacctac     180
   tacaacccgt ccctcaagag tcgagttacc atatcagtag acacgtctaa gaaccagttc     240
   tccctgaagt tgagctctgt gactgccgcg gacacggccg tgtattactg tgcgagagcc     300
   gattacgatt tttggaatgg ttattttgac tactggggcc agggaaccct ggtcaccgtc     360
   tcctca                                                                 366
<210> 138
   <211> 122
   <212> PRT
   <213> Artificial
<220>
   <223> Antibody
<400> 138
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Asn Gly Tyr Phe Asp Tyr Trp
               100                 105                 110
   Gly Gln Gly Thr Leu Val Thr Val Ser Ser
           115                 120
<210> 139
   <211> 321
   <212> DNA
   <213> Artificial
<220>
   <223> Antibody
<400> 139
   gacatccaga tgacccagtc tccatcctcc ctgtctgcat ctgtaggaga cagagtcacc      60
   atcacttgcc gggcaagtca gggcattaga aatgatttag gctggtatca gcagaaacca     120
   gggaaagccc ctaagcgcct gatctatgct gcatccagtt tgcaaagtgg ggtcccatca     180
   aggttcagcg gcagtggatc tgggacagaa ttcactctca caatcagcag cctgcagcct     240
   gaagattttg caacttatta ctgtctacag cataataatt acccgtggac gttcggccaa     300
   gggaccaagg tggaaatcaa a                                               321
<210> 140
   <211> 107
   <212> PRT
   <213> Artificial
<220>
   <223> Antibody
<400> 140
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Asn Tyr Pro Trp
                   85                  90                  95
   Thr Phe Gly Gln Gly Thr Lys Val Glu Ile Lys
               100                 105
<210> 141
   <211> 366
   <212> DNA
   <213> Artificial
<220>
   <223> Antibody
<400> 141
   caggtgcagc tgcaggagtc gggcccagga ctggtgaagc cttcacagac cctgtccctc      60
   acctgcactg tctctggtgg ctccatcagc agtggtgatt actactggag ctggatccgc     120
   cagcacccag ggaagggcct ggagtggatt gggtacatct attacagtgg gagcacctac     180
   tacaacccgt ccctcaagag tcgagttacc atatcagtag acacgtctaa gaaccagttc     240
   tccctgaagc tgagctctgt gactgccgcg gacacggccg tgtattactg tgcgagagcc     300
   gattacgatt tttggagtgg ttattttgac tactggggcc agggaaccct ggtcaccgtc     360
   tcctca                                                                366
<210> 142
   <211> 122
   <212> PRT
   <213> Artificial
<220>
   <223> Antibody
<400> 142
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 143
   <211> 322
   <212> DNA
   <213> Artificial
<220>
   <223> Antibody
<400> 143
   gacatccagc tgacccagtc tccatcctcc ctgtctgcat ctgtaggaga cagagtcacc 60
   atcacttgcc gggcaagtca gggcattaga aatgatttag gctggtatca gcagaaacca 120
   gggaaagccc ctaagcgcct gatctatgct gcatccagtt tgcaaaatgg ggtcccatca 180
   aggttcagcg gcagtggatc tgggacagaa ttcactctca caatcagcag cctgcagcct 240
   gaagattttg caacttatta ctgtctacag cataatagtt acccgtggac gttcggccaa 300
   gggaccaagg tggaaatcaa ac 322
<210> 144
   <211> 107
   <212> PRT
   <213> Artificial
<220>
   <223> Antibody
<400> 144
   Asp Ile Gln Leu Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn Asp
   20 25 30
   Leu Gly Trp Tyr Gln Gln Lys Pro Gly Lys Ala Pro Lys Arg Leu Ile
   35 40 45
   Tyr Ala Ala Ser Ser Leu Gln Asn Gly Val Pro Ser Arg Phe Ser Gly
   50 55 60
   Ser Gly Ser Gly Thr Glu Phe Thr Leu Thr Ile Ser Ser Leu Gln Pro
   65 70 75 80
   Glu Asp Phe Ala Thr Tyr Tyr Cys Leu Gln His Asn Ser Tyr Pro Trp
   85 90 95
   Thr Phe Gly Gln Gly Thr Lys Val Glu Ile Lys
   100 105
<210> 145
   <211> 365
   <212> DNA
   <213> Artificial
<220>
   <223> Antibody
<400> 145
   caggtgcagc tgcaggagtc gggcccagga ctggtgaagc cttcacagac cctgtccctc     60
   acctgcactg tctctggtgg ctccatcagc agtggtgatt actactggag ctggatccgc    120
   cagtacccag ggaagggcct ggagtggatt gggtacatct attacagtgg gagcacctac    180
   tacaacccgt ccctcaagag tcgagttacc atatcagtag acacgtctaa gaaccagttc    240
   tccctgaagc tgggctctgt gactgccgcg gacacggccg tgtatttctg tgcgagagcc    300
   gattacgatt tttggagtgg ttattttgac ttctggggcc agggaaccct ggtcaccgtc    360
   tcctc                                                                365
<210> 146
   <211> 121
   <212> PRT
   <213> Artificial
<220>
   <223> Antibody
<400> 146
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln Tyr Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Gly Ser Val Thr Ala Ala Asp Thr Ala Val Tyr Phe
                   85                  90                  95
   Cys Ala Arg Ala Asp Tyr Asp Phe Trp Ser Gly Tyr Phe Asp Phe Trp
               100                 105                 110
   Gly Gln Gly Thr Leu Val Thr Val Ser
           115                 120
<210> 147
   <211> 322
   <212> DNA
   <213> Artificial
<220>
   <223> Antibody
<400> 147
   gacatccaga tgacccagtc tccatcctcc ctgtctgcat ctgtaggaga cagagtcacc     60
   atcacttgcc gggcaagtca gggcattaga aatgatttag gctggtatca gcagaaacca    120
   gggaaagccc ctaagcgcct gatctatgct gcatccagtt tgcaaagtgg ggtcccatca    180
   aggttcagcg gcagtggatc tgggacagaa ttcactctca caatcagcag cctgcagcct    240
   gaagattttg caacttatta ctgtctacag cataatggtt acccgtggac gttcggccaa    300
   gggaccaagg tggaaatcaa ac                                             322
<210> 148
   <211> 107
   <212> PRT
   <213> Artificial
<220>
   <223> Antibody
<400> 148
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Gly Tyr Pro Trp
                   85                  90                  95
   Thr Phe Gly Gln Gly Thr Lys Val Glu Ile Lys
               100                 105
<210> 149
   <211> 365
   <212> DNA
   <213> Artificial
<220>
   <223> Antibody
<400> 149
   caggtgcagc tgcaggagtc gggcccagga ctggtgaagc cttcacagac cctgtccctc     60
   acctgcactg tctctggtgg ctccatcagc agtggtgatt actactggag ctggatccgc    120
   cagtacccag ggaagggcct ggagtggatt gggtacatct attacagtgg gagcacctac    180
   tacaacccgt ccctcaagag tcgagttacc atatcagtag acacgtctaa gaaccagttc    240
   tccctgaagc tgggctctgt gactgccgcg gacacggccg tgtatttctg tgcgagagcc    300
   gattacgatt tttggagtgg ttattttgac ttctggggcc agggaaccct ggtcaccgtc    360
   tcctc                                                                365
<210> 150
   <211> 121
   <212> PRT
   <213> Artificial
<220>
   <223> Antibody
<400> 150
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln Tyr Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Gly Ser Val Thr Ala Ala Asp Thr Ala Val Tyr Phe
                   85                  90                  95
   Cys Ala Arg Ala Asp Tyr Asp Phe Trp Ser Gly Tyr Phe Asp Phe Trp
               100                 105                 110
   Gly Gln Gly Thr Leu Val Thr Val Ser
           115                 120
<210> 151
   <211> 322
   <212> DNA
   <213> Artificial
<220>
   <223> Antibody
<400> 151
   gacatccaga tgacccagtc tccatcctcc ctgtctgcat ctgtaggaga cagagtcacc     60
   atcacttgcc gggcaagtca gggcattaga aatgatttag gctggtatca gcagaaacca    120
   gggaaagccc ctaagcgcct gatctatgct gcatccagtt tgcaaagtgg ggtcccatca    180
   aggttcagcg gcagtggatc tgggacagaa ttcactctca caatcagcag cctgcagcct    240
   gaagattttg caacttatta ctgtctacag cataatggtt acccgtggac gttcggccaa    300
   gggaccaagg tggaaatcaa ac                                             322
<210> 152
   <211> 107
   <212> PRT
   <213> Artificial
<220>
   <223> Antibody
<400> 152
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Gly Tyr Pro Trp
                   85                  90                  95
   Thr Phe Gly Gln Gly Thr Lys Val Glu Ile Lys
               100                 105
<210> 153
   <211> 366
   <212> DNA
   <213> Artificial
<220>
   <223> Antibody
<400> 153
   caggtgcagc tgcaggagtc gggcccagga ctggtgaagc cttcacagac cctgtccctc     60
   acctgcactg tctctggtgg ctccatcagt agtggtgatt actactggag ctggatccgc    120
   cagcacccag ggaagggcct ggagtggatt gggtacatct attacagtgg gagcacctac    180
   tacaacccgt ccctcaagag tcgagttacc atatcagtag acacgtctaa gaaccagttc    240
   tccctgaagc tgagctctgt gactgccgcg gacacggccg tgtattactg tgcgagagcg    300
   gattacgatt tttggagtgg ttattttgac tcctggggcc agggaaccct ggtcaccgtc    360
   tcctca                                                               366
<210> 154
   <211> 122
   <212> PRT
   <213> Artificial
<220>
   <223> Antibody
<400> 154
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
   20 25 30
   Asp Tyr Tyr Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Ala Asp Tyr Asp Phe Trp Ser Gly Tyr Phe Asp Ser Trp
   100 105 110
   Gly Gln Gly Thr Leu Val Thr Val Ser Ser
   115 120
<210> 155
   <211> 321
   <212> DNA
   <213> Artificial
<220>
   <223> Antibody
<400> 155
   gacatccaga tgacccagtc tccatcctcc ctgtctgcat ctgtaggaga cagagtcacc 60
   atcacttgcc gggcaagtca gggcattaga aatgatttag gctggtatca gcagatacct 120
   gggaaagccc ctaagcgcct gatctatgct gcatccagtt tgcaaagtgg ggtcccatca 180
   aggttcagcg gcagtggatc tgggacagaa ttcactctca caatcagcag cctgcagcct 240
   gaagattttg caacttatta ctgtctacag cataatggtt acccgtggac gttcggccaa 300
   gggaccaagg tggaaatcaa a 321
<210> 156
   <211> 107
   <212> PRT
   <213> Artificial
<220>
   <223> Antibody
<400> 156
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Ile Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Gly Tyr Pro Trp
                   85                  90                  95
   Thr Phe Gly Gln Gly Thr Lys Val Glu Ile Lys
               100                 105
<210> 157
   <211> 366
   <212> DNA
   <213> Artificial
<220>
   <223> Antibody
<400> 157
   caggttcagc tggtgcagtc tggagctgag gtgaagaagc ctggggcctc agtgaaggtc     60
   tcctgcaagg cttctggtta cacctttacc aactatggta tcagctgggt gcggcaggcc    120
   cctggacaag ggcttgagtg gatgggatgg atcagcgctt acgatggtta cagaaactat    180
   gcacagaagc tccagggcag agtcaccatg accacagaca catccacgac cactgcctac    240
   atggagctga ggagcctgag atctgacgac acggccgtgt attactgtgc gagagatgtt    300
   caagactacg gtgactacga ctactttgac tactggggcc agggaaccct ggtcaccgtc    360
   tcctca                                                               366
<210> 158
   <211> 122
   <212> PRT
   <213> Artificial
<220>
   <223> Antibody
<400> 158
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Ala Tyr Asp Gly Tyr Arg Asn Tyr Ala Gln Lys Leu
       50                  55                  60
   Gln Gly Arg Val Thr Met Thr Thr Asp Thr Ser Thr Thr Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Asp Val Gln Asp Tyr Gly Asp Tyr Asp Tyr Phe Asp Tyr Trp
               100                 105                 110
   Gly Gln Gly Thr Leu Val Thr Val Ser Ser
           115                 120
<210> 159
   <211> 321
   <212> DNA
   <213> Artificial
<220>
   <223> Antibody
<400> 159
   gacatccaga tgacccagtc tccatcctcc ctgtctgcat ctgtaggaga cagagtcacc      60
   atcacttgcc gggcaagtca gagcattagc agttatttaa attggtatca gcagaaacca     120
   gggaaagccc ctaacctcct gatctatgct gcatccagtt tgcaaagtgg ggtcccatca     180
   agattcaggg gcagtggatc tgggacagat ttcactctca ccatcagcag tctgcaacct     240
   gaagattttg caacttacta ctgtcaacag agttacagta cccccatcac cttcggccaa     300
   gggacacgac tggagattaa a                                               321
<210> 160
   <211> 107
   <212> PRT
   <213> Artificial
<220>
   <223> Antibody
<400> 160
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Asn Leu Leu Ile
           35                  40                  45
   Tyr Ala Ala Ser Ser Leu Gln Ser Gly Val Pro Ser Arg Phe Arg Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Ile
                   85                  90                  95
   Thr Phe Gly Gln Gly Thr Arg Leu Glu Ile Lys
               100                 105
<210> 161
   <211> 366
   <212> DNA
   <213> Artificial
<220>
   <223> Antibody
<400> 161
   caggtgcagc tgcaggagtc gggcccagga ctggtgaagc ctttacagac cctgtccctc 60
   acctgcactg tctctggtgg ctccatcagc agtggtgatt actactggag ctggatccgc 120
   cagcacccag ggaagggcct ggagtggatt gggtacatct attacagtgg gaccacctac 180
   tacaacccgt ccctcaagag tcgagttacc atatcagtag acacgtctaa gaaccagttc 240
   gccctgaagc tgaactctgt gactgccgcg gacacggccg tgtattactg tgcgagagcc 300
   gattacgatt tttggagtgg ttattttgac tactggggcc agggaaccct ggtcaccgtc 360
   tcctca 366
<210> 162
   <211> 122
   <212> PRT
   <213> Artificial
<220>
   <223> Antibody
<400> 162
   Gln Val Gln Leu Gln Glu Ser Gly Pro Gly Leu Val Lys Pro Leu Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Thr Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ala Leu Lys Leu Asn Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 163
   <211> 322
   <212> DNA
   <213> Artificial
<220>
   <223> Antibody
<400> 163
   gacatccaga tgacccagtc tccatcctcc ctgtctgcat ctgtaggaga cagagtcacc      60
   atcacttgcc gggcaggtca gggcattaga aatgatttag gctggtatca gcagaaacca     120
   gggaaagccc ctcagcgcct gatctatgct gcatccagtt tgcaaagtgg ggtcccatca     180
   aggttcagcg gcagtggatc tgggacagaa ttctctctca caatctccag cctgcagcct     240
   gaagattttg caacttatta ctgtctacag cataatagtt acccgtggac gttcggccaa     300
   gggaccaagg tggaaatcaa ac                                              322
<210> 164
   <211> 107
   <212> PRT
   <213> Artificial
<220>
   <223> Antibody
<400> 164
   Asp Ile Gln Met Thr Gln Ser Pro Ser Ser Leu Ser Ala Ser Val Gly
   1               5                   10                  15
   Asp Arg Val Thr Ile Thr Cys Arg Ala Gly Gln Gly Ile Arg Asn Asp
               20                  25                  30
   Leu Gly Trp Tyr Gln Gln Lys Pro Gly Lys Ala Pro Gln Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Ser Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 165
   <211> 369
   <212> DNA
   <213> Artificial
<220>
   <223> Antibody
<400> 165
   caggtgcagc tggtggagtc tgggggaggc ttggtcaagc ctggagggtc cctgagactc     60
   tcctgtgcag cctctggatt caccttcagt gactactaca tgagctggat ccgccaggct    120
   ccagggaagg ggctggagtg ggtttcatat attagtagta gtggtaataa catataccac    180
   gcagactctg tgaagggccg attcaccatc tccagggaca acgccaagaa ctcactgtat    240
   ctgcaaatga acagcctgag agccgaggac acggccgtgt attactgtgc gagagagaga    300
   tatagtggct acgacgaccc tgatggtttt gatatctggg gccaagggac aatggtcacc    360
   gtctcttca                                                            369
<210> 166
   <211> 123
   <212> PRT
   <213> Artificial
<220>
   <223> Antibody
<400> 166
   Gln Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Asp Tyr
               20                  25                  30
   Tyr Met Ser Trp Ile Arg Gln Ala Pro Gly Lys Gly Leu Glu Trp Val
           35                  40                  45
   Ser Tyr Ile Ser Ser Ser Gly Asn Asn Ile Tyr His Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Arg Tyr Ser Gly Tyr Asp Asp Pro Asp Gly Phe Asp Ile
               100                 105                 110
   Trp Gly Gln Gly Thr Met Val Thr Val Ser Ser
           115                 120
<210> 167
   <211> 321
   <212> DNA
   <213> Artificial
<220>
   <223> Antibody
<400> 167
   gacatccaga tgacccagtc tccatcctcc ctgtctgcat ctgtaggaga cagagtcacc      60
   atcacttgcc aggcgagtca ggacattagc aactatttaa gttggtttca gcagaaacca     120
   gggaaagccc ctaagctcct gatccacgat gcatccaatt tggaaacagg ggtcccttca     180
   aggttcagtg gaagtggatc tgggacagat tttactttca ccatcagcag cctgcagcct     240
   gaagatattg caacatatta ctgtcaacag tatgataatc ccccgtgcag ttttggccag     300
   gggaccaagc tggagatcaa a                                               321
<210> 168
   <211> 107
   <212> PRT
   <213> Artificial
<220>
   <223> Antibody
<400> 168
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asp Ile Ser Asn Tyr
               20                  25                  30
   Leu Ser Trp Phe Gln Gln Lys Pro Gly Lys Ala Pro Lys Leu Leu Ile
           35                  40                  45
   His Asp Ala Ser Asn Leu Glu Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Gln Gln Tyr Asp Asn Pro Pro Cys
                   85                  90                  95
   Ser Phe Gly Gln Gly Thr Lys Leu Glu Ile Lys
               100                 105
<210> 169
   <211> 366
   <212> DNA
   <213> Artificial
<220>
   <223> Antibody
<400> 169
   caggtgcagc tgcaggagtc gggcccagga ctggtgaagc cttcacagac cctgtccctc      60
   acctgcactg tctctggtgg ctccatcagc agtggttatt actactggag ctggatccgc     120
   cagcacccag ggaagggcct ggagtggatt gggtacatct attacagtgg gaccacctac     180
   tacaatccgt ccttcaagag tcgagttacc atatcagtag acacgtctaa gaaccagttc     240
   tccctgaaac tgagctctgt gactgccgcg gacacggccg tgtattactg tgcgagagcc     300
   gattacgatt tttggagtgg tcactttgac tactggggcc agggaaccct ggtcaccgtc     360
   tcctca                                                                366
<210> 170
   <211> 122
   <212> PRT
   <213> Artificial
<220>
   <223> Antibody
<400> 170
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Tyr Tyr Tyr Trp Ser Trp Ile Arg Gln His Pro Gly Lys Gly Leu Glu
           35                  40                  45
   Trp Ile Gly Tyr Ile Tyr Tyr Ser Gly Thr Thr Tyr Tyr Asn Pro Ser
       50                  55                  60
   Phe Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly His Phe Asp Tyr Trp
               100                 105                 110
   Gly Gln Gly Thr Leu Val Thr Val Ser Ser
           115                 120
<210> 171
   <211> 321
   <212> DNA
   <213> Artificial
<220>
   <223> Antibody
<400> 171
   gacatccaga tgacccagtc tccatcctcc ctgtctgcat ctgtaggaga cagagtcacc     60
   atcacttgcc gggcaagtca gggcattaga aatgatttag gctggtatca gcagaaacca    120
   gggaaagccc ctaagcgcct gatctatgct gcatccagtt tgcaaagtgg ggtcccatca    180
   aggttcagcg gcagtggatc tgggacagaa ttcactctca caatcagcag cctgcagcct    240
   gaagattttg caacttatta ctgtctacag cataatagtt acccgtggac gttcggccaa    300
   gggaccaagg tggaaatcaa a                                              321
<210> 172
   <211> 107
   <212> PRT
   <213> Artificial
<220>
   <223> Antibody
<400> 172
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 173
   <211> 365
   <212> DNA
   <213> Artificial
<220>
   <223> Antibody
<400> 173
   caggttcagc tggtgcagtc tggagctgag gtgaagaagc ctggggcctc agtgaaggtc 60
   tcctgcaagg cttctggtta cacctttacc agctatggta tcagctgggt gcgacaggcc 120
   cctggacaag gacttgagtg gatgggatgg atcagcgctt acgatggtca cacaaactat 180
   gcacagaagc tccagggcag agtcaccatg accacagaca catccacgaa cacagcctac 240
   atggagctga ggagcctgag atctgacgac acggccgttt attactgtgc gagagacccc 300
   catgactaca gtaactacga ggcttttgac ttctggggcc agggaaccct ggtcaccgtc 360
   tcctc 365
<210> 174
   <211> 121
   <212> PRT
   <213> Artificial
<220>
   <223> Antibody
<400> 174
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Ser Tyr
   20 25 30
   Gly Ile Ser Trp Val Arg Gln Ala Pro Gly Gln Gly Leu Glu Trp Met
   35 40 45
   Gly Trp Ile Ser Ala Tyr Asp Gly His Thr Asn Tyr Ala Gln Lys Leu
   50 55 60
   Gln Gly Arg Val Thr Met Thr Thr Asp Thr Ser Thr Asn Thr Ala Tyr
   65 70 75 80
   Met Glu Leu Arg Ser Leu Arg Ser Asp Asp Thr Ala Val Tyr Tyr Cys
   85 90 95
   Ala Arg Asp Pro His Asp Tyr Ser Asn Tyr Glu Ala Phe Asp Phe Trp
   100 105 110
   Gly Gln Gly Thr Leu Val Thr Val Ser
   115 120
<210> 175
   <211> 519
   <212> DNA
   <213> Artificial
<220>
   <223> Antibody
<400> 175
   atgaggtccc ctgctcagct cctggggctc ctgctactct ggctccgagg tgccagatgt      60
   gacatccaga tgacccagtc tccatcctcc ctgtctgcat ctgtaggaga cagagtcacc     120
   atcacttgcc gggcaagtca gagcattagc agttatttaa attggtatca gcagaaacca     180
   gggaaagccc ctaacctcct gatctatgct gcatccagtt tgcaaagtgg ggtcccatca     240
   agattcagtg gcagtggatc tgggacagat ttcactctca ccatcagcag tctgcaacct     300
   gaagattttg caacttacta ctgtcaacag agttacagta cccccatcac cttcggccaa     360
   gggacacgac tggagattaa acgaactgtg gctgcaccat ctgtcttcat cttcccgcca     420
   tctgatgagc agttgaaatc tggaactgcc tctgttgtgt gcctgctgaa taacttctat     480
   cccagagagg ccaaagtaca gtggaaggtg gataacgcc                            519
<210> 176
   <211> 107
   <212> PRT
   <213> Artificial
<220>
   <223> Antibody
<400> 176
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Asn Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Ile
                   85                  90                  95
   Thr Phe Gly Gln Gly Thr Arg Leu Glu Ile Lys
               100                 105
<210> 177
   <211> 534
   <212> DNA
   <213> Artificial
<220>
   <223> Antibody
<400> 177
   accatggact ggacctggag ggtccttttc ttggtggcag cagcaacagg tgcccactcc      60
   caggttcagc tggtgcagtc tggagctgag gtgaagaagc ctggggcctc agtgaaggtc     120
   tcctgcaagg cttctggtta cacctttacc aactatggta tcagctgggt gcggcaggcc     180
   cctggacaag ggcttgagtg gatgggatgg atcagcgctt acgatggtta cagaaactat     240
   gcacagaagc tccagggcag agtcaccatg accacagaca catccacgac cactgcctac     300
   atggagctga ggagcctgag atctgacgac acggccgtgt attactgtgc gagagatgtt     360
   caagactacg gtgactacga ctactttgac tactggggcc agggaaccct ggtcaccgtc     420
   tcctcagctt ccaccaaggg cccatccgtc ttccccctgg tgccctgctc caggagcacc     480
   tccgagagca cagccgccct gggctgcctg gtcaaggact acttccccga accg           534
<210> 178
   <211> 122
   <212> PRT
   <213> Artificial
<220>
   <223> Antibody
<400> 178
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Ala Tyr Asp Gly Tyr Arg Asn Tyr Ala Gln Lys Leu
       50                  55                  60
   Gln Gly Arg Val Thr Met Thr Thr Asp Thr Ser Thr Thr Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Asp Val Gln Asp Tyr Gly Asp Tyr Asp Tyr Phe Asp Tyr Trp
               100                 105                 110
   Gly Gln Gly Thr Leu Val Thr Val Ser Ser
           115                 120
<210> 179
   <211> 504
   <212> DNA
   <213> Artificial
<220>
   <223> Antibody
<400> 179
   cagctcctgg ggctcctgct actctggctc cgaggtgcca gatgtgacat ccagatgacc     60
   cagtctccat cctccctgtc tgcatctgta ggagacagag tcaccatcac ttgccgggca    120
   agtcagagca ttagcagtta tttaaattgg tatcagcaga aaccagggaa agcccctaac    180
   ctcctgatct atgctgcatc cagtttgcaa agtggggtcc catcaagatt cagtggcagt    240
   ggatctggga cagatttcac tctcaccatc agcagtctgc aacctgaaga ttttgcaact    300
   tactactgtc aacagagtta cagtaccccc atcaccttcg gccaagggac acgactggag    360
   attaaacgaa ctgtggctgc accatctgtc ttcatcttcc cgccatctga tgagcagttg    420
   aaatctggaa ctgcctctgt tgtgtgcctg ctgaataact tctatcccag agaggccaaa    480
   gtacagtgga aggtggataa cgcc                                           504
<210> 180
   <211> 107
   <212> PRT
   <213> Artificial
<220>
   <223> Antibody
<400> 180
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Asn Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Ile
                   85                  90                  95
   Thr Phe Gly Gln Gly Thr Arg Leu Glu Ile Lys
               100                 105
<210> 181
   <211> 493
   <212> DNA
   <213> Artificial
<220>
   <223> Antibody
<400> 181
   catctgtggt tcttcctcct gctggtggca gctcccagat gggtcctgtc ccaggtgcag      60
   ctgcaggagt cgggcccagg actggtgaag ccttcacaga ccctgtccct cacctgcact     120
   gtctctggtg gctccatcaa cagtggtgat tactactgga gctggatccg ccagcaccca     180
   gggaagggcc tggagtggat tgggtacatc tattacagtg ggagcaccta ctacaacccg     240
   tccctcaaga gtcgagttac catatcagta gacacgtcta agaaccagtt ctccctgaag     300
   ctgagctctg tgactgccgc ggacacggcc gtgtattact gtgcgagagc agattacgat     360
   ttttggagtg gttactttga ctactggggc cagggaaccc tggtcaccgt ctcctcagcc     420
   tccaccaagg gcccatcggt cttccccctg gcaccctcct ccaagagcac ctctgggggc     480
   acaacggccc tgg                                                        493
<210> 182
   <211> 122
   <212> PRT
   <213> Artificial
<220>
   <223> Antibody
<400> 182
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Asn Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 183
   <211> 518
   <212> DNA
   <213> Artificial
<220>
   <223> Antibody
<400> 183
   atgagggtcc ctgctcagct cctggggctc ctgctgctct ggttcccagg tgccaggtgt     60
   gacatccaga tgacccagtc tccatcctcc ctgtctgcat ctgtaggaga cagagtcacc    120
   atcacttgcc gggcaagtca gggcattaga aatgatttag gctggtatca gcagaaacca    180
   gggaaagccc ctaagcgcct gatctatgct gcatccagtt tgcaaagtgg ggtcccatca    240
   aggttcagcg gcagtggatc tgggacagaa ttcactctca caatcagcag cctgcagcct    300
   gaagattttg caacttatta ctgtctacag cataatagtt acccgtggac gttcggccaa    360
   gggaccaagg tggaaatcaa acgaactgtg gctgcaccat ctgtcttcat cttcccgcca    420
   tctgatgagc agttgaaatc tggaactgcc tctgttgtgt gcctgctgaa taacttctat    480
   cccagagagg ccaaagtaca gtggaaggtg gataacgc                             518
<210> 184
   <211> 107
   <212> PRT
   <213> Artificial
<220>
   <223> Antibody
<400> 184
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 185
   <211> 436
   <212> DNA
   <213> Artificial
<220>
   <223> Antibody
<400> 185
   tggttcttcc ttctgctggt ggcagctccc agatgggtcc tgtcccaggt gcagctgcag      60
   gagtcgggcc caggactggt gaagccttca cagaccctgt ccctcacctg cactgtctct     120
   ggtggctcca tcagcagtgg tggttactac tggagctgga tccgccagca cccagggaag     180
   ggcctggagt ggattgggta catctattac agtgggagca cctactacaa cccgtccctc     240
   aagagtcgag ttaccatatc agtagacacg tctaagaacc agttctccct gaagctgagc     300
   tctgtgactg ccgcggacac ggccgtgtat tactgtgcga gagatggcta tgatagtagt     360
   ggttattacc acggctactt tgactactgg ggccagggaa ccctggtcac cgtctcctca     420
   gcctccacca agggcc                                                      436
<210> 186
   <211> 125
   <212> PRT
   <213> Artificial
<220>
   <223> Antibody
<400> 186
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Asp Gly Tyr Asp Ser Ser Gly Tyr Tyr His Gly Tyr Phe
               100                 105                 110
   Asp Tyr Trp Gly Gln Gly Thr Leu Val Thr Val Ser Ser
           115                 120                 125
<210> 187
   <211> 521
   <212> DNA
   <213> Artificial
<220>
   <223> Antibody
<400> 187
   caggtcttca tttctctgtt gctctggatc tctggtgcct acggggacat cgtgatgacc      60
   cagtctccag actccctggc tgtgtctctg ggcgagaggg ccaccatcaa ctgcaagtcc     120
   agccagagtg ttttatacag ctccaacaat aagaactact tagcttggta ccagcagaaa     180
   ccaggacagc ctcctaagct gctcatttac tgggcatcta cccgggaatc cggggtccct     240
   gaccgattca gtggcagcgg gtctgggaca gatttcactc tcaccatcag cagcctgcag     300
   gctgaagatg tggcagttta ttactgtcag caatattata gtactccgct cactttcggc     360
   ggagggacca aggtggagat caaacgaact gtggctgcac catctgtctt catcttcccg     420
   ccatctgatg agcagttgaa atctggaact gcctctgttg tgtgcctgct gaataacttc     480
   tatcccagag aggccaaagt acagtggaag gtggataacg c                         521
<210> 188
   <211> 113
   <212> PRT
   <213> Artificial
<220>
   <223> Antibody
<400> 188
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Ser Thr Pro Leu Thr Phe Gly Gly Gly Thr Lys Val Glu Ile
               100                 105                 110
   Lys
<210> 189
   <211> 455
   <212> DNA
   <213> Artificial
<220>
   <223> Antibody
<400> 189
   ctgtggttct tcctcctgct ggtggcagct cccagatggg tcctgtccca ggtgcagctg     60
   caggagtcgg gcccaggact ggtgaagcct tcacagaccc tgtccctcac ctgcactgtc    120
   tctggtggct ccatcagtag tggtgattac tactggagct ggatccgcca gcacccaggg    180
   aagggcctgg agtggattgg gtacatctat tacagtggga gcacctacta caacccgtcc    240
   ctcaagagtc gagttaccat atcagtagac acgtctaaga accagttctc cctgaagttg    300
   agctctgtga ctgccgcgga cacggccgtg tattactgtg cgagagccga ttacgatttt    360
   tggagtggtt attttgacta ctggggccag ggaaccctgg tcaccgtctc ctcagcctcc    420
   accaagggcc catcggtctt ccccctggca ccctc                               455
<210> 190
   <211> 122
   <212> PRT
   <213> Artificial
<220>
   <223> Antibody
<400> 190
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Ile Ser Ser Gly
   20 25 30
   Asp Tyr Tyr Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Ala Asp Tyr Asp Phe Trp Ser Gly Tyr Phe Asp Tyr Trp
   100 105 110
   Gly Gln Gly Thr Leu Val Thr Val Ser Ser
   115 120
<210> 191
   <211> 442
   <212> DNA
   <213> Artificial
<220>
   <223> Antibody
<400> 191
   gtgcccgctc agcgcctggg gctcctgctg ctctggttcc caggtgccag gtgtgacatc 60
   cagatgaccc agtctccatc ctccctgtct gcatctgtag gagacagagt caccatcact 120
   tgccgggcaa gtcagggcat tagaaatgat ttaggctggt atcagcagaa accagggaaa 180
   gcccctaagc gcctgatcta tgctgcatcc agtttgcaaa gtggggtccc atcaaggttc 240
   agcggcagtg gatctgggac agaattcact ctcacaatca gcagcctgca gcctgaagat 300
   tttgcaactt attactgtct acagcataat aattacccgt ggacgttcgg ccaagggacc 360
   aaggtggaaa tcaaacgaac tgtggctgca ccatctgtct tcatcttccc gccatctgat 420
   gagcagttga aatctggaac tg 442
<210> 192
   <211> 107
   <212> PRT
   <213> Artificial
<220>
   <223> Antibody
<400> 192
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Asn Tyr Pro Trp
                   85                  90                  95
   Thr Phe Gly Gln Gly Thr Lys Val Glu Ile Lys
               100                 105
<210> 193
   <211> 427
   <212> DNA
   <213> Artificial
<220>
   <223> Antibody
<400> 193
   tggttcttcc ttctgctggt ggcagctccc agatgggtcc tgtcccaggt gcagctgcag     60
   gagtcgggcc caggactggt gaagccttca cagaccctgt ccctcacctg cactgtctct    120
   ggtggctcca tcagcagtgg tgattactac tggagctgga tccgccagca cccagggaag    180
   ggcctggagt ggattgggta catctattac agtgggagca cctactacaa cccgtccctc    240
   aagagtcgag ttaccatatc agtagacacg tctaagaacc agttctccct gaagctgagc    300
   tctgtgactg ccgcggacac ggccgtgtat ttctgtgcga gagccgatta cgatttttgg    360
   agtggttatt ttgactactg gggccaggga accctggtca ccgtctcctc agcctccacc    420
   aagggcc                                                              427
<210> 194
   <211> 122
   <212> PRT
   <213> Artificial
<220>
   <223> Antibody
<400> 194
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Phe
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 195
   <211> 518
   <212> DNA
   <213> Artificial
<220>
   <223> Antibody
<400> 195
   atgagggtcc ccgctcagct cctggggctc ctgctgctct ggttcccagg tgccaggtgt     60
   gacatccaga tgacccagtc tccatcctcc ctgtctgcat ctgtaggaga cagagtcacc    120
   atcacttgcc gggcaagtca gggcattaga aatgatttag gctggtatca gcagaaacca    180
   gggaaagccc ctaagcgcct gatctatgct gcatccagtt tgcaaagtgg ggtcccatca    240
   aggttcagcg gcagtggatc tgggacagaa ttcactctca caatcagcag cctgcagcct    300
   gaagattttg caacttatta ctgtctacag cataatactt acccgtggac gttcggccaa    360
   gggaccaagg tggaaatcaa acgaactgtg gctgcaccat ctgtcttcat cttcccgcca    420
   tctgatgagc agttgaaatc tggaactgcc tctgttgtgt gcctgctgaa taacttctat    480
   cccagagagg ccaaagtaca gtggaaggtg gataacgc                            518
<210> 196
   <211> 107
   <212> PRT
   <213> Artificial
<220>
   <223> Antibody
<400> 196
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Thr Tyr Pro Trp
                   85                  90                  95
   Thr Phe Gly Gln Gly Thr Lys Val Glu Ile Lys
               100                 105
<210> 197
   <211> 428
   <212> DNA
   <213> Artificial
<220>
   <223> Antibody
<400> 197
   tggttcttcc ttctgctggt ggcagctccc agatgggtcc tgtcccaggt gcagctgcag     60
   gagtcgggcc caggactggt gaagccttca cagaccctgt ccctcacctg cactgtctct    120
   ggtggctcca tcagcagtgg tgattactac tggagctgga tccgccagca cccagggaag    180
   ggcctggagt ggattgggta catctattac agtgggagca cctactacaa cccgtccctc    240
   aagagtcgag ttaccatatc agtagacacg tctaagaacc agttctccct gaagctgagc    300
   tctgtgactg ccgcggacac ggccgtgtat ttctgtgcga gagccgatta cgatttttgg    360
   aatggttatt ttgactactg gggccaggga accctggtca ccgtctcctc agcctccacc    420
   aagggccc                                                             428
<210> 198
   <211> 122
   <212> PRT
   <213> Artificial
<220>
   <223> Antibody
<400> 198
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Phe
   85 90 95
   Cys Ala Arg Ala Asp Tyr Asp Phe Trp Asn Gly Tyr Phe Asp Tyr Trp
   100 105 110
   Gly Gln Gly Thr Leu Val Thr Val Ser Ser
   115 120
<210> 199
   <211> 519
   <212> DNA
   <213> Artificial
<220>
   <223> Antibody
<400> 199
   atgagggtcc ccgctcagct cctggggctc ctgctgctct ggttcccagg tgccaggtgt 60
   gacatccaga tgacccagtc tccatcctcc ctgtctgcat ctgtaggaga cagagtcacc 120
   atcacttgcc gggcaagtca gggcattaga aatgatttag gctggtatca gcagaaacca 180
   gggaaagccc ctaagcgcct gatctatgct gcttccagtt tgcaaagtgg ggtcccatca 240
   aggttcagcg gcagtggatc tgggacagaa ttcactctca caatcagcag cctgcagcct 300
   gaagattttg caacttatta ctgtctacag cataatactt acccgtggac gttcggccaa 360
   gggaccaagg tggaaatcaa acgaactgtg gctgcaccat ctgtcttcat cttcccgcca 420
   tctgatgagc agttgaaatc tggaactgcc tctgttgtgt gcctgctgaa taacttctat 480
   cccagagagg ccaaagtaca gtggaaggtg gataacgcc 519
<210> 200
   <211> 107
   <212> PRT
   <213> Artificial
<220>
   <223> Antibody
<400> 200
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Thr Tyr Pro Trp
                   85                  90                  95
   Thr Phe Gly Gln Gly Thr Lys Val Glu Ile Lys
               100                 105
<210> 201
   <211> 398
   <212> DNA
   <213> Artificial
<220>
   <223> Antibody
<400> 201
   ttggtggcag cagctacagg cacccacgcc caggtccagc tggtacagtc tggggctgag      60
   gtgaagaagc ctggggcctc agtgaaggtc tcctgcaagg tttccggata caccctcact     120
   gaattatcca tgtactgggt gcgacaggct cctggaaaag ggcttgagtg gatgggaggt     180
   tttgatcctg aagatggtga aacaatctac gcacagaagt tccagggcag agtcaccatg     240
   accgaggaca catctacaga cacagcctac atggagctga gcagcctgag atctgaggac     300
   acggccgtgt attactgtgc aactgggtgg aactacgtct ttgactactg gggccaggga     360
   accctggtca ccgtctcctc agcctccacc aagggccc                             398
<210> 202
   <211> 117
   <212> PRT
   <213> Artificial
<220>
   <223> Antibody
<400> 202
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Thr Leu Thr Glu Leu
               20                  25                  30
   Ser Met Tyr Trp Val Arg Gln Ala Pro Gly Lys Gly Leu Glu Trp Met
           35                  40                  45
   Gly Gly Phe Asp Pro Glu Asp Gly Glu Thr Ile Tyr Ala Gln Lys Phe
       50                  55                  60
   Gln Gly Arg Val Thr Met Thr Glu Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Thr Gly Trp Asn Tyr Val Phe Asp Tyr Trp Gly Gln Gly Thr Leu
               100                 105                 110
   Val Thr Val Ser Ser
           115
<210> 203
   <211> 388
   <212> DNA
   <213> Artificial
<220>
   <223> Antibody
<400> 203
   ggatccagtg gggatattgt gatgactcag tctccactct ccctgcccgt cacccctgga      60
   gagccggcct ccatctcctg caggtccagt cagagcctcc tgcatagtaa tggatacaac    120
   tatttggatt ggtacctgca gaagccaggg cagtctccac agctcctgat ctatttggat    180
   tctcatcggg cctccggggt ccctgacagg ttcagtggca gtggatcagg cacagatttt    240
   acactgaaaa tcagcagagt ggaggctgag gatgttgggg tttattactg catgcaagct    300
   ctacaaactc cgctcacttt cggcggaggg accaaggtgg agatcaaacg aactgtggct    360
   gcaccatctg tcttcatctt cccgccat                                       388
<210> 204
   <211> 112
   <212> PRT
   <213> Artificial
<220>
   <223> Antibody
<400> 204
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Asp Ser His Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 205
   <211> 446
   <212> DNA
   <213> Artificial
<220>
   <223> Antibody
<400> 205
   tggttcttcc ttctgctggt ggcagctccc agatgggtcc tgtcccaggt gcagctgcag     60
   gagtcgggcc caggactggt gaagccttca cagaccctgt ccctcacctg cactgtctct    120
   ggtggctcca tcagcagtgg tgattactac tggagctgga tccgccagca cccagggaag    180
   ggcctggagt ggattgggta catctattac agtgggagca cctactacaa cccgtccctc    240
   aagagtcgag ttaccatatc agtagacacg tctaagaacc agttctccct gaagctgagc    300
   tctgtgactg ccgcggacac ggccgtgtat ttctgtgcga gagccgatta cgatttttgg    360
   agtggttatt ttgactactg gggccaggga accctggtca ccgtctcctc agcctccacc    420
   aagggcccat cgagtcttcc ccctgg                                         446
<210> 206
   <211> 122
   <212> PRT
   <213> Artificial
<220>
   <223> Antibody
<400> 206
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Phe
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 207
   <211> 519
   <212> DNA
   <213> Artificial
<220>
   <223> Antibody
<400> 207
   atgagggtcc ccgctcagct cctggggctc ctgctgctct ggttcccagg tgccaggtgt      60
   gacatccaga tgacccagtc tccatcctcc ctgtctgcat ctgtaggaga cagagtcacc     120
   atcacttgcc gggcaagtca gggcattaga aatgatttag gctggtatca gcagaaacca     180
   gggaaagccc ctaagcgcct gatctatgct gcatccagtt tgcaaagtgg ggtcccatca     240
   aggttcagcg gcagtggatc tgggacaaaa ttcactctca ctatcagcag cctgcagcct     300
   gaagattttg caacttatta ctgtctacag cataatactt acccgtggac gttcggccaa     360
   gggaccaagg tggaaatcag acgaactgtg gctgcaccat ctgtcttcat cttcccgcca     420
   tctgatgagc agttgaaatc tggaactgcc tctgttgtgt gcctgctgaa taacttctat     480
   cccagagagg ccaaagtaca gtggaaggtg gataacgcc                            519
<210> 208
   <211> 107
   <212> PRT
   <213> Artificial
<220>
   <223> Antibody
<400> 208
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Lys Phe Thr Leu Thr Ile Ser Ser Leu Gln Pro
   65                  70                  75                  80
   Glu Asp Phe Ala Thr Tyr Tyr Cys Leu Gln His Asn Thr Tyr Pro Trp
                   85                  90                  95
   Thr Phe Gly Gln Gly Thr Lys Val Glu Ile Arg
               100                 105
<210> 209
   <211> 564
   <212> DNA
   <213> Artificial
<220>
   <223> Antibody
<400> 209
   accatgaaac atctgtggtt cttcctcctg ctggtggcag ctcccagatg ggtcctgtcc     60
   caggtgcagc tgcaggagtc gggcccagga ctggtgaagc cttcacagac cctgtccctc    120
   acctgcactg tctctggtgg ctccatcagc agtggtgatt actactggag ctggatccgc    180
   cagcacccag ggaagggcct ggagtggatt gggtacatct attacagtgg gagcacctac    240
   tacaacccgt ccctcaagag tcgagttacc atatcagtag acacgtctaa gaaccagttc    300
   tccctgaagc tgagctctgt gactgccgcg gacacggccg tgtattactg tgcgagagcg    360
   gattacgatt tttggagtgg ttattttgac tactggggcc agggaatcct ggtcaccgtc    420
   tcctcagcct ccaccaaggg cccatcggtc ttccccctgg caccctcctc caagaacacc    480
   tctgggggca cagcggccct gggctgcctg gtcaaggact acttccccga accggtgacg    540
   gtgtcctgga actcaggcgc cctg                                           564
<210> 210
   <211> 122
   <212> PRT
   <213> Artificial
<220>
   <223> Antibody
<400> 210
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Ile Leu Val Thr Val Ser Ser
           115                 120
<210> 211
   <211> 519
   <212> DNA
   <213> Artificial
<220>
   <223> Antibody
<400> 211
   atgagggtcc ccgctcagct cctggggctc ctgctgctct ggttcccagg tgccaggtgt     60
   gacatccaga tgacccagtc tccatcctcc ctgtctgcat ctgtaggaga cagagtcacc    120
   atcacttgcc gggcaagtca gggcattaga aatgatttag gctggtatca gcagaaacca    180
   gggaaagccc ctaagcgcct gatctatgct gcatccagtt tgcaaagtgg ggtcccatca    240
   aggttcagcg gcagtggatc tgggacagaa ttcactctca caatcagcag cctgcagcct    300
   gaagattttg caacttatta ctgtctacag cataatagtt acccgtggac gttcggccaa    360
   gggaccaagg tggaaatcaa acgaactgtg gctgcaccat ctgtcttcat cttcccgcca    420
   tctgatgagc agttgaaatc tggaactgcc tctgttgtgt gcctgctgaa taacttctat    480
   cccagagagg ccaaagtaca gtggaaggtg gataacgcc                           519
<210> 212
   <211> 107
   <212> PRT
   <213> Artificial
<220>
   <223> Antibody
<400> 212
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 213
   <211> 432
   <212> DNA
   <213> Artificial
<220>
   <223> Antibody
<400> 213
   tggttcttcc ttctgctggt ggcagctccc agatgggtcc tgtcccaggt gcagctgcag     60
   gagtcgggcc caggactggt gaagccttca cagaccctgt ccctcacctg cactgtctct    120
   ggtggctcca tcagcagtgg tgattactac tggagctgga tccgccagca cccagggaag    180
   ggcctggagt ggattggata catctattac agtgggagca cctactacaa ttcgtccctc    240
   aagagtcgag ttaccatatc agtagacacg tctaagaacc agttctccct gaagctgagc    300
   tctgtgactg ccgcggacac ggccgtgtat tactgtgcga gagcggatta cgatttttgg    360
   agtggttatt ttgactactg gggccaggga accctggtca ccgtctcctc agcctccacc    420
   aagggcccat cg                                                        432
<210> 214
   <211> 122
   <212> PRT
   <213> Artificial
<220>
   <223> Antibody
<400> 214
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Ser Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 215
   <211> 372
   <212> DNA
   <213> Artificial
<220>
   <223> Antibody
<400> 215
   ggtgccaggt gtgacatcca gatgacccag tctccatcct ccctgtctgc atctgtagga     60
   gacagagtca ccatcacttg ccgggcaagt cagggcatta gaaatgattt aggctggtat    120
   cagcagaaac ctgggaaagc ccctaagcgc ctgatctatg ctgcatccag tttgcaaagt    180
   ggggtcccat caaggttcag cggcagtgga tctgggacag aattcactct cacaatcagc    240
   agcctgcagc ctgaagattt tgcaacttat tactgtctac agcacaatag ttacccgtgg    300
   acgttcggcc aagggaccaa ggtggaaatc aaacgaactg tggctgcacc atctgtcttc    360
   atcttcccgc ca                                                        372
<210> 216
   <211> 107
   <212> PRT
   <213> Artificial
<220>
   <223> Antibody
<400> 216
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 217
   <211> 548
   <212> DNA
   <213> Artificial
<220>
   <223> Antibody
<400> 217
   aggttcttcc ttctgctggt ggcagctccc agatgggtcc tgtcccaggt gcagctgcag     60
   gagtcgggcc caggactggt gaagccttca cagaccctgt ccctcacctg cactgtctct    120
   ggtggctcca tcagcagtgg tgattactac tggagctgga tccgccagca cccagggaag    180
   ggcctggagt ggattggata catctattac agtgggagca cctactacaa cccgtccctc    240
   aagagtcgag ttaccatatc agtagacacg tctaagaacc agttctccct gaagctgagc    300
   tctgtgactg ccgcggacac ggccgtgtat tactgtgcga gagccgatta cgatttttgg    360
   agtggttatt ttgactactg gggccaggga accctggtca ccgtctcctc agcctccacc    420
   aagggcccat cggtcttccc cctggcaccc tcctccaaga gcacctctgg gggcacagcg    480
   gccctgggct gcctggtcaa ggactacttc cccgaaccgg tgacggtgtc gtggaactca    540
   ggcgccct                                                             548
<210> 218
   <211> 122
   <212> PRT
   <213> Artificial
<220>
   <223> Antibody
<400> 218
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 219
   <211> 517
   <212> DNA
   <213> Artificial
<220>
   <223> Antibody
<400> 219
   atgagggtcc ccgctcagct cctggggctc ctgctgctct ggttcccagg tgccaggtgt      60
   gacatccaga tgacccagtc tccatcctcc ctgtctgcat ctgtaggaga cagagtcacc     120
   atcacttgcc gggcaagtca gggcattaga aatgatttag gctggtatca gcagaaacca     180
   gggaaagccc ctaagcgcct gatctatgct gcatccagtt tgcaaagtgg ggtcccatca     240
   aggttcagcg gcagtggatc tgggacagaa ttcactctca caatcagcag cctgcagcct     300
   gaagattttg caacttatta ctgtctacag cataatagtt acccgtggac gttcggccaa     360
   gggaccaagg tggaaatcaa acgaactgtg gctgcaccat ctgtcttcat cttcccgcca     420
   tctgatgagc agttgaaatc tggaactgcc tctgttgtgt gcctgctgaa taacttctat     480
   cccagagagg ccaaagtaca gtggaaggtg gataacg                              517
<210> 220
   <211> 107
   <212> PRT
   <213> Artificial
<220>
   <223> Antibody
<400> 220
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 221
   <211> 446
   <212> DNA
   <213> Artificial
<220>
   <223> Antibody
<400> 221
   ctgtggttct tccttctgct ggtggcagct cccagatggg tcctgtccca ggtgcagctg      60
   caggagtcgg gcccaggact ggtgaagcct tcacagaccc tgtccctcac ctgcactgtc     120
   tctggtggct ccatcagcag tggtgattac tactggagct ggatccgcca gcacccaggg     180
   aagggcctgg agtggattgg gtacatctat tacagtggga gcacctacta caacccgtcc     240
   ctcaagagtc gagttaccat gtcagtagac acgtctaaga accagttctc cctgaagctg     300
   agctctgtga ctgccgcgga cacggccgtg tattactgtg cgagagccga ttacgatttt     360
   tggagtggtc actttgactg ctggggccag ggaaccctgg tcaccgtctc ctcagcttcc     420
   accaagggcc ccatccgtct tccccc                                          446
<210> 222
   <211> 122
   <212> PRT
   <213> Artificial
<220>
   <223> Antibody
<400> 222
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Met Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Asp Tyr Asp Phe Trp Ser Gly His Phe Asp Cys Trp
               100                 105                 110
   Gly Gln Gly Thr Leu Val Thr Val Ser Ser
           115                 120
<210> 223
   <211> 419
   <212> DNA
   <213> Artificial
<220>
   <223> Antibody
<400> 223
   atgagggtcc ccgctcagct cctggggctc ctgctgctct ggttcccagg tgccaggtgt     60
   gacatccaga tgacccagtc tccatcctcc ctgtctgcat ctgtaggaga cagagtcacc    120
   atcacttgcc gggcaagtca gggcattaga gatgatttag gctggtatca gcagaaacca    180
   gggaaagccc ctaagcgcct gatctatgct gaatccagtt tgcaaagtgg ggtcccatca    240
   aggttcagcg gcagtggatc tgggacagaa ttcactctca caatcagcag cctgcagcct    300
   gaagattttg caacttatta ctgtctacag catcatagtt acccgtggac gttcggccaa    360
   gggaccaagg tggaaatcaa acgaactgtg gctgcaccat ctgtcttcat cttcccgcc     419
<210> 224
   <211> 107
   <212> PRT
   <213> Artificial
<220>
   <223> Antibody
<400> 224
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asp Asp
               20                  25                  30
   Leu Gly Trp Tyr Gln Gln Lys Pro Gly Lys Ala Pro Lys Arg Leu Ile
           35                  40                  45
   Tyr Ala Glu Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His His Ser Tyr Pro Trp
                   85                  90                  95
   Thr Phe Gly Gln Gly Thr Lys Val Glu Ile Lys
               100                 105
<210> 225
   <211> 504
   <212> DNA
   <213> Artificial
<220>
   <223> Antibody
<400> 225
   tggctgagct gggttttcct cgttgctctt ttaagaggtg tccagtgtca ggtgcagctg 60
   gtggagtctg ggggaggcgt ggtccagcct gggaggtccc tgagactctc ctgtgcagcg 120
   tctggattca ccttcaatag ctatgacatg cactgggtcc gccaggctcc aggcaagggg 180
   ctggagtggg tggcagttat atggtatgat ggaagtaata aatactatgc agactccgtg 240
   aagggccgat tcaccatctc tagagacaat tccaagaaca cgctgtatct gcaaatgaac 300
   agcctgagag ccgaggacac ggctgtgtat tactgtgcga gagaccgctt gtgtactaat 360
   ggtgtatgct atgaagacta cggtatggac gtctggggcc aagggaccac ggtcaccgtc 420
   tcctcagctt ccaccaaggg cccatccgtc ttccccctgg cgccctgctc caggagcacc 480
   tccgagagca cagccgccct gggc 504
<210> 226
   <211> 126
   <212> PRT
   <213> Artificial
<220>
   <223> Antibody
<400> 226
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Asn Ser Tyr
               20                  25                  30
   Asp Met His Trp Val Arg Gln Ala Pro Gly Lys Gly Leu Glu Trp Val
           35                  40                  45
   Ala Val Ile Trp Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Arg Leu Cys Thr Asn Gly Val Cys Tyr Glu Asp Tyr Gly
               100                 105                 110
   Met Asp Val Trp Gly Gln Gly Thr Thr Val Thr Val Ser Ser
           115                 120                 125
<210> 227
   <211> 504
   <212> DNA
   <213> Artificial
<220>
   <223> Antibody
<400> 227
   atgagggtcc ctgctcagct cctggggctc ctgctgctct ggctctcagg tgccagatgt     60
   gacatccaga tgacccagtc tccatcctcc ctgtctgcat ctgtaggaga cagagtcacc    120
   atcacttgcc aggcgagtca ggacattagc aactatttaa attggtatca gcagaaacca    180
   gggaaagccc ctaaggtcct gatctacgat gcatccaatt tggaaacagg ggtcccatca    240
   aggttcagtg gaagtggatc tgggacagat tttactttca ccatcagcag cctgcagcct    300
   gaagatgttg caacatatta ctgtcaacac tatgatactc tcccgctcac tttcggcgga    360
   gggaccaagg tggagatcaa acgaactgtg gctgcaccat ctgtcttcat cttcccgcca    420
   tctgatgagc agttgaaatc tggaactgcc tctgttgtgt gcctgctgaa taacttctat    480
   cccagagagg ccaaagtaca gtgg                                           504
<210> 228
   <211> 107
   <212> PRT
   <213> Artificial
<220>
   <223> Antibody
<400> 228
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asp Ile Ser Asn Tyr
               20                  25                  30
   Leu Asn Trp Tyr Gln Gln Lys Pro Gly Lys Ala Pro Lys Val Leu Ile
           35                  40                  45
   Tyr Asp Ala Ser Asn Leu Glu Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Val Ala Thr Tyr Tyr Cys Gln His Tyr Asp Thr Leu Pro Leu
                   85                  90                  95
   Thr Phe Gly Gly Gly Thr Lys Val Glu Ile Lys
               100                 105
<210> 229
   <211> 472
   <212> DNA
   <213> Artificial
<220>
   <223> Antibody
<400> 229
   ggactgtgca agaacatgaa acacctgtgg ttcttcctcc tgctggtggc agctcccaga      60
   tgggtcctgt cccaggtgca gctgcaggag tcgggcccag gactggtgaa gcctttacag     120
   accctgtccc tcacctgcac tgtctctggt ggctccatca gcagtggtga ttactactgg     180
   agctggatcc gccagcaccc agggaagggc ctggagtgga ttgggtacat ctattacagt     240
   gggaccacct actacaaccc gtccctcaag agtcgagtta ccatatcagt agacacgtct     300
   aagaaccagt tcgccctgaa gctgaactct gtgactgccg cggacacggc cgtgtattac     360
   tgtgcgagag ccgattacga tttttggagt ggttattttg actactgggg ccagggaacc     420
   ctggtcaccg tctcctcagc ttccaccaag ggcccatccg tcttccccct gg             472
<210> 230
   <211> 122
   <212> PRT
   <213> Artificial
<220>
   <223> Antibody
<400> 230
   Gln Val Gln Leu Gln Glu Ser Gly Pro Gly Leu Val Lys Pro Leu Gln
   1               5                   10                  15
   Thr Leu Ser Leu Thr Cys Thr Val Ser Gly Gly Ser Ile Ser Ser Gly
               20                  25                  30
   Asp Tyr Tyr Trp Ser Trp Ile Arg Gln His Pro Gly Lys Gly Leu Glu
           35                  40                  45
   Trp Ile Gly Tyr Ile Tyr Tyr Ser Gly Thr Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ala Leu Lys Leu Asn Ser Val Thr Ala Ala Asp Thr Ala Val Tyr Tyr
                   85                  90                  95
   Cys Ala Arg Ala Asp Tyr Asp Phe Trp Ser Gly Tyr Phe Asp Tyr Trp
               100                 105                 110
   Gly Gln Gly Thr Leu Val Thr Val Ser Ser
           115                 120
<210> 231
   <211> 531
   <212> DNA
   <213> Artificial
<220>
   <223> Antibody
<400> 231
   atgagggtcc ctgctcagct cctggggctc ctgctgctct ggttcccagg tgccaggtgt     60
   gacatccaga tgacccagtc tccatcctcc ctgtctgcat ctgtaggaga cagagtcacc    120
   atcacttgcc gggcaggtca gggcattaga aatgatttag gctggtatca gcagaaacca    180
   gggaaagccc ctcagcgcct gatctatgct gcatccagtt tgcaaagtgg ggtcccatca    240
   aggttcagcg gcagtggatc tgggacagaa ttctctctca caatctccag cctgcagcct    300
   gaagattttg caacttatta ctgtctacag cataatagtt acccgtggac gttcggccaa    360
   gggaccaagg tggaaatcaa acgaactgtg gctgcaccat ctgtcttcat cttcccgcca    420
   tctgatgagc agttgaaatc tggaactgcc tctgttgtgt gcctgctgaa taacttctat    480
   cccagagagg ccaaagtaca gtggaaggtg gataacgccc ttccaatcgg g             531
<210> 232
   <211> 107
   <212> PRT
   <213> Artificial
<220>
   <223> Antibody
<400> 232
   Asp Ile Gln Met Thr Gln Ser Pro Ser Ser Leu Ser Ala Ser Val Gly
   1               5                   10                  15
   Asp Arg Val Thr Ile Thr Cys Arg Ala Gly Gln Gly Ile Arg Asn Asp
               20                  25                  30
   Leu Gly Trp Tyr Gln Gln Lys Pro Gly Lys Ala Pro Gln Arg Leu Ile
               35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Ser Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 233
   <211> 26
   <212> DNA
   <213> Artificial
<220>
   <223> Primer
<220>
   <221> prim_transcript
   <222> (1)..(26)
<400> 233
   cgggatccat gtcctagcct aggggc 26
<210> 234
   <211> 39
   <212> DNA
   <213> Artificial
<220>
   <223> Primer
<220>
   <221> prim_transcript
   <222> (1)..(39)
<400> 234
   gctctagatt aatgatgatg atgatgatgt tgtcctaaa 39
<210> 235
   <211> 12
   <212> PRT
   <213> Artificial
<220>
   <223> U1-1 CDR-1-h
<400> 235
   Gly Gly Ser Ile Asn Ser Gly Asp Tyr Tyr Trp Ser
   1 5 10
<210> 236
   <211> 16
   <212> PRT
   <213> Artificial
<220>
   <223> U1-1 CDR-2-h
<400> 236
   Tyr Ile Tyr Tyr Ser Gly Ser Thr Tyr Tyr Asn Pro Ser Leu Lys Ser
   1 5 10 15
<210> 237
   <211> 12
   <212> PRT
   <213> Artificial
<220>
   <223> U1-1 CDR-3-h
<400> 237
   Ala Asp Tyr Asp Phe Trp Ser Gly Tyr Phe Asp Tyr
   1 5 10
<210> 238
   <211> 11
   <212> PRT
   <213> Artificial
<220>
   <223> U1-1 CDR-1-1
<400> 238
   Arg Ala Ser Gln Gly Ile Arg Asn Asp Leu Gly
   1 5 10
<210> 239
   <211> 7
   <212> PRT
   <213> Artificial
<220>
   <223> U1-1 CDR-2-1
<400> 239
   Ala Ala Ser Ser Leu Gln Ser
   1 5
<210> 240
   <211> 9
   <212> PRT
   <213> Artificial
<220>
   <223> U1-1 CDR-3-1
<400> 240
   Leu Gln His Asn Ser Tyr Pro Trp Thr
   1 5
<210> 241
   <211> 12
   <212> PRT
   <213> Artificial
<220>
   <223> U 1 - 2-CDR-1h
<400> 241
   Gly Gly Ser Ile Ser Ser Gly Asp Tyr Tyr Trp Ser
   1 5 10
<210> 242
   <211> 16
   <212> PRT
   <213> Artificial
<220>
   <223> U 1 - 2-CDR-2h
<400> 242
   Tyr Ile Tyr Tyr Ser Gly Ser Thr Tyr Tyr Asn Pro Ser Leu Arg Ser
   1 5 10 15
<210> 243
   <211> 12
   <212> PRT
   <213> Artificial
<220>
   <223> U 1 - 2-CDR-3h
<400> 243
   Ala Asp Tyr Asp Phe Trp Ser Gly Tyr Phe Asp Tyr
   1 5 10
<210> 244
   <211> 11
   <212> PRT
   <213> Artificial
<220>
   <223> U 1 - 2-CDR-1l
<400> 244
   Arg Ala Ser Gln Gly Ile Arg Asn Asp Leu Gly
   1 5 10
<210> 245
   <211> 7
   <212> PRT
   <213> Artificial
<220>
   <223> U 1 - 2-CDR-2l
<400> 245
   Ala Ala Ser Ser Leu Gln Ser
   1 5
<210> 246
   <211> 9
   <212> PRT
   <213> Artificial
<220>
   <223> U 1 - 2-CDR-3l
<400> 246
   Leu Gln His Asn Gly Tyr Pro Trp Thr
   1 5
<210> 247
   <211> 12
   <212> PRT
   <213> Artificial
<220>
   <223> U 1 - 3-CDR-1-h
<400> 247
   Gly Gly Ser Ile Ser Ser Gly Gly Tyr Tyr Trp Ser
   1 5 10
<210> 248
   <211> 16
   <212> PRT
   <213> Artificial
<220>
   <223> U 1 - 3-CDR-2h
<400> 248
   Tyr Ile Tyr Tyr Ser Gly Ser Thr Tyr Tyr Asn Pro Ser Leu Lys Ser
   1 5 10 15
<210> 249
   <211> 15
   <212> PRT
   <213> Artificial
<220>
   <223> U 1 - 3-CDR-3h
<400> 249
   Asp Gly Tyr Asp Ser Ser Gly Tyr Tyr His Gly Tyr Phe Asp Tyr
   1 5 10 15
<210> 250
   <211> 17
   <212> PRT
   <213> Artificial
<220>
   <223> U 1 - 3-CDR-1l
<400> 250
   Lys Ser Ser Gln Ser Val Leu Tyr Ser Ser Asn Asn Lys Asn Tyr Leu
   1 5 10 15
   Ala
<210> 251
   <211> 7
   <212> PRT
   <213> Artificial
<220>
   <223> U 1 - 3-CDR-2l
<400> 251
   Trp Ala Ser Thr Arg Glu Ser
   1 5
<210> 252
   <211> 9
   <212> PRT
   <213> Artificial
<220>
   <223> U 1 - 3-CDR-3l
<400> 252
   Gln Gln Tyr Tyr Ser Thr Pro Leu Thr
   1 5
<210> 253
   <211> 12
   <212> PRT
   <213> Artificial
<220>
   <223> U 1 - 4-CDR-1h
<400> 253
   Gly Gly Ser Ile Ser Ser Gly Asp Tyr Tyr Trp Ser
   1 5 10
<210> 254
   <211> 16
   <212> PRT
   <213> Artificial
<220>
   <223> U 1 - 4-CDR-2h
<400> 254
   Tyr Ile Tyr Tyr Ser Gly Ser Thr Tyr Tyr Asn Pro Ser Leu Lys Ser
   1 5 10 15
<210> 255
   <211> 12
   <212> PRT
   <213> Artificial
<220>
   <223> U 1 - 4-CDR-3h
<400> 255
   Ala Asp Tyr Asp Phe Trp Ser Gly Tyr Phe Asp Tyr
   1 5 10
<210> 256
   <211> 11
   <212> PRT
   <213> Artificial
<220>
   <223> U 1 - 4-CDR-1l
<400> 256
   Arg Ala Ser Gln Gly Ile Arg Asn Asp Leu Gly
   1 5 10
<210> 257
   <211> 7
   <212> PRT
   <213> Artificial
<220>
   <223> U 1 - 4-CDR-2l
<400> 257
   Ala Ala Ser Ser Leu Gln Ser
   1 5
<210> 258
   <211> 9
   <212> PRT
   <213> Artificial
<220>
   <223> U 1 - 4-CDR-3l
<400> 258
   Leu Gln His Asn Asn Tyr Pro Trp Thr
   1               5
<210> 259
   <211> 12
   <212> PRT
   <213> Artificial
<220>
   <223> U 1 - 5-CDR-1h
<400> 259
   Gly Gly Ser Ile Ser Ser Gly Asp Tyr Tyr Trp Ser
   1               5                   10
<210> 260
   <211> 16
   <212> PRT
   <213> Artificial
<220>
   <223> U 1 - 5-CDR-2h
<400> 260
   Tyr Ile Tyr Tyr Ser Gly Ser Thr Tyr Tyr Asn Pro Ser Leu Lys Ser
   1               5                   10                  15
<210> 261
   <211> 12
   <212> PRT
   <213> Artificial
<220>
   <223> U 1 - 5-CDR-3h
<400> 261
   Ala Asp Tyr Asp Phe Trp Ser Gly Tyr Phe Asp Tyr
   1               5                   10
<210> 262
   <211> 11
   <212> PRT
   <213> Artificial
<220>
   <223> U1 - 5-CDR-1l
<400> 262
   Arg Ala Ser Gln Gly Ile Arg Asn Asp Leu Gly
   1               5                   10
<210> 263
   <211> 7
   <212> PRT
   <213> Artificial
<220>
   <223> U 1 - 5-CDR-2l
<400> 263
   Ala Ala Ser Ser Leu Gln Ser
   1               5
<210> 264
   <211> 9
   <212> PRT
   <213> Artificial
<220>
   <223> U 1 - 5-CDR-3l
<400> 264
   Leu Gln His Asn Thr Tyr Pro Trp Thr
   1               5
<210> 265
   <211> 12
   <212> PRT
   <213> Artificial
<220>
   <223> U1-6-CDR-1h
<400> 265
   Gly Gly Ser Ile Ser Ser Gly Asp Tyr Tyr Trp Ser
   1               5                   10
<210> 266
   <211> 16
   <212> PRT
   <213> Artificial
<220>
   <223> U1-6-CDR-2h
<400> 266
   Tyr Ile Tyr Tyr Ser Gly Ser Thr Tyr Tyr Asn Pro Ser Leu Lys Ser
   1               5                   10                  15
<210> 267
   <211> 12
   <212> PRT
   <213> Artificial
<220>
   <223> U1-6-CDR-3h
<400> 267
   Ala Asp Tyr Asp Phe Trp Asn Gly Tyr Phe Asp Tyr
   1               5                   10
<210> 268
   <211> 11
   <212> PRT
   <213> Artificial
<220>
   <223> U1-6-CDR-1l
<400> 268
   Arg Ala Ser Gln Gly Ile Arg Asn Asp Leu Gly
   1               5                   10
<210> 269
   <211> 7
   <212> PRT
   <213> Artificial
<220>
   <223> U1-6-CDR-2l
<400> 269
   Ala Ala Ser Ser Leu Gln Ser
   1               5
<210> 270
   <211> 9
   <212> PRT
   <213> Artificial
<220>
   <223> U1-6-CDR-3l
<400> 270
   Leu Gln His Asn Thr Tyr Pro Trp Thr
   1               5
<210> 271
   <211> 12
   <212> PRT
   <213> Artificial
<220>
   <223> U1 - 7-CDR-1h
<400> 271
   Gly Gly Ser Ile Ser Ser Gly Asp Tyr Tyr Trp Ser
   1 5 10
<210> 272
   <211> 16
   <212> PRT
   <213> Artificial
<220>
   <223> U 1 - 7-CDR-2h
<400> 272
   Tyr Ile Tyr Tyr Ser Gly Ser Thr Tyr Tyr Asn Pro Ser Leu Lys Ser
   1 5 10 15
<210> 273
   <211> 12
   <212> PRT
   <213> Artificial
<220>
   <223> U 1 - 7-CDR-3h
<400> 273
   Ala Asp Tyr Asp Phe Trp Ser Gly Tyr Phe Asp Tyr
   1 5 10
<210> 274
   <211> 11
   <212> PRT
   <213> Artificial
<220>
   <223> U1 - 7-CDR-1l
<400> 274
   Arg Ala Ser Gln Asp Ile Arg Asn Asp Leu Gly
   1 5 10
<210> 275
   <211> 7
   <212> PRT
   <213> Artificial
<220>
   <223> U1 - 7-CDR-2l
<400> 275
   Ala Ala Ser Ser Leu Gln Ser
   1 5
<210> 276
   <211> 9
   <212> PRT
   <213> Artificial
<220>
   <223> U1 - 7-CDR-3l
<400> 276
   Leu Gln His Asn Ser Tyr Pro Trp Thr
   1 5
<210> 277
   <211> 10
   <212> PRT
   <213> Artificial
<220>
   <223> U1 - 8-CDR-1h
<400> 277
   Gly Tyr Thr Leu Thr Glu Leu Ser Met Tyr
   1 5 10
<210> 278
   <211> 17
   <212> PRT
   <213> Artificial
<220>
   <223> U 1 - 8-CDR-2h
<400> 278
   Gly Phe Asp Pro Glu Asp Gly Glu Thr Ile Tyr Ala Gln Lys Phe Gln
   1 5 10 15
   Gly
<210> 279
   <211> 8
   <212> PRT
   <213> Artificial
<220>
   <223> U 1 - 8-CDR-3h
<400> 279
   Gly Trp Asn Tyr Val Phe Asp Tyr
   1 5
<210> 280
   <211> 16
   <212> PRT
   <213> Artificial
<220>
   <223> U1 - 8-CDR-1l
<400> 280
   Arg Ser Ser Gln Ser Leu Leu His Ser Asn Gly Tyr Asn Tyr Leu Asp
   1               5                   10                  15
<210> 281
   <211> 7
   <212> PRT
   <213> Artificial
<220>
   <223> U1 - 8-CDR-2l
<400> 281
   Leu Asp Ser His Arg Ala Ser
   1               5
<210> 282
   <211> 9
   <212> PRT
   <213> Artificial
<220>
   <223> U1 - 8-CDR-3l
<400> 282
   Met Gln Ala Leu Gln Thr Pro Leu Thr
   1               5
<210> 283
   <211> 12
   <212> PRT
   <213> Artificial
<220>
   <223> U1 - 9-CDR-1h
<400> 283
   Gly Gly Ser Ile Ser Ser Gly Asp Tyr Tyr Trp Ser
   1               5                   10
<210> 284
   <211> 16
   <212> PRT
   <213> Artificial
<220>
   <223> U 1 - 9-CDR-2h
<400> 284
   Tyr Ile Tyr Tyr Ser Gly Ser Thr Tyr Tyr Asn Pro Ser Leu Lys Ser
   1 5 10 15
<210> 285
   <211> 12
   <212> PRT
   <213> Artificial
<220>
   <223> U 1 - 9-CDR-3h
<400> 285
   Ala Asp Tyr Asp Phe Trp Asn Gly Tyr Phe Asp Tyr
   1 5 10
<210> 286
   <211> 11
   <212> PRT
   <213> Artificial
<220>
   <223> U1 - 9-CDR-1l
<400> 286
   Arg Ala Ser Gln Asp Ile Arg Asn Asp Leu Gly
   1 5 10
<210> 287
   <211> 7
   <212> PRT
   <213> Artificial
<220>
   <223> U1 - 9-CDR-2l
<400> 287
   Ala Ala Ser Ser Leu Gln Ser
   1 5
<210> 288
   <211> 9
   <212> PRT
   <213> Artificial
<220>
   <223> U1 - 9-CDR-3l
<400> 288
   Leu Gln His Asn Ser Tyr Pro Trp Thr
   1 5
<210> 289
   <211> 12
   <212> PRT
   <213> Artificial
<220>
   <223> U1-10-CDR-1h
<400> 289
   Gly Gly Ser Ile Ser Ser Gly Asp Tyr Tyr Trp Ser
   1               5                   10
<210> 290
   <211> 16
   <212> PRT
   <213> Artificial
<220>
   <223> U1-10-CDR-2h
<400> 290
   Tyr Ile Tyr Tyr Ser Gly Ser Thr Tyr Tyr Asn Pro Ser Leu Lys Ser
   1               5                   10                  15
<210> 291
   <211> 12
   <212> PRT
   <213> Artificial
<220>
   <223> U1-10-CDR-3h
<400> 291
   Ala Asp Tyr Asp Phe Trp Ser Gly Tyr Phe Asp Tyr
   1               5                   10
<210> 292
   <211> 11
   <212> PRT
   <213> Artificial
<220>
   <223> U1-10-CDR-1l
<400> 292
   Arg Ala Ser Gln Gly Ile Arg Asn Asp Leu Gly
   1               5                   10
<210> 293
   <211> 7
   <212> PRT
   <213> Artificial
<220>
   <223> U1-10-CDR-2l
<400> 293
   Ala Ala Ser Ser Leu Gln Ser
   1 5
<210> 294
   <211> 9
   <212> PRT
   <213> Artificial
<220>
   <223> U1-10-CDR-3l
<400> 294
   Leu Gln His Asn Asn Tyr Pro Trp Thr
   1 5
<210> 295
   <211> 12
   <212> PRT
   <213> Artificial
<220>
   <223> U1 -11-CDR-1h
<400> 295
   Gly Gly Ser Ile Ser Ser Gly Asp Tyr Tyr Trp Ser
   1 5 10
<210> 296
   <211> 16
   <212> PRT
   <213> Artificial
<220>
   <223> U1 -11-CDR-2h
<400> 296
   Tyr Ile Tyr Tyr Ser Gly Ser Thr Tyr Tyr Asn Pro Ser Leu Lys Ser
   1 5 10 15
<210> 297
   <211> 12
   <212> PRT
   <213> Artificial
<220>
   <223> U1 -11-CDR-3h
<400> 297
   Ala Asp Tyr Asp Phe Trp Ser Gly Tyr Phe Asp Tyr
   1 5 10
<210> 298
   <211> 11
   <212> PRT
   <213> Artificial
<220>
   <223> U1 -11-CDR-1l
<400> 298
   Arg Ala Ser Gln Gly Ile Arg Asn Asp Leu Gly
   1               5                   10
<210> 299
   <211> 7
   <212> PRT
   <213> Artificial
<220>
   <223> U1 -11-CDR-2l
<400> 299
   Ala Ala Ser Ser Leu Gln Ser
   1               5
<210> 300
   <211> 9
   <212> PRT
   <213> Artificial
<220>
   <223> U1 -11-CDR-3l
<400> 300
   Leu Gln His Asn Thr Tyr Pro Trp Thr
   1               5
<210> 301
   <211> 12
   <212> PRT
   <213> Artificial
<220>
   <223> U1 - 12-CDR-1h
<400> 301
   Gly Gly Ser Ile Ser Ser Gly Asp Tyr Tyr Trp Ser
   1               5                   10
<210> 302
   <211> 16
   <212> PRT
   <213> Artificial
<220>
   <223> U 1 - 12-CDR-2h
<400> 302
   Tyr Ile Tyr Tyr Ser Gly Ser Thr Tyr Tyr Asn Pro Ser Leu Lys Ser
   1 5 10 15
<210> 303
   <211> 12
   <212> PRT
   <213> Artificial
<220>
   <223> U1-12-CDR-3h
<400> 303
   Ala Asp Tyr Asp Phe Trp Ser Gly Tyr Phe Asp Tyr
   1 5 10
<210> 304
   <211> 11
   <212> PRT
   <213> Artificial
<220>
   <223> U1-12-CDR-1l
<400> 304
   Arg Ala Ser Gln Gly Ile Arg Asn Asp Leu Gly
   1 5 10
<210> 305
   <211> 7
   <212> PRT
   <213> Artificial
<220>
   <223> U1-12-CDR-2l
<400> 305
   Ala Ala Ser Ser Leu Gln Ser
   1 5
<210> 306
   <211> 9
   <212> PRT
   <213> Artificial
<220>
   <223> U1-12-CDR-3l
<400> 306
   Leu Gln His Asn Asn Tyr Pro Trp Thr
   1 5
<210> 307
   <211> 12
   <212> PRT
   <213> Artificial
<220>
   <223> U1 -13-CDR-1h
<400> 307
   Gly Gly Ser Ile Ser Ser Gly Gly Tyr Tyr Trp Ser
   1 5 10
<210> 308
   <211> 16
   <212> PRT
   <213> Artificial
<220>
   <223> U 1 -13-CDR-2h
<400> 308
   Tyr Ile Tyr Tyr Ser Gly Ser Thr Tyr Tyr Asn Pro Ser Leu Lys Ser
   1 5 10 15
<210> 309
   <211> 7
   <212> PRT
   <213> Artificial
<220>
   <223> U 1 -13-CDR-3h
<400> 309
   Glu Asp Asp Gly Met Asp Val
   1 5
<210> 310
   <211> 16
   <212> PRT
   <213> Artificial
<220>
   <223> U1 -13-CDR-1l
<400> 310
   Arg Ser Ser Gln Ser Leu Leu His Ser Asn Gly Tyr Asn Tyr Leu Glu
   1 5 10 15
<210> 311
   <211> 7
   <212> PRT
   <213> Artificial
<220>
   <223> U1 -13-CDR-2l
<400> 311
   Leu Gly Ser Asn Arg Ala Ser
   1 5
<210> 312
   <211> 9
   <212> PRT
   <213> Artificial
<220>
   <223> U1 -13-CDR-3l
<400> 312
   Met Gln Ala Leu Gln Thr Pro Ile Thr
   1 5
<210> 313
   <211> 12
   <212> PRT
   <213> Artificial
<220>
   <223> U1-14-CDR-1h
<400> 313
   Gly Gly Ser Ile Ser Ser Gly Asp Tyr Tyr Trp Ser
   1 5 10
<210> 314
   <211> 16
   <212> PRT
   <213> Artificial
<220>
   <223> U1-14-CDR-2h
<400> 314
   Tyr Ile Tyr Tyr Ser Gly Ser Thr Tyr Tyr Asn Pro Ser Leu Lys Ser
   1 5 10 15
<210> 315
   <211> 12
   <212> PRT
   <213> Artificial
<220>
   <223> U1-14-CDR-3h
<400> 315
   Ala Asp Tyr Asp Phe Trp Ser Gly Tyr Phe Asp Tyr
   1 5 10
<210> 316
   <211> 11
   <212> PRT
   <213> Artificial
<220>
   <223> U1-14-CDR-1l
<400> 316
   Arg Ala Ser Gln Gly Ile Arg Asn Asp Leu Gly
   1 5 10
<210> 317
   <211> 7
   <212> PRT
   <213> Artificial
<220>
   <223> U1-14-CDR-2l
<400> 317
   Ala Ala Ser Ser Leu Gln Ser
   1 5
<210> 318
   <211> 9
   <212> PRT
   <213> Artificial
<220>
   <223> U1-14-CDR-3l
<400> 318
   Leu Gln His Asn Thr Tyr Pro Trp Thr
   1 5
<210> 319
   <211> 12
   <212> PRT
   <213> Artificial
<220>
   <223> U1-15-CDR-1h
<400> 319
   Gly Gly Ser Val Ser Ser Gly Gly Tyr Tyr Trp Ser
   1 5 10
<210> 320
   <211> 16
   <212> PRT
   <213> Artificial
<220>
   <223> U1-15-CDR-2h
<400> 320
   Tyr Ile Tyr Tyr Ser Gly Ser Thr Asn Tyr Asn Pro Ser Leu Lys Ser
   1 5 10 15
<210> 321
   <211> 14
   <212> PRT
   <213> Artificial
<220>
   <223> U1-15-CDR-3h
<400> 321
   Asp Gly Asp Val Asp Thr Ala Met Val Asp Ala Phe Asp Ile
   1 5 10
<210> 322
   <211> 12
   <212> PRT
   <213> Artificial
<220>
   <223> U1-15-CDR-1l
<400> 322
   Arg Ala Ser Gln Ser Leu Ser Gly Asn Tyr Leu Ala
   1 5 10
<210> 323
   <211> 7
   <212> PRT
   <213> Artificial
<220>
   <223> U1-15-CDR-2l
<400> 323
   Gly Ala Ser Ser Arg Ala Thr
   1 5
<210> 324
   <211> 9
   <212> PRT
   <213> Artificial
<220>
   <223> U1-15-CDR-3l
<400> 324
   Gln Gln Tyr Asp Arg Ser Pro Leu Thr
   1 5
<210> 325
   <211> 12
   <212> PRT
   <213> Artificial
<220>
   <223> U1-16-CDR-1h
<400> 325
   Gly Gly Ser Ile Ser Ser Gly Asp Tyr Tyr Trp Ser
   1               5                   10
<210> 326
   <211> 16
   <212> PRT
   <213> Artificial
<220>
   <223> U1-16-CDR-2h
<400> 326
   Tyr Ile Tyr Tyr Ser Gly Ser Thr Tyr Tyr Asn Pro Ser Leu Lys Ser
   1               5                   10                  15
<210> 327
   <211> 12
   <212> PRT
   <213> Artificial
<220>
   <223> U1-16-CDR-3h
<400> 327
   Ala Asp Tyr Asp Phe Trp Ser Gly Tyr Phe Asp Tyr
   1               5                   10
<210> 328
   <211> 11
   <212> PRT
   <213> Artificial
<220>
   <223> U1-16-CDR-1l
<400> 328
   Arg Ala Ser Gln Gly Ile Arg Asn Asp Leu Gly
   1               5                   10
<210> 329
   <211> 7
   <212> PRT
   <213> Artificial
<220>
   <223> U1-16-CDR-2l
<400> 329
   Ala Ala Ser Ser Leu Gln Ser
   1 5
<210> 330
   <211> 9
   <212> PRT
   <213> Artificial
<220>
   <223> U1-16-CDR-3l
<400> 330
   Leu Gln His Asn Ser Tyr Pro Trp Thr
   1 5
<210> 331
   <211> 12
   <212> PRT
   <213> Artificial
<220>
   <223> U1-17-CDR-1h
<400> 331
   Gly Gly Ser Ile Ser Ser Gly Asp Tyr Tyr Trp Ser
   1 5 10
<210> 332
   <211> 16
   <212> PRT
   <213> Artificial
<220>
   <223> U1-17-CDR-2h
<400> 332
   Tyr Ile Tyr Tyr Ser Gly Ser Thr Tyr Tyr Asn Ser Ser Leu Lys Ser
   1 5 10 15
<210> 333
   <211> 12
   <212> PRT
   <213> Artificial
<220>
   <223> U1-17-CDR-3h
<400> 333
   Ala Asp Tyr Asp Phe Trp Ser Gly Tyr Phe Asp Tyr
   1 5 10
<210> 334
   <211> 11
   <212> PRT
   <213> Artificial
<220>
   <223> U1-17-CDR-1l
<400> 334
   Arg Ala Ser Gln Gly Ile Arg Asn Asp Leu Gly
   1               5                   10
<210> 335
   <211> 7
   <212> PRT
   <213> Artificial
<220>
   <223> U1-17-CDR-2l
<400> 335
   Ala Ala Ser Ser Leu Gln Ser
   1               5
<210> 336
   <211> 9
   <212> PRT
   <213> Artificial
<220>
   <223> U1-17-CDR-3l
<400> 336
   Leu Gln His Asn Ser Tyr Pro Trp Thr
   1               5
<210> 337
   <211> 12
   <212> PRT
   <213> Artificial
<220>
   <223> U1-18-CDR-1h
<400> 337
   Gly Gly Ser Ile Ser Ser Gly Asp Tyr Tyr Trp Ser
   1               5                   10
<210> 338
   <211> 16
   <212> PRT
   <213> Artificial
<220>
   <223> U1-18-CDR-2h
<400> 338
   Tyr Ile Tyr Tyr Ser Gly Ser Thr Tyr Tyr Asn Pro Ser Leu Lys Ser
   1 5 10 15
<210> 339
   <211> 12
   <212> PRT
   <213> Artificial
<220>
   <223> U1-18-CDR-3h
<400> 339
   Ala Asp Tyr Asp Phe Trp Ser Gly Tyr Phe Asp Tyr
   1 5 10
<210> 340
   <211> 11
   <212> PRT
   <213> Artificial
<220>
   <223> U1-18-CDR-1l
<400> 340
   Arg Ala Ser Gln Gly Ile Arg Asn Asp Leu Gly
   1 5 10
<210> 341
   <211> 7
   <212> PRT
   <213> Artificial
<220>
   <223> U1-18-CDR-2l
<400> 341
   Ala Ala Ser Ser Leu Gln Ser
   1 5
<210> 342
   <211> 9
   <212> PRT
   <213> Artificial
<220>
   <223> U1-18-CDR-3l
<400> 342
   Leu Gln His Asn Ser Tyr Pro Trp Thr
   1 5
<210> 343
   <211> 12
   <212> PRT
   <213> Artificial
<220>
   <223> U1-19-CDR-1h
<400> 343
   Gly Gly Ser Ile Ser Ser Gly Asp Tyr Tyr Trp Ser
   1               5                   10
<210> 344
   <211> 16
   <212> PRT
   <213> Artificial
<220>
   <223> U1-19-CDR-2h
<400> 344
   Tyr Ile Tyr Tyr Ser Gly Ser Thr Tyr Tyr Asn Pro Ser Leu Lys Ser
   1               5                   10                  15
<210> 345
   <211> 12
   <212> PRT
   <213> Artificial
<220>
   <223> U1-19-CDR-3h
<400> 345
   Gly Asp Tyr Asp Phe Trp Ser Gly Glu Phe Asp Tyr
   1               5                   10
<210> 346
   <211> 12
   <212> PRT
   <213> Artificial
<220>
   <223> U1-20-CDR-1h
<400> 346
   Gly Gly Ser Ile Ser Ser Gly Gly Tyr Tyr Trp Ser
   1               5                   10
<210> 347
   <211> 16
   <212> PRT
   <213> Artificial
<220>
   <223> U1-20-CDR-2h
<400> 347
   Tyr Ile Tyr Asp Ser Gly Ser Thr Tyr Tyr Asn Pro Ser Leu Lys Ser
   1 5 10 15
<210> 348
   <211> 19
   <212> PRT
   <213> Artificial
<220>
   <223> U1-20-CDR-3h
<400> 348
   Asp Gln Gly Gln Asp Gly Tyr Ser Tyr Gly Tyr Gly Tyr Tyr Tyr Gly
   1 5 10 15
   Met Asp Val
<210> 349
   <211> 11
   <212> PRT
   <213> Artificial
<220>
   <223> U1-20-CDR-1l
<400> 349
   Gln Ala Ser Gln Asp Ile Ser Asn Tyr Leu Asn
   1 5 10
<210> 350
   <211> 7
   <212> PRT
   <213> Artificial
<220>
   <223> U1-20-CDR-2l
<400> 350
   Val Ala Ser Asn Leu Glu Thr
   1 5
<210> 351
   <211> 9
   <212> PRT
   <213> Artificial
<220>
   <223> U1-20-CDR-3l
<400> 351
   Gln Gln Cys Asp Asn Leu Pro Leu Thr
   1 5
<210> 352
   <211> 12
   <212> PRT
   <213> Artificial
<220>
   <223> U1-21-CDR-1h
<400> 352
   Gly Gly Ser Ile Ser Ser Gly Asp Tyr Tyr Trp Ser
   1               5                   10
<210> 353
   <211> 16
   <212> PRT
   <213> Artificial
<220>
   <223> U1-21-CDR-2h
<400> 353
   Tyr Ile Tyr Tyr Ser Gly Ser Thr Tyr Tyr Asn Pro Ser Leu Lys Ser
   1               5                   10                  15
<210> 354
   <211> 12
   <212> PRT
   <213> Artificial
<220>
   <223> U1-21-CDR-3h
<400> 354
   Ala Asp Tyr Asp Phe Trp Ser Gly Tyr Phe Asp Tyr
   1               5                   10
<210> 355
   <211> 11
   <212> PRT
   <213> Artificial
<220>
   <223> U1-21-CDR-1l
<400> 355
   Arg Ala Ser Gln Asp Ile Arg Asn Asp Leu Gly
   1               5                   10
<210> 356
   <211> 7
   <212> PRT
   <213> Artificial
<220>
   <223> U1-21-CDR-2l
<400> 356
   Ala Ala Ser Arg Leu Gln Ser
   1 5
<210> 357
   <211> 9
   <212> PRT
   <213> Artificial
<220>
   <223> U1-21-CDR-3l
<400> 357
   Leu Gln His Asn Ser Tyr Pro Trp Thr
   1 5
<210> 358
   <211> 12
   <212> PRT
   <213> Artificial
<220>
   <223> U1-22-CDR-1h
<400> 358
   Gly Gly Ser Ile Ser Ser Gly Asp Tyr Tyr Trp Ser
   1 5 10
<210> 359
   <211> 16
   <212> PRT
   <213> Artificial
<220>
   <223> U1-22-CDR-2h
<400> 359
   Tyr Ile Tyr Tyr Ser Gly Ser Thr Tyr Tyr Asn Pro Ser Leu Lys Ser
   1 5 10 15
<210> 360
   <211> 12
   <212> PRT
   <213> Artificial
<220>
   <223> U1-22-CDR-3h
<400> 360
   Ala Asp Tyr Asp Phe Trp Ser Gly Tyr Phe Asp Tyr
   1 5 10
<210> 361
   <211> 11
   <212> PRT
   <213> Artificial
<220>
   <223> U1-22-CDR-1l
<400> 361
   Arg Ala Ser Gln Gly Ile Arg Asn Asp Leu Gly
   1               5                   10
<210> 362
   <211> 7
   <212> PRT
   <213> Artificial
<220>
   <223> U1-22-CDR-2l
<400> 362
   Ala Ala Ser Ser Leu Gln Asn
   1               5
<210> 363
   <211> 9
   <212> PRT
   <213> Artificial
<220>
   <223> U1-22-CDR-3l
<400> 363
   Leu Gln His Asn Ser Tyr Pro Trp Thr
   1               5
<210> 364
   <211> 12
   <212> PRT
   <213> Artificial
<220>
   <223> U1-23-CDR-1h
<400> 364
   Gly Gly Ser Ile Ser Ser Gly Asp Tyr Tyr Trp Ser
   1               5                   10
<210> 365
   <211> 16
   <212> PRT
   <213> Artificial
<220>
   <223> U1-23-CDR-2h
<400> 365
   Tyr Ile Tyr Tyr Ser Gly Ser Thr Tyr Tyr Asn Pro Ser Leu Lys Ser
   1               5                   10                  15
<210> 366
   <211> 12
   <212> PRT
   <213> Artificial
<220>
   <223> U1-23-CDR-3h
<400> 366
   Ala Asp Tyr Asp Phe Trp Ser Gly Tyr Phe Asp Tyr
   1               5                   10
<210> 367
   <211> 11
   <212> PRT
   <213> Artificial
<220>
   <223> U1-23-CDR-1l
<400> 367
   Arg Ala Ser Gln Gly Ile Arg Asn Asp Leu Gly
   1               5                   10
<210> 368
   <211> 7
   <212> PRT
   <213> Artificial
<220>
   <223> U1-23-CDR-2l
<400> 368
   Ala Ala Ser Ser Leu Gln Ser
   1               5
<210> 369
   <211> 9
   <212> PRT
   <213> Artificial
<220>
   <223> U1-23-CDR-3l
<400> 369
   Leu Gln His Asn Ser Tyr Pro Trp Thr
   1               5
<210> 370
   <211> 12
   <212> PRT
   <213> Artificial
<220>
   <223> U1-24-CDR-1h
<400> 370
   Gly Gly Ser Ile Ser Ser Gly Asp Tyr Tyr Trp Ser
   1               5                   10
<210> 371
   <211> 16
   <212> PRT
   <213> Artificial
<220>
   <223> U1-24-CDR-2h
<400> 371
   Tyr Ile Tyr Tyr Ser Gly Ser Thr Tyr Tyr Asn Pro Ser Leu Lys Ser
   1               5                   10                  15
<210> 372
   <211> 12
   <212> PRT
   <213> Artificial
<220>
   <223> U1-24-CDR-3h
<400> 372
   Ala Asp Tyr Asp Phe Trp Asn Gly Tyr Phe Asp Tyr
   1               5                   10
<210> 373
   <211> 11
   <212> PRT
   <213> Artificial
<220>
   <223> U1-24-CDR-1l
<400> 373
   Arg Ala Ser Gln Gly Ile Arg Asn Asp Leu Gly
   1               5                   10
<210> 374
   <211> 7
   <212> PRT
   <213> Artificial
<220>
   <223> U1-24-CDR-2l
<400> 374
   Ala Ala Ser Ser Leu Gln Ser
   1 5
<210> 375
   <211> 9
   <212> PRT
   <213> Artificial
<220>
   <223> U1-24-CDR-3l
<400> 375
   Leu Gln His Asn Asn Tyr Pro Trp Thr
   1 5
<210> 376
   <211> 12
   <212> PRT
   <213> Artificial
<220>
   <223> U1-25-CDR-1h
<400> 376
   Gly Gly Ser Ile Ser Ser Gly Asp Tyr Tyr Trp Ser
   1 5 10
<210> 377
   <211> 16
   <212> PRT
   <213> Artificial
<220>
   <223> U1-25-CDR-2h
<400> 377
   Tyr Ile Tyr Tyr Ser Gly Ser Thr Tyr Tyr Asn Pro Ser Leu Lys Ser
   1 5 10 15
<210> 378
   <211> 12
   <212> PRT
   <213> Artificial
<220>
   <223> U1-25-CDR-3h
<400> 378
   Ala Asp Tyr Asp Phe Trp Ser Gly Tyr Phe Asp Tyr
   1 5 10
<210> 379
   <211> 11
   <212> PRT
   <213> Artificial
<220>
   <223> U1-25-CDR-1l
<400> 379
   Arg Ala Ser Gln Gly Ile Arg Asn Asp Leu Gly
   1 5 10
<210> 380
   <211> 7
   <212> PRT
   <213> Artificial
<220>
   <223> U1-25-CDR-2l
<400> 380
   Ala Ala Ser Ser Leu Gln Asn
   1 5
<210> 381
   <211> 9
   <212> PRT
   <213> Artificial
<220>
   <223> U1-25-CDR-3l
<400> 381
   Leu Gln His Asn Ser Tyr Pro Trp Thr
   1 5
<210> 382
   <211> 12
   <212> PRT
   <213> Artificial
<220>
   <223> U1-26-CDR-1h
<400> 382
   Gly Gly Ser Ile Ser Ser Gly Asp Tyr Tyr Trp Ser
   1 5 10
<210> 383
   <211> 16
   <212> PRT
   <213> Artificial
<220>
   <223> U1-26-CDR-2h
<400> 383
   Tyr Ile Tyr Tyr Ser Gly Ser Thr Tyr Tyr Asn Pro Ser Leu Lys Ser
   1 5 10 15
<210> 384
   <211> 12
   <212> PRT
   <213> Artificial
<220>
   <223> U1-26-CDR-3h
<400> 384
   Ala Asp Tyr Asp Phe Trp Ser Gly Tyr Phe Asp Phe
   1 5 10
<210> 385
   <211> 11
   <212> PRT
   <213> Artificial
<220>
   <223> U1-26-CDR-1l
<400> 385
   Arg Ala Ser Gln Gly Ile Arg Asn Asp Leu Gly
   1 5 10
<210> 386
   <211> 7
   <212> PRT
   <213> Artificial
<220>
   <223> U1-26-CDR-2l
<400> 386
   Ala Ala Ser Ser Leu Gln Ser
   1 5
<210> 387
   <211> 9
   <212> PRT
   <213> Artificial
<220>
   <223> U1-26-CDR-3l
<400> 387
   Leu Gln His Asn Gly Tyr Pro Trp Thr
   1 5
<210> 388
   <211> 12
   <212> PRT
   <213> Artificial
<220>
   <223> U1-27-CDR-1h
<400> 388
   Gly Gly Ser Ile Ser Ser Gly Asp Tyr Tyr Trp Ser
   1               5                   10
<210> 389
   <211> 16
   <212> PRT
   <213> Artificial
<220>
   <223> U1-27-CDR-2h
<400> 389
   Tyr Ile Tyr Tyr Ser Gly Ser Thr Tyr Tyr Asn Pro Ser Leu Lys Ser
   1               5                   10                  15
<210> 390
   <211> 12
   <212> PRT
   <213> Artificial
<220>
   <223> U1-27-CDR-3h
<400> 390
   Ala Asp Tyr Asp Phe Trp Ser Gly Tyr Phe Asp Phe
   1               5                   10
<210> 391
   <211> 11
   <212> PRT
   <213> Artificial
<220>
   <223> U1-27-CDR-1l
<400> 391
   Arg Ala Ser Gln Gly Ile Arg Asn Asp Leu Gly
   1               5                   10
<210> 392
   <211> 7
   <212> PRT
   <213> Artificial
<220>
   <223> U1-27-CDR-2l
<400> 392
   Ala Ala Ser Ser Leu Gln Ser
   1               5
<210> 393
   <211> 9
   <212> PRT
   <213> Artificial
<220>
   <223> U1-27-CDR-3l
<400> 393
   Leu Gln His Asn Gly Tyr Pro Trp Thr
   1               5
<210> 394
   <211> 12
   <212> PRT
   <213> Artificial
<220>
   <223> U1-28-CDR-1h
<400> 394
   Gly Gly Ser Ile Ser Ser Gly Asp Tyr Tyr Trp Ser
   1               5                   10
<210> 395
   <211> 16
   <212> PRT
   <213> Artificial
<220>
   <223> U1-28-CDR-2h
<400> 395
   Tyr Ile Tyr Tyr Ser Gly Ser Thr Tyr Tyr Asn Pro Ser Leu Lys Ser
   1               5                   10                  15
<210> 396
   <211> 12
   <212> PRT
   <213> Artificial
<220>
   <223> U1-28-CDR-1l
<400> 396
   Ala Asp Tyr Asp Phe Trp Ser Gly Tyr Phe Asp Ser
   1               5                   10
<210> 397
   <211> 11
   <212> PRT
   <213> Artificial
<220>
   <223> U1-28-CDR-1l
<400> 397
   Arg Ala Ser Gln Gly Ile Arg Asn Asp Leu Gly
   1               5                   10
<210> 398
   <211> 7
   <212> PRT
   <213> Artificial
<220>
   <223> U1-28-CDR-2l
<400> 398
   Ala Ala Ser Ser Leu Gln Ser
   1               5
<210> 399
   <211> 9
   <212> PRT
   <213> Artificial
<220>
   <223> U1-28-CDR-3l
<400> 399
   Leu Gln His Asn Gly Tyr Pro Trp Thr
   1               5
<210> 400
   <211> 10
   <212> PRT
   <213> Artificial
<220>
   <223> U1-29-CDR-1h
<400> 400
   Gly Phe Thr Phe Asn Ser Tyr Asp Met His
   1               5                   10
<210> 401
   <211> 17
   <212> PRT
   <213> Artificial
<220>
   <223> U1-29-CDR-2h
<400> 401
   Val Ile Trp Tyr Asp Gly Ser Asn Lys Tyr Tyr Ala Asp Ser Val Lys
   1 5 10 15
   Gly
<210> 402
   <211> 17
   <212> PRT
   <213> Artificial
<220>
   <223> U1-29-CDR-3h
<400> 402
   Asp Arg Leu Cys Thr Asn Gly Val Cys Tyr Glu Asp Tyr Gly Met Asp
   1 5 10 15
   Val
<210> 403
   <211> 11
   <212> PRT
   <213> Artificial
<220>
   <223> U1-29-CDR-1l
<400> 403
   Gln Ala Ser Gln Asp Ile Ser Asn Tyr Leu Asn
   1 5 10
<210> 404
   <211> 7
   <212> PRT
   <213> Artificial
<220>
   <223> U1-29-CDR-2l
<400> 404
   Asp Ala Ser Asn Leu Glu Thr
   1 5
<210> 405
   <211> 9
   <212> PRT
   <213> Artificial
<220>
   <223> U1-29-CDR-3l
<400> 405
   Gln His Tyr Asp Thr Leu Pro Leu Thr
   1               5
<210> 406
   <211> 12
   <212> PRT
   <213> Artificial
<220>
   <223> U1-30-CDR-1h
<400> 406
   Gly Gly Ser Ile Ser Ser Gly Asp Tyr Tyr Trp Ser
   1               5                   10
<210> 407
   <211> 16
   <212> PRT
   <213> Artificial
<220>
   <223> U1-30-CDR-2h
<400> 407
   Tyr Ile Tyr Tyr Ser Gly Thr Thr Tyr Tyr Asn Pro Ser Leu Lys Ser
   1               5                   10                  15
<210> 408
   <211> 12
   <212> PRT
   <213> Artificial
<220>
   <223> U1-30-CDR-3h
<400> 408
   Ala Asp Tyr Asp Phe Trp Ser Gly Tyr Phe Asp Tyr
   1               5                   10
<210> 409
   <211> 11
   <212> PRT
   <213> Artificial
<220>
   <223> U1-30-CDR-1l
<400> 409
   Arg Ala Gly Gln Gly Ile Arg Asn Asp Leu Gly
   1 5 10
<210> 410
   <211> 7
   <212> PRT
   <213> Artificial
<220>
   <223> U1-30-CDR-2l
<400> 410
   Ala Ala Ser Ser Leu Gln Ser
   1 5
<210> 411
   <211> 9
   <212> PRT
   <213> Artificial
<220>
   <223> U1-30-CDR-3l
<400> 411
   Leu Gln His Asn Ser Tyr Pro Trp Thr
   1 5
<210> 412
   <211> 10
   <212> PRT
   <213> Artificial
<220>
   <223> U1-31-CDR-1h
<400> 412
   Gly Tyr Thr Phe Thr Asn Tyr Gly Ile Ser
   1 5 10
<210> 413
   <211> 17
   <212> PRT
   <213> Artificial
<220>
   <223> U1-31-CDR-2h
<400> 413
   Trp Ile Ser Ala Tyr Asp Gly Tyr Arg Asn Tyr Ala Gln Lys Leu Gln
   1 5 10 15
   Gly
<210> 414
   <211> 13
   <212> PRT
   <213> Artificial
<220>
   <223> U1-31-CDR-3h
<400> 414
   Asp Val Gln Asp Tyr Gly Asp Tyr Asp Tyr Phe Asp Tyr
   1 5 10
<210> 415
   <211> 11
   <212> PRT
   <213> Artificial
<220>
   <223> U1-31-CDR-1l
<400> 415
   Arg Ala Ser Gln Ser Ile Ser Ser Tyr Leu Asn
   1 5 10
<210> 416
   <211> 7
   <212> PRT
   <213> Artificial
<220>
   <223> U1-31-CDR-2l
<400> 416
   Ala Ala Ser Ser Leu Gln Ser
   1 5
<210> 417
   <211> 9
   <212> PRT
   <213> Artificial
<220>
   <223> U1-31-CDR-3l
<400> 417
   Gln Gln Ser Tyr Ser Thr Pro Ile Thr
   1 5
<210> 418
   <211> 12
   <212> PRT
   <213> Artificial
<220>
   <223> U1-32-CDR-1h
<400> 418
   Gly Gly Ser Ile Ser Ser Gly Asp Tyr Tyr Trp Ser
   1 5 10
<210> 419
   <211> 16
   <212> PRT
   <213> Artificial
<220>
   <223> U1-32-CDR-2h
<400> 419
   Tyr Ile Tyr Tyr Ser Gly Thr Thr Tyr Tyr Asn Pro Ser Leu Lys Ser
   1 5 10 15
<210> 420
   <211> 12
   <212> PRT
   <213> Artificial
<220>
   <223> U1-32-CDR-3h
<400> 420
   Ala Asp Tyr Asp Phe Trp Ser Gly Tyr Phe Asp Tyr
   1 5 10
<210> 421
   <211> 11
   <212> PRT
   <213> Artificial
<220>
   <223> U1-32-CDR-1l
<400> 421
   Arg Ala Gly Gln Gly Ile Arg Asn Asp Leu Gly
   1 5 10
<210> 422
   <211> 7
   <212> PRT
   <213> Artificial
<220>
   <223> U1-32-CDR-2l
<400> 422
   Ala Ala Ser Ser Leu Gln Ser
   1 5
<210> 423
   <211> 9
   <212> PRT
   <213> Artificial
<220>
   <223> U1-32-CDR-3l
<400> 423
   Leu Gln His Asn Ser Tyr Pro Trp Thr
   1               5
<210> 424
   <211> 12
   <212> PRT
   <213> Artificial
<220>
   <223> U1-33-CDR-1h
<400> 424
   Gly Gly Ser Ile Ser Ser Gly Asp Tyr Tyr Trp Ser
   1               5                   10
<210> 425
   <211> 16
   <212> PRT
   <213> Artificial
<220>
   <223> U1-33-CDR-2h
<400> 425
   Tyr Ile Tyr Tyr Ser Gly Ser Thr Tyr Tyr Asn Pro Ser Leu Lys Ser
   1               5                   10                  15
<210> 426
   <211> 12
   <212> PRT
   <213> Artificial
<220>
   <223> U1-33-CDR-3h
<400> 426
   Ala Asp Tyr Asp Phe Trp Ser Gly His Phe Asp Cys
   1               5                   10
<210> 427
   <211> 11
   <212> PRT
   <213> Artificial
<220>
   <223> U1-33-CDR-1l
<400> 427
   Arg Ala Ser Gln Gly Ile Arg Asp Asp Leu Gly
   1 5 10
<210> 428
   <211> 7
   <212> PRT
   <213> Artificial
<220>
   <223> U1-33-CDR-2l
<400> 428
   Ala Glu Ser Ser Leu Gln Ser
   1 5
<210> 429
   <211> 9
   <212> PRT
   <213> Artificial
<220>
   <223> U1-33-CDR-3l
<400> 429
   Leu Gln His His Ser Tyr Pro Trp Thr
   1 5
<210> 430
   <211> 10
   <212> PRT
   <213> Artificial
<220>
   <223> U1-34-CDR-1h
<400> 430
   Gly Tyr Thr Phe Thr Asn Tyr Gly Ile Ser
   1 5 10
<210> 431
   <211> 17
   <212> PRT
   <213> Artificial
<220>
   <223> U1-34-CDR-2h
<400> 431
   Trp Ile Ser Ala Tyr Asp Gly Tyr Arg Asn Tyr Ala Gln Lys Leu Gln
   1 5 10 15
   Gly
<210> 432
   <211> 13
   <212> PRT
   <213> Artificial
<220>
   <223> U1-34-CDR-3h
<400> 432
   Asp Val Gln Asp Tyr Gly Asp Tyr Asp Tyr Phe Asp Tyr
   1               5                   10
<210> 433
   <211> 11
   <212> PRT
   <213> Artificial
<220>
   <223> U1-34-CDR-1l
<400> 433
   Arg Ala Ser Gln Ser Ile Ser Ser Tyr Leu Asn
   1               5                   10
<210> 434
   <211> 7
   <212> PRT
   <213> Artificial
<220>
   <223> U1-34-CDR-2l
<400> 434
   Ala Ala Ser Ser Leu Gln Ser
   1               5
<210> 435
   <211> 9
   <212> PRT
   <213> Artificial
<220>
   <223> U1-34-CDR-3l
<400> 435
   Gln Gln Ser Tyr Ser Thr Pro Ile Thr
   1               5
<210> 436
   <211> 10
   <212> PRT
   <213> Artificial
<220>
   <223> U1-35-CDR-1h
<400> 436
   Gly Phe Thr Phe Ser Asp Tyr Tyr Met Ser
   1 5 10
<210> 437
   <211> 17
   <212> PRT
   <213> Artificial
<220>
   <223> U1-35-CDR-2h
<400> 437
   Tyr Ile Ser Ser Ser Gly Asn Asn Ile Tyr His Ala Asp Ser Val Lys
   1 5 10 15
   Gly
<210> 438
   <211> 14
   <212> PRT
   <213> Artificial
<220>
   <223> U1-35-CDR-3h
<400> 438
   Glu Arg Tyr Ser Gly Tyr Asp Asp Pro Asp Gly Phe Asp Ile
   1 5 10
<210> 439
   <211> 11
   <212> PRT
   <213> Artificial
<220>
   <223> U1-35-CDR-1l
<400> 439
   Gln Ala Ser Gln Asp Ile Ser Asn Tyr Leu Ser
   1 5 10
<210> 440
   <211> 7
   <212> PRT
   <213> Artificial
<220>
   <223> U1-35-CDR-2l
<400> 440
   Asp Ala Ser Asn Leu Glu Thr
   1 5
<210> 441
   <211> 9
   <212> PRT
   <213> Artificial
<220>
   <223> U1-35-CDR-3l
<400> 441
   Gln Gln Tyr Asp Asn Pro Pro Cys Ser
   1 5
<210> 442
   <211> 12
   <212> PRT
   <213> Artificial
<220>
   <223> U1-36-CDR-1h
<400> 442
   Gly Gly Ser Ile Ser Ser Gly Tyr Tyr Tyr Trp Ser
   1 5 10
<210> 443
   <211> 16
   <212> PRT
   <213> Artificial
<220>
   <223> U1-36-CDR-2h
<400> 443
   Tyr Ile Tyr Tyr Ser Gly Thr Thr Tyr Tyr Asn Pro Ser Phe Lys Ser
   1 5 10 15
<210> 444
   <211> 12
   <212> PRT
   <213> Artificial
<220>
   <223> U1-36-CDR-3h
<400> 444
   Ala Asp Tyr Asp Phe Trp Ser Gly His Phe Asp Tyr
   1 5 10
<210> 445
   <211> 11
   <212> PRT
   <213> Artificial
<220>
   <223> U1-36-CDR-1l
<400> 445
   Arg Ala Ser Gln Gly Ile Arg Asn Asp Leu Gly
   1 5 10
<210> 446
   <211> 7
   <212> PRT
   <213> Artificial
<220>
   <223> U1-36-CDR-2l
<400> 446
   Ala Ala Ser Ser Leu Gln Ser
   1 5
<210> 447
   <211> 9
   <212> PRT
   <213> Artificial
<220>
   <223> U1-36-CDR-3l
<400> 447
   Leu Gln His Asn Ser Tyr Pro Trp Thr
   1 5
<210> 448
   <211> 10
   <212> PRT
   <213> Artificial
<220>
   <223> U1-37-CDR-1h
<400> 448
   Gly Tyr Thr Phe Thr Ser Tyr Gly Ile Ser
   1 5 10
<210> 449
   <211> 17
   <212> PRT
   <213> Artificial
<220>
   <223> U1-37-CDR-2h
<400> 449
   Trp Ile Ser Ala Tyr Asp Gly His Thr Asn Tyr Ala Gln Lys Leu Gln
   1 5 10 15
   Gly
<210> 450
   <211> 13
   <212> PRT
   <213> Artificial
<220>
   <223> U1-37-CDR-3h
<400> 450
   Asp Pro His Asp Tyr Ser Asn Tyr Glu Ala Phe Asp Phe
   1 5 10
<210> 451
   <211> 11
   <212> PRT
   <213> Artificial
<220>
   <223> U1-37-CDR-1l
<400> 451
   Arg Ala Ser Gln Ser Ile Ser Ser Tyr Leu Asn
   1 5 10
<210> 452
   <211> 7
   <212> PRT
   <213> Artificial
<220>
   <223> U1-37-CDR-2l
<400> 452
   Ala Ala Ser Ser Leu Gln Ser
   1 5
<210> 453
   <211> 9
   <212> PRT
   <213> Artificial
<220>
   <223> U1-37-CDR-3l
<400> 453
   Gln Gln Ser Tyr Ser Thr Pro Ile Thr
   1               5
<210> 454
   <211> 12
   <212> PRT
   <213> Artificial
<220>
   <223> U1-38-CDR-1h
<400> 454
   Gly Phe Ser Leu Ser Thr Ser Gly Val Gly Val Gly
   1               5                   10
<210> 455
   <211> 16
   <212> PRT
   <213> Artificial
<220>
   <223> U1-38-CDR-2h
<400> 455
   Leu Ile Tyr Trp Asn Asp Asp Lys Arg Tyr Ser Pro Ser Leu Lys Ser
   1               5                   10                  15
<210> 456
   <211> 9
   <212> PRT
   <213> Artificial
<220>
   <223> U1-38-CDR-3h
<400> 456
   Arg Asp Glu Val Arg Gly Phe Asp Tyr
   1               5
<210> 457
   <211> 16
   <212> PRT
   <213> Artificial
<220>
   <223> U1-38-CDR-1l
<400> 457
   Arg Ser Ser Gln Ser Leu Val Tyr Ser Asp Gly Tyr Thr Tyr Leu His
   1               5                   10                  15
<210> 458
   <211> 7
   <212> PRT
   <213> Artificial
<220>
   <223> U1-38-CDR-2l
<400> 458
   Lys Val Ser Asn Trp Asp Ser
   1 5
<210> 459
   <211> 9
   <212> PRT
   <213> Artificial
<220>
   <223> U1-38-CDR-3l
<400> 459
   Met Gln Gly Ala His Trp Pro Ile Thr
   1 5
<210> 460
   <211> 10
   <212> PRT
   <213> Artificial
<220>
   <223> U1-39-CDR-1h
<400> 460
   Gly Phe Thr Val Ser Ser Asn Tyr Met Ser
   1 5 10
<210> 461
   <211> 16
   <212> PRT
   <213> Artificial
<220>
   <223> U1-39-CDR-2h
<400> 461
   Val Ile Tyr Ser Gly Gly Ser Thr Tyr Tyr Ala Asp Ser Val Lys Gly
   1 5 10 15
<210> 462
   <211> 6
   <212> PRT
   <213> Artificial
<220>
   <223> U1-39-CDR-3h
<400> 462
   Gly Gln Trp Leu Asp Val
   1               5
<210> 463
   <211> 16
   <212> PRT
   <213> Artificial
<220>
   <223> U1-39-CDR-1l
<400> 463
   Arg Ser Ser Gln Ser Leu Leu His Ser Asn Gly Tyr Asn Tyr Leu Asp
   1               5                   10                  15
<210> 464
   <211> 7
   <212> PRT
   <213> Artificial
<220>
   <223> U1-39-CDR-2l
<400> 464
   Leu Gly Phe His Arg Ala Ser
   1               5
<210> 465
   <211> 9
   <212> PRT
   <213> Artificial
<220>
   <223> U1-39-CDR-3l
<400> 465
   Arg Gln Ala Leu Gln Thr Pro Leu Thr
   1               5
<210> 466
   <211> 12
   <212> PRT
   <213> Artificial
<220>
   <223> U1-40-CDR-1h
<400> 466
   Gly Gly Ser Ile Ser Ser Gly Gly Tyr Tyr Trp Ser
   1               5                   10
<210> 467
   <211> 16
   <212> PRT
   <213> Artificial
<220>
   <223> U1-40-CDR-2h
<400> 467
   Tyr Ile Tyr Ser Ser Gly Ser Thr Tyr Tyr Asn Pro Ser Leu Lys Ser
   1               5                   10                  15
<210> 468
   <211> 15
   <212> PRT
   <213> Artificial
<220>
   <223> U1-40-CDR-3h
<400> 468
   Asp Arg Glu Leu Glu Leu Tyr Tyr Tyr Tyr Tyr Gly Met Asp Val
   1               5                   10                  15
<210> 469
   <211> 16
   <212> PRT
   <213> Artificial
<220>
   <223> U1-40-CDR-1l
<400> 469
   Arg Ser Ser Gln Ser Leu Leu Tyr Ser Asn Gly Tyr Asn Tyr Leu Asp
   1               5                   10                  15
<210> 470
   <211> 7
   <212> PRT
   <213> Artificial
<220>
   <223> U1-40-CDR-2l
<400> 470
   Leu Gly Ser Asn Arg Ala Ser
   1               5
<210> 471
   <211> 9
   <212> PRT
   <213> Artificial
<220>
   <223> U1-40-CDR-3l
<400> 471
   Met Gln Ala Leu Gln Thr Pro Leu Thr
   1 5
<210> 472
   <211> 12
   <212> PRT
   <213> Artificial
<220>
   <223> U1-41-CDR-1h
<400> 472
   Gly Gly Ser Ile Ser Ser Gly Gly Tyr Tyr Trp Ser
   1 5 10
<210> 473
   <211> 16
   <212> PRT
   <213> Artificial
<220>
   <223> U1-41-CDR-2h
<400> 473
   Tyr Ile Tyr Tyr Ser Gly Ser Thr Tyr Tyr Asn Pro Ser Leu Lys Ser
   1 5 10 15
<210> 474
   <211> 15
   <212> PRT
   <213> Artificial
<220>
   <223> U1-41-CDR-3h
<400> 474
   Asp Arg Glu Leu Glu Gly Tyr Ser Asn Tyr Tyr Gly Val Asp Val
   1 5 10 15
<210> 475
   <211> 11
   <212> PRT
   <213> Artificial
<220>
   <223> U1-41-CDR-1l
<400> 475
   Arg Ala Ser Gln Ala Ile Ser Asn Tyr Leu Asn
   1 5 10
<210> 476
   <211> 7
   <212> PRT
   <213> Artificial
<220>
   <223> U1-41-CDR-2l
<400> 476
   Ala Ala Ser Ser Leu Gln Ser
   1               5
<210> 477
   <211> 9
   <212> PRT
   <213> Artificial
<220>
   <223> U1-41-CDR-3l
<400> 477
   Gln Gln Asn Asn Ser Leu Pro Ile Thr
   1               5
<210> 478
   <211> 10
   <212> PRT
   <213> Artificial
<220>
   <223> U1-42-CDR-1h
<400> 478
   Gly Tyr Ser Phe Thr Ser Tyr Trp Ile Gly
   1               5                   10
<210> 479
   <211> 17
   <212> PRT
   <213> Artificial
<220>
   <223> U1-42-CDR-2h
<400> 479
   Ile Ile Tyr Pro Gly Asp Ser Asp Thr Arg Tyr Ser Pro Ser Phe Gln
   1               5                   10                  15
   Gly
<210> 480
   <211> 9
   <212> PRT
   <213> Artificial
<220>
   <223> U1-42-CDR-3h
<400> 480
   His Glu Asn Tyr Gly Asp Tyr Asn Tyr
   1 5
<210> 481
   <211> 11
   <212> PRT
   <213> Artificial
<220>
   <223> U1-42-CDR-1l
<400> 481
   Arg Ala Ser Gln Ser Ile Arg Ser Tyr Leu Asn
   1 5 10
<210> 482
   <211> 7
   <212> PRT
   <213> Artificial
<220>
   <223> U1-42-CDR-2l
<400> 482
   Ala Ala Ser Ser Leu Gln Ser
   1 5
<210> 483
   <211> 9
   <212> PRT
   <213> Artificial
<220>
   <223> U1-42-CDR-3l
<400> 483
   Gln Gln Ser Asn Gly Ser Pro Leu Thr
   1 5
<210> 484
   <211> 12
   <212> PRT
   <213> Artificial
<220>
   <223> U1-43-CDR-1h
<400> 484
   Gly Gly Ser Ile Ser Ser Gly Gly Tyr Tyr Trp Ser
   1 5 10
<210> 485
   <211> 16
   <212> PRT
   <213> Artificial
<220>
   <223> U1-43-CDR-2h
<400> 485
   Tyr Ile Tyr Tyr Ser Gly Ser Thr Tyr Tyr Asn Pro Ser Leu Arg Ser
   1 5 10 15
<210> 486
   <211> 17
   <212> PRT
   <213> Artificial
<220>
   <223> U1-43-CDR-3h
<400> 486
   Asp Arg Glu Arg Glu Trp Asp Asp Tyr Gly Asp Pro Gln Gly Met Asp
   1 5 10 15
   Val
<210> 487
   <211> 11
   <212> PRT
   <213> Artificial
<220>
   <223> U1-43-CDR-1l
<400> 487
   Arg Ala Ser Gln Ser Ile Ser Ser Tyr Leu His
   1 5 10
<210> 488
   <211> 7
   <212> PRT
   <213> Artificial
<220>
   <223> U1-43-CDR-2l
<400> 488
   Ala Ala Ser Ser Leu Gln Ser
   1 5
<210> 489
   <211> 9
   <212> PRT
   <213> Artificial
<220>
   <223> U1-43-CDR-3l
<400> 489
   Gln Gln Ser Tyr Ser Asn Pro Leu Thr
   1               5
<210> 490
   <211> 10
   <212> PRT
   <213> Artificial
<220>
   <223> U1-44-CDR-1h
<400> 490
   Gly Tyr Ser Phe Thr Ser Tyr Trp Ile Gly
   1               5                   10
<210> 491
   <211> 17
   <212> PRT
   <213> Artificial
<220>
   <223> U1-44-CDR-2h
<400> 491
   Ile Ile Trp Pro Gly Asp Ser Asp Thr Ile Tyr Ser Pro Ser Phe Gln
   1               5                   10                  15
   Gly
<210> 492
   <211> 9
   <212> PRT
   <213> Artificial
<220>
   <223> U1-44-CDR-3h
<400> 492
   His Glu Asn Tyr Gly Asp Tyr Asn Tyr
   1               5
<210> 493
   <211> 11
   <212> PRT
   <213> Artificial
<220>
   <223> U1-44-CDR-1l
<400> 493
   Arg Ala Ser Gln Ser Ile Arg Ser Tyr Leu Asn
   1               5                   10
<210> 494
   <211> 7
   <212> PRT
   <213> Artificial
<220>
   <223> U1-44-CDR-2l
<400> 494
   Ala Ala Ser Ser Leu Gln Ser
   1               5
<210> 495
   <211> 9
   <212> PRT
   <213> Artificial
<220>
   <223> U1-44-CDR-3l
<400> 495
   Gln Gln Ser Ile Ser Ser Pro Leu Thr
   1               5
<210> 496
   <211> 10
   <212> PRT
   <213> Artificial
<220>
   <223> U1-45-CDR-1h
<400> 496
   Gly Tyr Thr Phe Thr Ser Tyr Asp Ile Asn
   1               5                   10
<210> 497
   <211> 17
   <212> PRT
   <213> Artificial
<220>
   <223> U1-45-CDR-2h
<400> 497
   Trp Met Asn Pro Asn Ser Gly Asp Thr Gly Tyr Ala Gln Val Phe Gln
   1 5 10 15
   Gly
<210> 498
   <211> 16
   <212> PRT
   <213> Artificial
<220>
   <223> U1-45-CDR-3h
<400> 498
   Phe Gly Asp Leu Pro Tyr Asp Tyr Ser Tyr Tyr Glu Trp Phe Asp Pro
   1 5 10 15
<210> 499
   <211> 11
   <212> PRT
   <213> Artificial
<220>
   <223> U1-45-CDR-1l
<400> 499
   Arg Ala Ser Gln Ser Ile Ser Ser Tyr Leu Asn
   1 5 10
<210> 500
   <211> 7
   <212> PRT
   <213> Artificial
<220>
   <223> U1-45-CDR-2l
<400> 500
   Ala Ala Ser Ser Leu Gln Ser
   1 5
<210> 501
   <211> 9
   <212> PRT
   <213> Artificial
<220>
   <223> U1-45-CDR-3l
<400> 501
   Gln Gln Ser Tyr Ser Thr Pro Leu Thr
   1 5
<210> 502
   <211> 12
   <212> PRT
   <213> Artificial
<220>
   <223> U1-46-CDR-1h
<400> 502
   Gly Asp Ser Val Ser Ser Asn Ser Ala Ala Trp Asn
   1 5 10
<210> 503
   <211> 18
   <212> PRT
   <213> Artificial
<220>
   <223> U1-46-CDR-2h
<400> 503
   Arg Thr Tyr Tyr Arg Ser Lys Trp Tyr Asn Asp Tyr Ala Val Ser Val
   1 5 10 15
   Lys Ser
<210> 504
   <211> 17
   <212> PRT
   <213> Artificial
<220>
   <223> U1-46-CDR-3h
<400> 504
   Asp Leu Tyr Asp Phe Trp Ser Gly Tyr Pro Tyr Tyr Tyr Gly Met Asp
   1 5 10 15
   Val
<210> 505
   <211> 12
   <212> PRT
   <213> Artificial
<220>
   <223> U1-47-CDR-1h
<400> 505
   Gly Asp Ser Val Ser Ser Asn Ser Ala Ala Trp Asn
   1 5 10
<210> 506
   <211> 18
   <212> PRT
   <213> Artificial
<220>
   <223> U1-47-CDR-2h
<400> 506
   Arg Thr Tyr Tyr Arg Ser Lys Trp Tyr Asn Asp Tyr Ala Val Ser Val
   1 5 10 15
   Lys Ser
<210> 507
   <211> 16
   <212> PRT
   <213> Artificial
<220>
   <223> U1-47-CDR-3h
<400> 507
   Asp Tyr Tyr Gly Ser Gly Ser Phe Tyr Tyr Tyr Tyr Gly Met Asp Val
   1 5 10 15
<210> 508
   <211> 11
   <212> PRT
   <213> Artificial
<220>
   <223> U1-47-CDR-1l
<400> 508
   Arg Ala Ser Gln Ser Ile Ser Ser Tyr Leu Asn
   1 5 10
<210> 509
   <211> 7
   <212> PRT
   <213> Artificial
<220>
   <223> U1-47-CDR-2l
<400> 509
   Ala Ala Ser Asn Leu Gln Ser
   1 5
<210> 510
   <211> 9
   <212> PRT
   <213> Artificial
<220>
   <223> U1-47-CDR-3l
<400> 510
   Gln Gln Ser Tyr Ser Thr Pro Arg Thr
   1               5
<210> 511
   <211> 10
   <212> PRT
   <213> Artificial
<220>
   <223> U1-48-CDR-1h
<400> 511
   Gly Gly Ser Ile Ser Ser Tyr Tyr Trp Ser
   1               5                   10
<210> 512
   <211> 16
   <212> PRT
   <213> Artificial
<220>
   <223> U1-48-CDR-2h
<400> 512
   His Ile Tyr Thr Ser Gly Ser Thr Asn Tyr Asn Pro Ser Leu Lys Ser
   1               5                   10                  15
<210> 513
   <211> 16
   <212> PRT
   <213> Artificial
<220>
   <223> U1-48-CDR-3h
<400> 513
   Glu Ala Ile Phe Gly Val Gly Pro Tyr Tyr Tyr Tyr Gly Met Asp Val
   1               5                   10                  15
<210> 514
   <211> 10
   <212> PRT
   <213> Artificial
<220>
   <223> U1-49-CDR-1h
<400> 514
   Gly Tyr Thr Phe Thr Gly Tyr Tyr Met His
   1 5 10
<210> 515
   <211> 17
   <212> PRT
   <213> Artificial
<220>
   <223> U1-49-CDR-2h
<400> 515
   Trp Ile Asn Pro Asn Ile Gly Gly Thr Asn Cys Ala Gln Lys Phe Gln
   1 5 10 15
   Gly
<210> 516
   <211> 17
   <212> PRT
   <213> Artificial
<220>
   <223> U1-49-CDR-3h
<400> 516
   Gly Gly Arg Tyr Ser Ser Ser Trp Ser Tyr Tyr Tyr Tyr Gly Met Asp
   1 5 10 15
   Val
<210> 517
   <211> 16
   <212> PRT
   <213> Artificial
<220>
   <223> U1-49-CDR-1l
<400> 517
   Lys Ser Ser Gln Ser Leu Leu Leu Ser Asp Gly Gly Thr Tyr Leu Tyr
   1 5 10 15
<210> 518
   <211> 7
   <212> PRT
   <213> Artificial
<220>
   <223> U1-49-CDR-2l
<400> 518
   Glu Val Ser Asn Arg Phe Ser
   1 5
<210> 519
   <211> 9
   <212> PRT
   <213> Artificial
<220>
   <223> U1-49-CDR-3l
<400> 519
   Met Gln Ser Met Gln Leu Pro Ile Thr
   1 5
<210> 520
   <211> 12
   <212> PRT
   <213> Artificial
<220>
   <223> U1-50-CDR-1h
<400> 520
   Gly Gly Ser Val Ser Ser Gly Gly Tyr Tyr Trp Ser
   1 5 10
<210> 521
   <211> 16
   <212> PRT
   <213> Artificial
<220>
   <223> U1-50-CDR-2h
<400> 521
   Tyr Ile Tyr Tyr Ser Gly Ser Thr Asn Tyr Asn Pro Ser Leu Lys Ser
   1 5 10 15
<210> 522
   <211> 17
   <212> PRT
   <213> Artificial
<220>
   <223> U1-50-CDR-3h
<400> 522
   Gly Gly Asp Ser Asn Tyr Glu Asp Tyr Tyr Tyr Tyr Tyr Gly Met Asp
   1 5 10 15
   Val
<210> 523
   <211> 11
   <212> PRT
   <213> Artificial
<220>
   <223> U1-50-CDR-1l
<400> 523
   Arg Ala Ser Gln Ser Ile Ser Ile Tyr Leu His
   1 5 10
<210> 524
   <211> 7
   <212> PRT
   <213> Artificial
<220>
   <223> U1-50-CDR-2l
<400> 524
   Ala Ala Ser Ser Leu Gln Ser
   1 5
<210> 525
   <211> 9
   <212> PRT
   <213> Artificial
<220>
   <223> U1-50-CDR-3l
<400> 525
   Gln Gln Ser Tyr Thr Ser Pro Ile Thr
   1 5
<210> 526
   <211> 10
   <212> PRT
   <213> Artificial
<220>
   <223> U1-51-CDR-1h
<400> 526
   Gly Gly Ser Ile Ser Ser Tyr Tyr Trp Ser
   1 5 10
<210> 527
   <211> 16
   <212> PRT
   <213> Artificial
<220>
   <223> U1-51-CDR-2h
<400> 527
   Tyr Ile Tyr Tyr Ser Gly Ser Thr Asn Tyr Asn Pro Ser Leu Lys Ser
   1               5                   10                  15
<210> 528
   <211> 19
   <212> PRT
   <213> Artificial
<220>
   <223> U1-51-CDR-3h
<400> 528
   Asp Ser Ser Tyr Tyr Asp Ser Ser Gly Tyr Tyr Leu Tyr Tyr Tyr Ala
   1               5                   10                  15
   Met Asp Val
<210> 529
   <211> 17
   <212> PRT
   <213> Artificial
<220>
   <223> U1-51-CDR-11
<400> 529
   Lys Ser Ser Gln Ser Val Leu Tyr Ser Ser Asn Asn Lys Asn Tyr Leu
   1               5                   10                  15
   Ala
<210> 530
   <211> 7
   <212> PRT
   <213> Artificial
<220>
   <223> U1-51-CDR-2l
<400> 530
   Trp Ala Ser Thr Arg Glu Ser
   1               5
<210> 531
   <211> 9
   <212> PRT
   <213> Artificial
<220>
   <223> U1-51-CDR-3l
<400> 531
   Gln Gln Tyr Tyr Thr Thr Pro Leu Thr
   1               5
<210> 532
   <211> 12
   <212> PRT
   <213> Artificial
<220>
   <223> U1-52-CDR-1h
<400> 532
   Gly Gly Ser Ile Ser Ser Gly Gly Tyr Tyr Trp Ser
   1               5                   10
<210> 533
   <211> 16
   <212> PRT
   <213> Artificial
<220>
   <223> U1-52-CDR-2h
<400> 533
   Asn Ile Tyr Tyr Ser Gly Ser Thr Tyr Tyr Asn Pro Ser Leu Lys Ser
   1               5                   10                  15
<210> 534
   <211> 15
   <212> PRT
   <213> Artificial
<220>
   <223> U1-52-CDR-3h
<400> 534
   Gly Gly Thr Gly Thr Asn Tyr Tyr Tyr Tyr Tyr Gly Met Asp Val
   1               5                   10                  15
<210> 535
   <211> 12
   <212> PRT
   <213> Artificial
<220>
   <223> U1-52-CDR-1l
<400> 535
   Arg Ala Ser Gln Ser Val Ser Ser Ser Tyr Leu Ala
   1 5 10
<210> 536
   <211> 7
   <212> PRT
   <213> Artificial
<220>
   <223> U1-52-CDR-2l
<400> 536
   Gly Ala Ser Ser Trp Ala Thr
   1 5
<210> 537
   <211> 9
   <212> PRT
   <213> Artificial
<220>
   <223> U1-52-CDR-3l
<400> 537
   Gln Gln Tyr Gly Ser Ser Pro Leu Thr
   1 5
<210> 538
   <211> 10
   <212> PRT
   <213> Artificial
<220>
   <223> U1-53-CDR-1h
<400> 538
   Gly Phe Thr Phe Ser Ile Tyr Ser Met Asn
   1 5 10
<210> 539
   <211> 17
   <212> PRT
   <213> Artificial
<220>
   <223> U1-53-CDR-2h
<400> 539
   Tyr Ile Ser Ser Ser Ser Ser Thr Ile Tyr Tyr Ala Asp Ser Val Lys
   1 5 10 15
   Gly
<210> 540
   <211> 10
   <212> PRT
   <213> Artificial
<220>
   <223> U1-53-CDR-3h
<400> 540
   Asp Arg Gly Asp Phe Asp Ala Phe Asp Ile
   1 5 10
<210> 541
   <211> 11
   <212> PRT
   <213> Artificial
<220>
   <223> U1-53-CDR-1l
<400> 541
   Gln Ala Ser Gln Asp Ile Thr Asn Tyr Leu Asn
   1 5 10
<210> 542
   <211> 7
   <212> PRT
   <213> Artificial
<220>
   <223> U1-53-CDR-2l
<400> 542
   Asp Ala Ser Asn Leu Glu Thr
   1 5
<210> 543
   <211> 9
   <212> PRT
   <213> Artificial
<220>
   <223> U1-53-CDR-3l
<400> 543
   Gln Gln Cys Glu Asn Phe Pro Ile Thr
   1 5
<210> 544
   <211> 12
   <212> PRT
   <213> Artificial
<220>
   <223> U1-55.1-CDR-1h
<400> 544
   Gly Gly Ser Val Ser Ser Gly Gly Tyr Tyr Trp Asn
   1               5                   10
<210> 545
   <211> 16
   <212> PRT
   <213> Artificial
<220>
   <223> U1-55.1-CDR-2h
<400> 545
   Tyr Ile Asn Tyr Ser Gly Ser Thr Asn Tyr Asn Pro Ser Leu Lys Ser
   1               5                   10                  15
<210> 546
   <211> 15
   <212> PRT
   <213> Artificial
<220>
   <223> U1-55.1-CDR-3h
<400> 546
   Asp Arg Glu Leu Glu Leu Tyr Tyr Tyr Tyr Tyr Gly Met Asp Val
   1               5                   10                  15
<210> 547
   <211> 16
   <212> PRT
   <213> Artificial
<220>
   <223> U1-55-CDR-1l
<400> 547
   Arg Ser Ser Gln Ser Leu Leu Tyr Ser Asn Gly Tyr Lys Tyr Leu Asp
   1               5                   10                  15
<210> 548
   <211> 7
   <212> PRT
   <213> Artificial
<220>
   <223> U1-55-CDR-2l
<400> 548
   Leu Gly Ser Asn Arg Ala Ser
   1 5
<210> 549
   <211> 9
   <212> PRT
   <213> Artificial
<220>
   <223> U1-55-CDR-3l
<400> 549
   Met Gln Ala Leu Gln Thr Pro Ile Thr
   1 5
<210> 550
   <211> 16
   <212> PRT
   <213> Artificial
<220>
   <223> U1-57.1-CDR-1l
<400> 550
   Arg Ser Ser Gln Ser Leu Leu Tyr Ser Asn Gly Tyr Lys Tyr Leu Asp
   1 5 10 15
<210> 551
   <211> 7
   <212> PRT
   <213> Artificial
<220>
   <223> U1-57.1-CDR-2l
<400> 551
   Leu Gly Ser Asn Arg Ala Ser
   1 5
<210> 552
   <211> 9
   <212> PRT
   <213> Artificial
<220>
   <223> U1-57.1-CDR-3l
<400> 552
   Met Gln Ala Leu Gln Thr Pro Ile Thr
   1 5
<210> 553
   <211> 12
   <212> PRT
   <213> Artificial
<220>
   <223> U1-57_CDR-1h
<400> 553
   Gly Gly Ser Val Ser Ser Gly Gly Tyr Tyr Trp Asn
   1 5 10
<210> 554
   <211> 16
   <212> PRT
   <213> Artificial
<220>
   <223> U1-57_CDR-2h
<400> 554
   Tyr Ile Asn Tyr Ser Gly Ser Thr Asn Tyr Asn Pro Ser Leu Lys Ser
   1 5 10 15
<210> 555
   <211> 15
   <212> PRT
   <213> Artificial
<220>
   <223> U1-57_CDR-3h
<400> 555
   Asp Arg Glu Leu Glu Leu Tyr Tyr Tyr Tyr Tyr Gly Met Asp Val
   1 5 10 15
<210> 556
   <211> 10
   <212> PRT
   <213> Artificial
<220>
   <223> U1-58-CDR-1h
<400> 556
   Gly Phe Thr Phe Ser Ser Tyr Gly Met His
   1 5 10
<210> 557
   <211> 17
   <212> PRT
   <213> Artificial
<220>
   <223> U1-58-CDR-2h
<400> 557
   Val Ile Trp Tyr Asp Gly Ser Asn Lys Tyr Tyr Ala Asp Ser Val Lys
   1 5 10 15
   Gly
<210> 558
   <211> 12
   <212> PRT
   <213> Artificial
<220>
   <223> U1-58-CDR-3h
<400> 558
   Ala Ala Arg Leu Asp Tyr Tyr Tyr Gly Met Asp Val
   1 5 10
<210> 559
   <211> 11
   <212> PRT
   <213> Artificial
<220>
   <223> U1-58-CDR-1l
<400> 559
   Arg Ala Ser Gln Ser Ile Asn Ser Tyr Leu Asn
   1 5 10
<210> 560
   <211> 7
   <212> PRT
   <213> Artificial
<220>
   <223> U1-58-CDR-2l
<400> 560
   Gly Ala Ser Gly Leu Gln Ser
   1 5
<210> 561
   <211> 9
   <212> PRT
   <213> Artificial
<220>
   <223> U1-58-CDR-3l
<400> 561
   Gln Gln Ser Tyr Ser Ser Pro Leu Thr
   1 5
<210> 562
   <211> 10
   <212> PRT
   <213> Artificial
<220>
   <223> U1-59-CDR-1h
<400> 562
   Gly Gly Ser Phe Ser Gly Tyr Tyr Trp Ser
   1 5 10
<210> 563
   <211> 16
   <212> PRT
   <213> Artificial
<220>
   <223> U1-59-CDR-2h
<400> 563
   Glu Ile Asn His Ser Gly Ser Thr Asn Tyr Asn Pro Ser Leu Lys Ser
   1 5 10 15
<210> 564
   <211> 9
   <212> PRT
   <213> Artificial
<220>
   <223> U1-59-CDR-3h
<400> 564
   Asp Lys Trp Thr Trp Tyr Phe Asp Leu
   1 5
<210> 565
   <211> 17
   <212> PRT
   <213> Artificial
<220>
   <223> U1-59-CDR-1l
<400> 565
   Arg Ser Ser Gln Ser Val Leu Tyr Ser Ser Ser Asn Arg Asn Tyr Leu
   1               5                   10                  15
   Ala
<210> 566
   <211> 7
   <212> PRT
   <213> Artificial
<220>
   <223> U1-59-CDR-2l
<400> 566
   Trp Ala Ser Thr Arg Glu Ser
   1               5
<210> 567
   <211> 9
   <212> PRT
   <213> Artificial
<220>
   <223> U1-59-CDR-3l
<400> 567
   Gln Gln Tyr Tyr Ser Thr Pro Arg Thr
   1               5
<210> 568
   <211> 12
   <212> PRT
   <213> Artificial
<220>
   <223> U1-61.1-CDR-1h
<400> 568
   Gly Val Ser Ile Ser Ser Gly Gly Tyr Tyr Trp Ser
   1               5                   10
<210> 569
   <211> 16
   <212> PRT
   <213> Artificial
<220>
   <223> U1-61.1-CDR-2h
<400> 569
   Tyr Ile Tyr Tyr Ser Gly Ser Thr Tyr Tyr Asn Pro Ser Leu Lys Ser
   1               5                   10                  15
<210> 570
   <211> 16
   <212> PRT
   <213> Artificial
<220>
   <223> U1-61.1-CDR-3h
<400> 570
   Asp Ser Glu Ser Glu Tyr Ser Ser Ser Ser Asn Tyr Gly Met Asp Val
   1               5                   10                  15
<210> 571
   <211> 11
   <212> PRT
   <213> Artificial
<220>
   <223> U1-61.1-CDR-1l
<400> 571
   Arg Ala Ser Gln Thr Ile Ser Ser Tyr Leu Asn
   1               5                   10
<210> 572
   <211> 7
   <212> PRT
   <213> Artificial
<220>
   <223> U1-61.1-CDR-2l
<400> 572
   Ala Ala Ser Ser Leu Gln Gly
   1               5
<210> 573
   <211> 9
   <212> PRT
   <213> Artificial
<220>
   <223> U1-61.1-CDR-3l
<400> 573
   Gln Gln Ser Tyr Ser Asn Pro Leu Thr
   1               5
<210> 574
   <211> 12
   <212> PRT
   <213> Artificial
<220>
   <223> U1-61-CDR-1h
<400> 574
   Gly Val Ser Ile Ser Ser Gly Gly Tyr Tyr Trp Ser
   1               5                   10
<210> 575
   <211> 16
   <212> PRT
   <213> Artificial
<220>
   <223> U1-61-CDR-2h
<400> 575
   Tyr Ile Tyr Tyr Ser Gly Ser Thr Tyr Tyr Asn Pro Ser Leu Lys Ser
   1               5                   10                  15
<210> 576
   <211> 16
   <212> PRT
   <213> Artificial
<220>
   <223> U1-61-CDR-3h
<400> 576
   Asp Ser Glu Ser Glu Tyr Ser Ser Ser Ser Asn Tyr Gly Met Asp Val
   1               5                   10                  15
<210> 577
   <211> 10
   <212> PRT
   <213> Artificial
<220>
   <223> U1-62-CDR-1h
<400> 577
   Gly Tyr Ser Phe Thr Ser Tyr Trp Ile Gly
   1               5                   10
<210> 578
   <211> 17
   <212> PRT
   <213> Artificial
<220>
   <223> U1-62-CDR-2h
<400> 578
   Ile Ile Tyr Pro Gly Asp Ser Asp Thr Arg Tyr Ser Pro Ser Phe Gln
   1 5 10 15
   Gly
<210> 579
   <211> 12
   <212> PRT
   <213> Artificial
<220>
   <223> U1-62-CDR-3h
<400> 579
   Gln Met Ala Gly Asn Tyr Tyr Tyr Gly Met Asp Val
   1 5 10
<210> 580
   <211> 12
   <212> PRT
   <213> Artificial
<220>
   <223> U1-62-CDR-1l
<400> 580
   Arg Ala Ser Gln Ser Val Ile Ser Ile Tyr Leu Ala
   1 5 10
<210> 581
   <211> 7
   <212> PRT
   <213> Artificial
<220>
   <223> U1-62-CDR-2l
<400> 581
   Gly Ala Ser Ser Arg Ala Thr
   1 5
<210> 582
   <211> 9
   <212> PRT
   <213> Artificial
<220>
   <223> U1-62-CDR-3l
<400> 582
   Gln Gln Tyr Gly Ser Ser Pro Cys Ser
   1 5